# EUROPEAN PATENT APPLICATION

(11) **EP 4 056 566 A1**
(43) Date of publication of application: **14.09.2022**
(21) Application number: 20886138.5
(22) Date of filing: 05.11.2020
(51) Int. Cl.: C07D 405/14, C07D 405/04, C07D 409/14, C07D 491/048, C07D 495/04, H01L 51/00, H01L 51/50

(54) **HETEROCYCLIC COMPOUND, ORGANIC LIGHT-EMITTING DEVICE COMPRISING SAME, COMPOSITION FOR ORGANIC MATERIAL LAYER OF ORGANIC LIGHT-EMITTING DEVICE, AND METHOD FOR MANUFACTURING ORGANIC LIGHT-EMITTING DEVICE**

(30) Priority: 06.11.2019 KR 20190140749
(71) Applicant: LT Materials Co., Ltd., Yongin-si, Gyeonggi-do 17118 (KR)
(72) Inventor: YANG, Seung-Gyu, Yongin-si, Gyeonggi-do 17118 (KR); KIM, Su-Yeon, Yongin-si, Gyeonggi-do 17118 (KR); CHOI, Eui-Jeong, Yongin-City, Gyeonggi-do 17118 (KR); NO, Young-Seok, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2020/015414
(87) International publication number: WO 2021/091259

(57) **Abstract**

The present specification provides a heterocyclic compound represented by Chemical Formula 1, an organic light emitting device comprising the same, a composition for an organic material layer of an organic light emitting device, and a method for manufacturing an organic light emitting device.

## Description

### [Technical Field]

This application claims priority to and the benefits of Korean Patent Application No. 10-2019-0140749, filed with the Korean Intellectual Property Office on November 6, 2019, the entire contents of which are incorporated herein by reference.

The present specification relates to a heterocyclic compound, an organic light emitting device comprising the same, a composition for an organic material layer of an organic light emitting device, and a method for manufacturing an organic light emitting device.

### [Background Art]

An organic electroluminescent device is one type of selfemissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a hostdopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

Studies on an organic light emitting device comprising a compound capable of satisfying conditions required for materials usable in an organic light emitting device, for example, satisfying proper energy level, electrochemical stability, thermal stability and the like, and having a chemical structure capable of performing various roles required in an organic light emitting device depending on substituents have been required.

### Prior Art Documents

### Patent Documents

US Patent. No. 4,356,429

### [Disclosure]

### [Technical Problem]

The present specification relates to a heterocyclic compound, an organic light emitting device comprising the same, a composition for an organic material layer of an organic light emitting device, and a method for manufacturing an organic light emitting device.

### [Technical Solution]

One embodiment of the present application provides a heterocyclic compound represented by the following Chemical Formula 1.

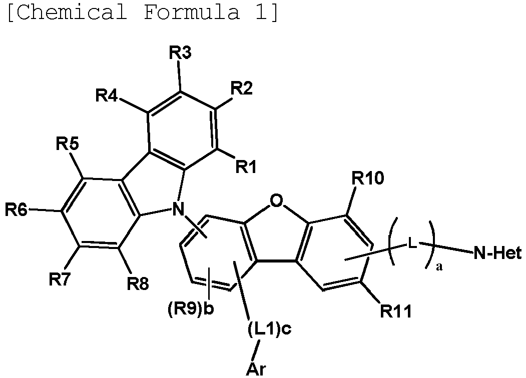

In Chemical Formula 1,
N-Het is a monocyclic or polycyclic C2 to C60 heterocyclic group substituted or unsubstituted, and comprising one or more Ns,
L and L1 are the same as or different from each other, and each independently a direct bond; a substituted or unsubstituted C6 to C60 arylene group; or a substituted or unsubstituted C2 to C60 heteroarylene group,
Ar is a substituted or unsubstituted C1 to C60 alkyl group; a substituted or unsubstituted C6 to C60 aryl group; or a substituted or unsubstituted C2 to C60 heteroaryl group,
R1 to R11 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R"; and -NRR', or two or more groups adjacent to each other bond to each other to form a substituted or unsubstituted C6 to C60 aromatic hydrocarbon ring or a substituted or unsubstituted C2 to C60 heteroring,
R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group,
a and c are an integer of 0 to 4, and
b is an integer of 0 to 2.

In addition, one embodiment of embodiment of the present application provides an organic light emitting device comprising a first electrode; a second electrode provided opposite to the first electrode; and one or more organic material layers provided between the first electrode and the second electrode, wherein one or more layers of the organic material layers comprise the heterocyclic compound represented by Chemical Formula 1.

In addition, one embodiment of the present application provides a composition for an organic material layer of an organic light emitting device, the composition comprising the heterocyclic compound represented by Chemical Formula 1 and a heterocyclic compound represented by the following Chemical Formula 2.

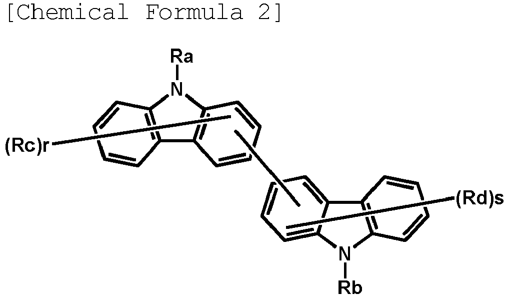

In Chemical Formula 2,
Ra and Rb are the same as or different from each other, and each independently a substituted or unsubstituted C6 to C60 aryl group; or a substituted or unsubstituted C2 to C60 heteroaryl group,
Rc and Rd are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; and a substituted or unsubstituted amine group, and
r and s are an integer of 0 to 7.

Lastly, one embodiment of the present application provides a method for manufacturing an organic light emitting device, the method comprising preparing a substrate; forming a first electrode on the substrate; forming one or more organic material layers on the first electrode; and forming a second electrode on the organic material layer, wherein the forming of organic material layers comprises forming one or more organic material layers using the composition for an organic material layer according to one embodiment of the present application.

### [Advantageous Effects]

A compound described in the present specification can be used as a material of an organic material layer of an organic light emitting device. In the organic light emitting device, the compound is capable of performing a role of a hole injection material, a hole transfer material, a light emitting material, an electron transfer material, an electron injection material or the like. Particularly, the compound can be used as a light emitting material of the organic light emitting device. For example, the compound can be used alone as a light emitting material, or two of the compounds can be used together as a light emitting material, and can be used as a host material of a light emitting layer.

Particularly, in the compound of Chemical Formula 1, a specific position of one side benzene ring of the dibenzofuran structure is substituted with an N-containing ring, and another benzene ring not substituted with the N-containing ring in the dibenzofuran ring is substituted with a carbazole substituent and a specific substituent. In this case, overall linearity of the material increases, and a dipole moment of the material is more strengthened. As a result, an electron withdrawing effect, a property that a strong ET unit comprising an N-containing ring has, more strongly reveals, and a property of further withdrawing delocalized electrons of the dibenzofuran to the ET unit is obtained.

By using the compounds of Chemical Formula 1, an organic light emitting device with improved lifetime, driving stability and efficiency can be manufactured.

### [Description of Drawings]

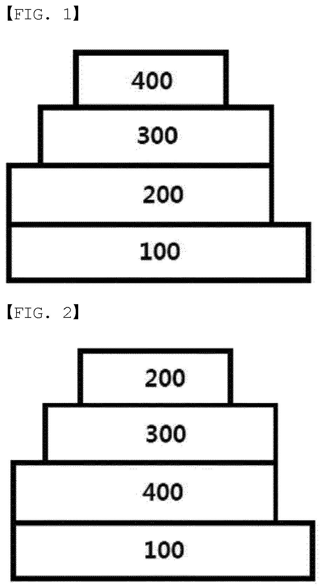

FIG. 1 to FIG. 3 are diagrams each schematically illustrating a lamination structure of an organic light emitting device according to one embodiment of the present application.

### [Reference Numeral]

- 100:: Substrate
- 200:: Anode
- 300:: Organic Material Layer
- 301:: Hole Injection Layer
- 302:: Hole Transfer Layer
- 303:: Light Emitting Layer
- 304:: Hole Blocking Layer
- 305:: Electron Transfer Layer
- 306:: Electron Injection Layer
- 400:: Cathode

### [Mode for Disclosure]

Hereinafter, the present application will be described in detail.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0% or a hydrogen content being 100%. In other words, an expression of "substituent X is hydrogen" does not exclude deuterium such as a hydrogen content being 100% or a deuterium content being 0%, and therefore, may mean a state in which hydrogen and deuterium are mixed.

In one embodiment of the present application, deuterium is one of isotopes of hydrogen, is an element having deuteron formed with one proton and one neutron as a nucleus, and may be expressed as hydrogen-2, and the elemental symbol may also be written as D or 2H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 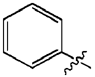 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

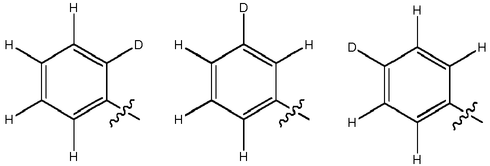

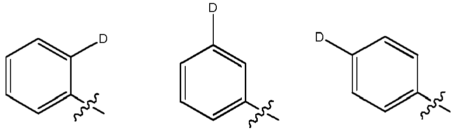

In addition, in one embodiment of the present application, "a phenyl group having a deuterium content of 0%" may mean a phenyl group that does not comprise a deuterium atom, that is, a phenyl group that has 5 hydrogen atoms.

In the present specification, the halogen may be fluorine, chlorine, bromine or iodine.

In the present specification, the alkyl group comprises linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may comprise a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethylbutyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group comprises linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may comprise a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group comprises linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may comprise methoxy, ethoxy, n-propoxy, isopropoxy, i-propyloxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the cycloalkyl group comprises monocyclic or polycyclic having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may comprise a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group comprises O, S, Se, N or Si as a heteroatom, comprises monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group comprises monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The number of carbon atoms of the aryl group may be from 6 to 60, specifically from 6 to 40 and more specifically from 6 to 25. Specific examples of the aryl group may comprise a phenyl group, a biphenyl group, a triphenyl group (terphenyl group), a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, an indenyl group, an acenaphthylenyl group, a 2,3-dihydro-1H-indenyl group, a fused ring thereof, and the like, but are not limited thereto.

In the present specification, a fluorenyl group may be substituted, and adjacent substituents may bond to each other to form a ring.

When the fluorenyl group is substituted, the substituted fluorenyl group may be represented by the following structures, but is not limited thereto.

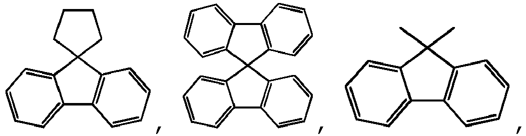

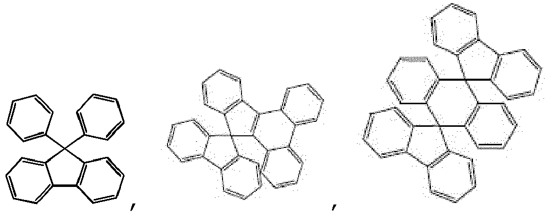

In the present specification, the heteroaryl group comprises S, O, Se, N or Si as a heteroatom, comprises monocyclic or polycyclic having 2 to 60 carbon atoms, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may comprise a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, a 5,10-dihydrobenzo[b,e][1,4]azasilinyl group, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may comprise a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except for those that are each a divalent group. In addition, the heteroarylene group means the heteroaryl group having two bonding sites, that is, a divalent group. The descriptions on the heteroaryl group provided above may be applied thereto except for those that are each a divalent group.

In the present specification, the phosphine oxide group is represented by -P (=O) R₁₀₁R₁₀₂, and R₁₀₁ and R₁₀₂ are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the phosphine oxide may comprise a diphenylphosphine oxide group, a dinaphthylphosphine oxide group and the like, but are not limited thereto.

In the present specification, the silyl group is a substituent comprising Si, having the Si atom directly linked as a radical, and is represented by -SiR₁₀₄R₁₀₅R₁₀₆. R₁₀₄ to R₁₀₆ are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may comprise a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

As the aliphatic or aromatic hydrocarbon ring or heteroring that adjacent groups may form, the structures illustrated as the cycloalkyl group, the cycloheteroalkyl group, the aryl group and the heteroaryl group described above may be used except for those that are not a monovalent group.

In the present specification, the term "substitution" means a hydrogen atom bonding to a carbon atom of a compound being changed to another substituent, and the position of substitution is not limited as long as it is a position at which the hydrogen atom is substituted, that is, a position at which a substituent can substitute, and when two or more substituents substitute, the two or more substituents may be the same as or different from each other.

In the present specification, "substituted or unsubstituted" means being substituted with one or more substituents selected from the group consisting of C1 to C60 linear or branched alkyl; C2 to C60 linear or branched alkenyl; C2 to C60 linear or branched alkynyl; C3 to C60 monocyclic or polycyclic cycloalkyl; C2 to C60 monocyclic or polycyclic heterocycloalkyl; C6 to C60 monocyclic or polycyclic aryl; C2 to C60 monocyclic or polycyclic heteroaryl; -SiRR'R"; - P(=O)RR'; C1 to C20 alkylamine; C6 to C60 monocyclic or polycyclic arylamine; and C2 to C60 monocyclic or polycyclic heteroarylamine, or being unsubstituted, or being substituted with a substituent linking two or more substituents selected from among the substituents illustrated above, or being unsubstituted, and
R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group.

One embodiment of the present application provides a compound represented by Chemical Formula 1.

In one embodiment of the present application, Chemical Formula 1 may be represented by the following Chemical Formula 3 or 4.

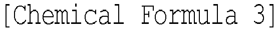

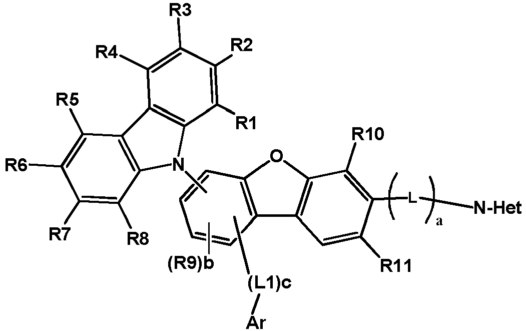

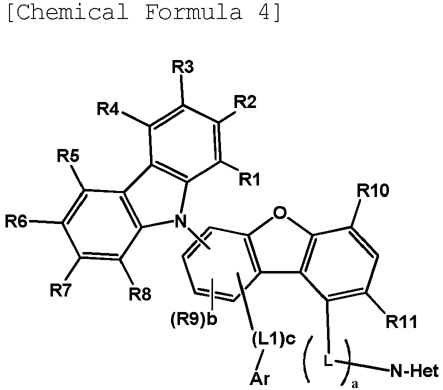

In Chemical Formulae 3 and 4,
R1 to R11, N-Het, L, L1, Ar, a, b and c have the same definitions as in Chemical Formula 1.

In Chemical Formula 3, a No. 3 position of the dibenzofuran is substituted with a substituent of N-Het, and in this case, overall linearity of the material increases, and a dipole moment of the material is more strengthened. As a result, an electron withdrawing effect, a property that a strong ET unit comprising the substituent of N-Het has, more strongly reveals, and delocalized electrons of the dibenzofuran are further withdrawn toward the ET unit.

Herein, the LUMO site of the host material is positioned centering on the substituent of N-Het that is the ET unit, and when electrons are strongly attracted, an area overlapping with the HOMO site significantly decreases around the dibenzofuran. In addition, electron density in the HOMO site decreases since electrons present in the area are localized toward the LUMO site. Accordingly, charge transfer in the molecule caused by the overlap between LUMO-HOMO also decreases leading to more increased stability of the molecular structure, and as a result, a device lifetime particularly increases.

In Chemical Formula 4, a No. 1 position of the dibenzofuran is substituted with a substituent of N-Het, and in this case, a relatively slightly decreased linearity is obtained, however, an effect of increasing or decreasing linearity to a certain level is obtained depending on the substituted position of the carbazole-based substituent or Ar substituent locating on the opposite side of the dibenzofuran. Accordingly, some electron withdrawing effect is obtained, however, charge transfer in the molecule is more active since the degree of electron delocalization tends to be higher. In other words, Chemical Formula 4 is particularly superior in driving voltage and current efficiency in the device due to the presence of delocalized electrons and their active charge transfer effect.

In one embodiment of the present application, Chemical Formula 3 may be represented by any one of the following Chemical Formulae 3-1 to 3-6.

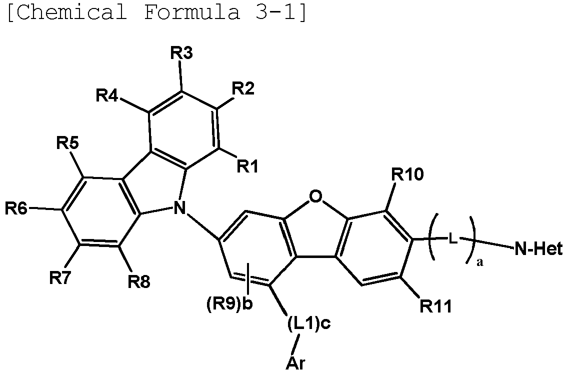

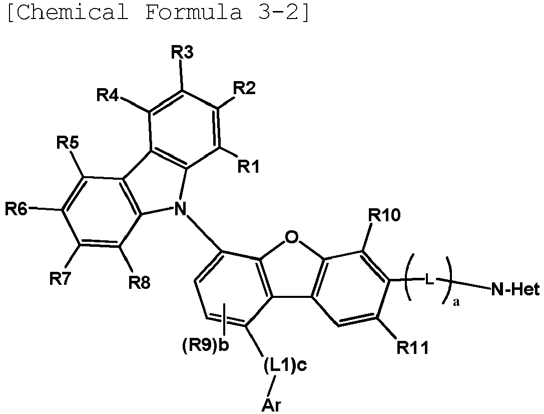

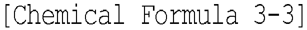

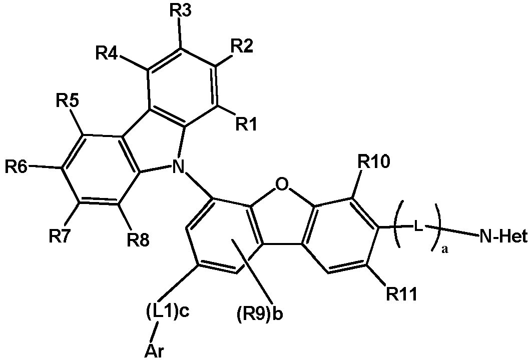

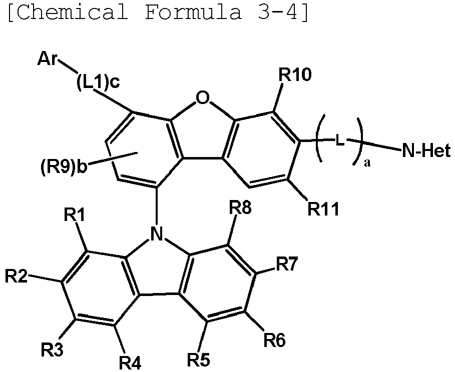

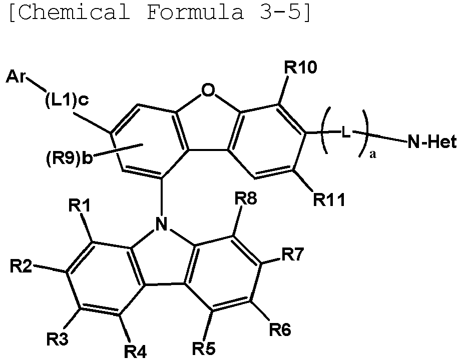

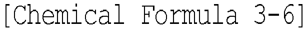

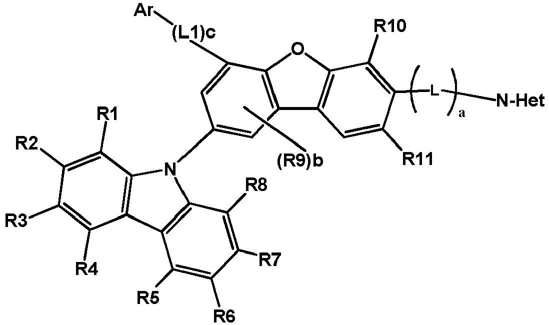

In Chemical Formulae 3-1 to 3-6,
R1 to R11, N-Het, L, L1, Ar, a, b and c have the same definitions as in Chemical Formula 3.

In one embodiment of the present application, Chemical Formula 4 may be represented by any one of the following Chemical Formulae 4-1 to 4-6.

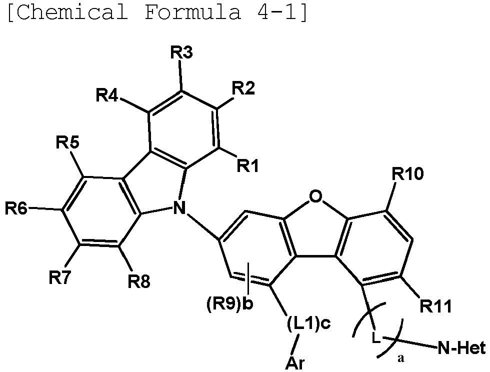

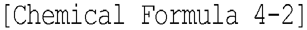

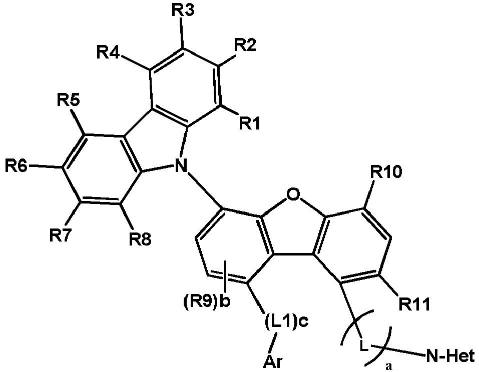

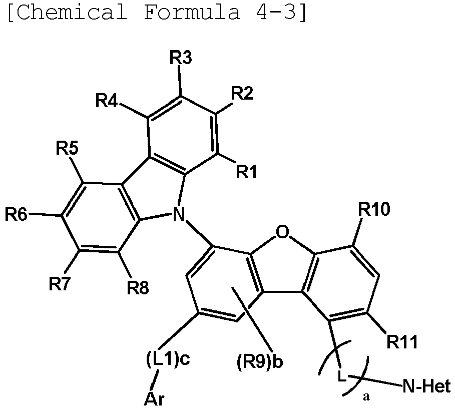

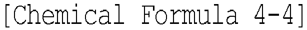

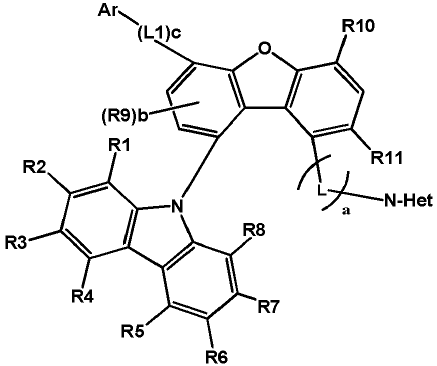

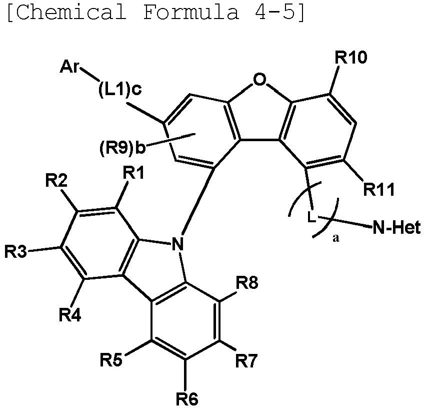

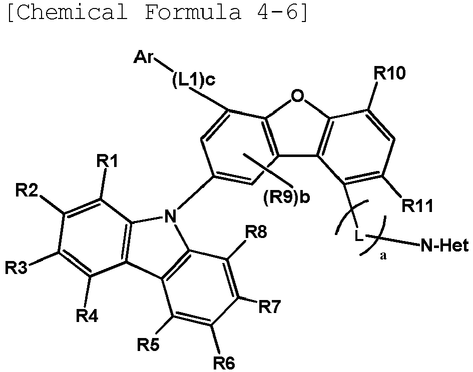

In Chemical Formulae 4-1 to 4-6,
R1 to R11, N-Het, L, L1, Ar, a, b and c have the same definitions as in Chemical Formula 4.

In one embodiment of the present application, L and L1 are the same as or different from each other, and each independently a direct bond; a substituted or unsubstituted C6 to C60 arylene group; or a substituted or unsubstituted C2 to C60 heteroarylene group.

In another embodiment, L and L1 are the same as or different from each other, and may be each independently a direct bond; a substituted or unsubstituted C6 to C40 arylene group; or a substituted or unsubstituted C2 to C40 heteroarylene group.

In another embodiment, L and L1 are the same as or different from each other, and may be each independently a direct bond; or a substituted or unsubstituted C6 to C40 arylene group.

In another embodiment, L and L1 are the same as or different from each other, and may be each independently a direct bond; or a substituted or unsubstituted C6 to C40 monocyclic or polycyclic arylene group.

In another embodiment, L and L1 are the same as or different from each other, and may be each independently a direct bond; a substituted or unsubstituted C6 to C20 monocyclic arylene group; or a substituted or unsubstituted C10 to C30 polycyclic arylene group.

In another embodiment, L and L1 are the same as or different from each other, and may be each independently a direct bond; a C6 to C20 monocyclic arylene group; or a C10 to C30 polycyclic arylene group.

In another embodiment, L and L1 are the same as or different from each other, and may be each independently a direct bond; a phenylene group; or a biphenylene group.

In another embodiment, L may be a direct bond.

In another embodiment, L1 may be a direct bond; a phenylene group; or a biphenylene group.

In one embodiment of the present application, Ar may be a substituted or unsubstituted C1 to C60 alkyl group; a substituted or unsubstituted C6 to C60 aryl group; or a substituted or unsubstituted C2 to C60 heteroaryl group.

In another embodiment, Ar may be a substituted or unsubstituted C6 to C40 aryl group; or a substituted or unsubstituted C2 to C40 heteroaryl group.

In another embodiment, Ar may be a C6 to C40 aryl group unsubstituted or substituted with a C1 to C20 alkyl group or deuterium; or a C2 to C40 heteroaryl group.

In another embodiment, Ar may be a C6 to C40 aryl group unsubstituted or substituted with a C1 to C20 alkyl group or deuterium; or a C2 to C40 heteroaryl group comprising O or S.

In another embodiment, Ar may be a C6 to C30 aryl group unsubstituted or substituted with a C1 to C20 alkyl group or deuterium; or a C2 to C30 heteroaryl group comprising O or S.

In another embodiment, Ar may be a phenyl group unsubstituted or substituted with a methyl group or deuterium; a biphenyl group; a naphthyl group; a terphenyl group; a dibenzothiophene group; or a dibenzofuran group.

In another embodiment, Ar may be any one of the following structures.

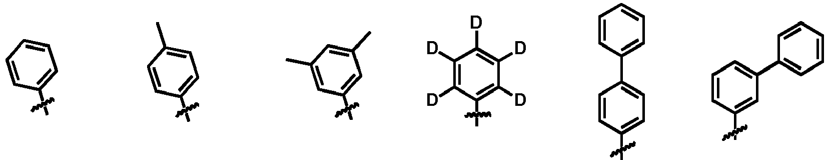

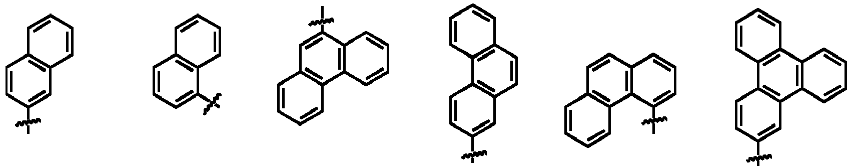

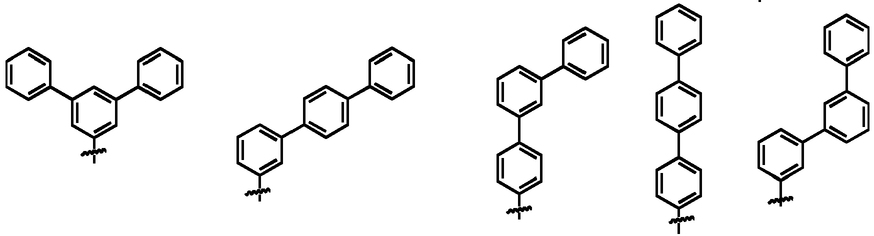

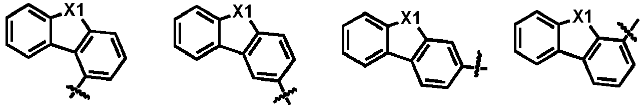

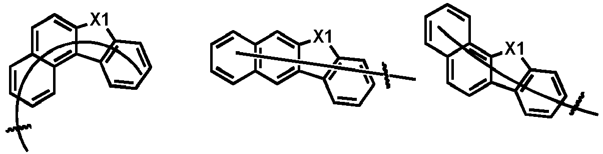

In the structural formulae,
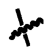 means a site linked to L1, and
X1 is O; or S.

In one embodiment of the present application, R1 to R11 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R"; and a substituted or unsubstituted amine group, or two or more groups adjacent to each other may bond to each other to form a substituted or unsubstituted C6 to C60 aromatic hydrocarbon ring or a substituted or unsubstituted C2 to C60 heteroring.

In another embodiment, R1 to R11 are the same as or different from each other, and each independently selected from the group consisting of hydrogen; a substituted or unsubstituted C6 to C60 aryl group; a substituted or unsubstituted C2 to C60 heteroaryl group; -P(=O)RR'; -SiRR'R"; and a substituted or unsubstituted amine group, or two or more groups adjacent to each other may bond to each other to form a substituted or unsubstituted C6 to C60 aromatic hydrocarbon ring or a substituted or unsubstituted C2 to C60 heteroring.

In another embodiment, R1 to R11 are the same as or different from each other, and each independently selected from the group consisting of hydrogen; and a substituted or unsubstituted C6 to C60 aryl group, or two or more groups adjacent to each other may bond to each other to form a substituted or unsubstituted C6 to C60 aromatic hydrocarbon ring or a substituted or unsubstituted C2 to C60 heteroring.

In another embodiment, R1 to R11 are the same as or different from each other, and each independently selected from the group consisting of hydrogen; and a substituted or unsubstituted C6 to C40 aryl group, or two or more groups adjacent to each other may bond to each other to form a substituted or unsubstituted C6 to C40 aromatic hydrocarbon ring or a substituted or unsubstituted C2 to C40 heteroring.

In another embodiment, R1 to R11 are the same as or different from each other, and each independently selected from the group consisting of hydrogen; and a C6 to C40 aryl group unsubstituted or substituted with deuterium, or two or more groups adjacent to each other may bond to each other to form a C6 to C40 aromatic hydrocarbon ring unsubstituted or substituted with a C1 to C20 alkyl group or a C2 to C40 heteroring unsubstituted or substituted with a C6 to C30 aryl group.

In another embodiment, R1 to R11 are the same as or different from each other, and each independently hydrogen; or a phenyl group unsubstituted or substituted with deuterium, or two or more groups adjacent to each other may bond to each other to form an indene ring unsubstituted or substituted with a methyl group; a benzene ring; a benzofuran ring; a benzothiophene ring; or an indole ring unsubstituted or substituted with a phenyl group.

In one embodiment of the present application, R9 to R11 may be hydrogen.

In one embodiment of the present application, R1 to R8 are the same as or different from each other, and each independently hydrogen; or a phenyl group unsubstituted or substituted with deuterium, or two or more groups adjacent to each other may bond to each other to form an indene ring unsubstituted or substituted with a methyl group; a benzene ring; a benzofuran ring; a benzothiophene ring; or an indole ring unsubstituted or substituted with a phenyl group.

In one embodiment of the present application, N-Het may be a monocyclic or polycyclic C2 to C60 heterocyclic group substituted or unsubstituted, and comprising one or more Ns.

In another embodiment, N-Het may be a monocyclic or polycyclic C2 to C60 heterocyclic group substituted or unsubstituted, and comprising one or more and three or less Ns.

In another embodiment, N-Het may be a monocyclic or polycyclic C2 to C60 heterocyclic group substituted or unsubstituted, and comprising one or more and two or less Ns.

In another embodiment, N-Het may be a monocyclic or polycyclic C2 to C40 heterocyclic group substituted or unsubstituted, and comprising one or more and three or less Ns.

In another embodiment, N-Het may be a monocyclic C2 to C40 heterocyclic group substituted or unsubstituted, and comprising one or more and three or less Ns.

In another embodiment, N-Het may be a monocyclic C2 to C40 heterocyclic group unsubstituted or substituted with one or more substituents selected from the group consisting of a C1 to C20 alkyl group, a C6 to C40 aryl group, a C2 to C40 heteroaryl group, -P(=)ORR' and -SiRR'R" or a substituent linking two or more of the substituents, and comprising one or more and three or less Ns.

In another embodiment, N-Het may be a pyridine group; a pyrimidine group; or a triazine group unsubstituted or substituted with one or more substituents selected from the group consisting of a C6 to C40 aryl group and a C2 to C40 heteroaryl group or a substituent linking two or more of the substituents.

In another embodiment, N-Het may be a pyridine group; a pyrimidine group; or a triazine group unsubstituted or substituted with one or more substituents selected from the group consisting of a phenyl group, a biphenyl group, a naphthyl group, a dibenzofuran group and a dibenzothiophene group.

In one embodiment of the present application, N-Het may be selected from among the following structural formulae.

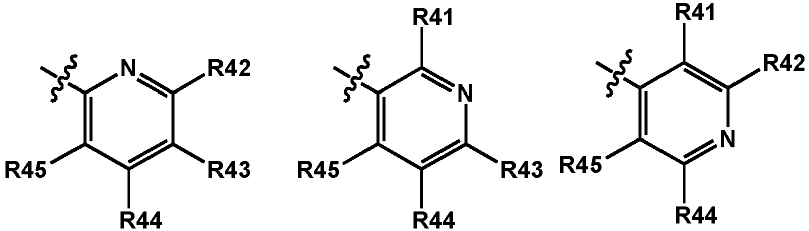

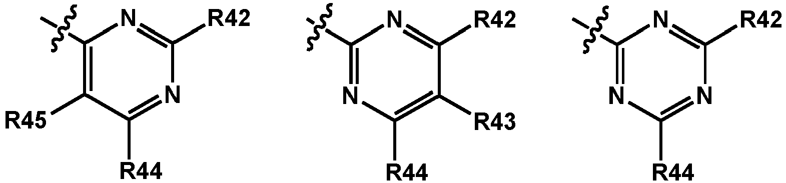

In the structural formulae, 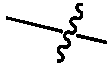 means a site linked to L of Chemical Formula 1,

R41 to R45 are the same as or different from each other, and each independently hydrogen; a substituted or unsubstituted C1 to C60 alkyl group; a substituted or unsubstituted C6 to C60 aryl group; or a substituted or unsubstituted C2 to C60 heteroaryl group.

In one embodiment of the present application, R41 to R45 are the same as or different from each other, and may be each independently hydrogen; a substituted or unsubstituted C1 to C60 alkyl group; a substituted or unsubstituted C6 to C60 aryl group; or a substituted or unsubstituted C2 to C60 heteroaryl group.

In another embodiment, R41 to R45 are the same as or different from each other, and may be each independently hydrogen; a substituted or unsubstituted C1 to C40 alkyl group; a substituted or unsubstituted C6 to C40 aryl group; or a substituted or unsubstituted C2 to C40 heteroaryl group.

In another embodiment, R41 to R45 are the same as or different from each other, and may be each independently a C6 to C40 aryl group; or a C2 to C40 heteroaryl group.

In another embodiment, R41 to R45 are the same as or different from each other, and may be each independently a phenyl group; a biphenyl group; a naphthyl group; a dibenzofuran group; or a dibenzothiophene group.

In one embodiment of the present application, R, R' and R" are the same as or different from each other, and may be each independently a substituted or unsubstituted C1 to C60 alkyl group; a substituted or unsubstituted C6 to C60 aryl group; or a substituted or unsubstituted C2 to C60 heteroaryl group.

In another embodiment, R, R' and R" are the same as or different from each other, and may be each independently a substituted or unsubstituted C6 to C60 aryl group.

In another embodiment, R, R' and R" are the same as or different from each other, and may be each independently a substituted or unsubstituted C6 to C60 monocyclic or polycyclic aryl group.

In another embodiment, R, R' and R" are the same as or different from each other, and may be each independently a substituted or unsubstituted C6 to C40 monocyclic aryl group.

In another embodiment, R, R' and R" are the same as or different from each other, and may be each independently a C6 to C20 monocyclic aryl group.

In another embodiment, R, R' and R" may be a phenyl group.

According to one embodiment of the present application, Chemical Formula 1 may be represented by any one of the following compounds, but is not limited thereto.

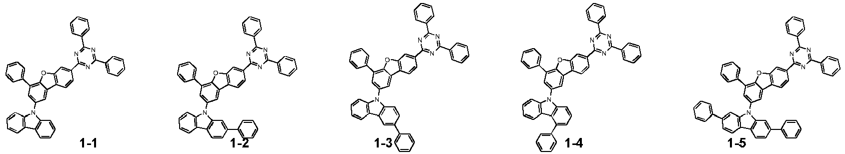

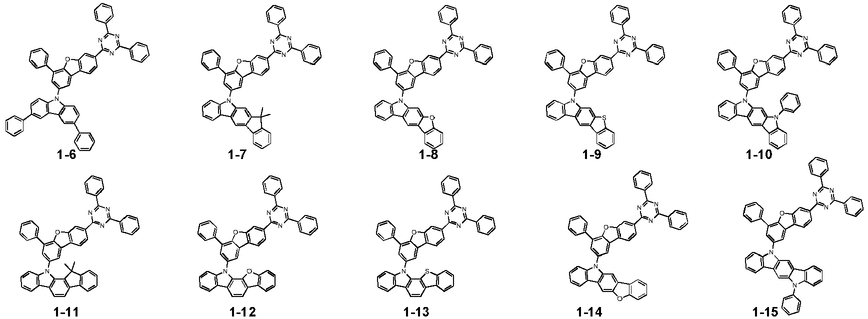

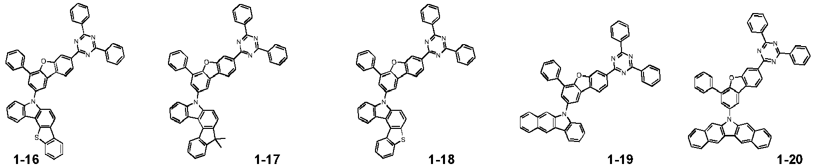

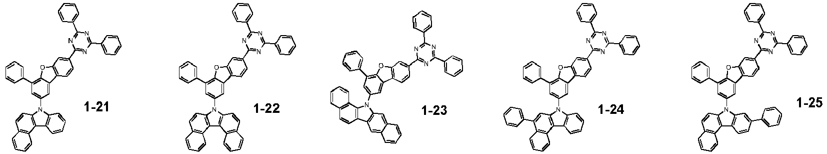

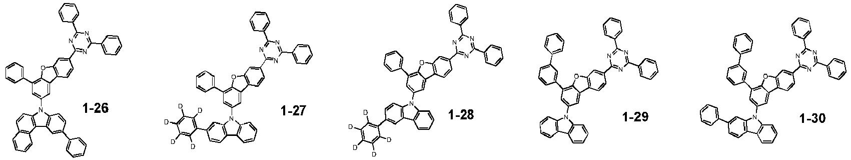

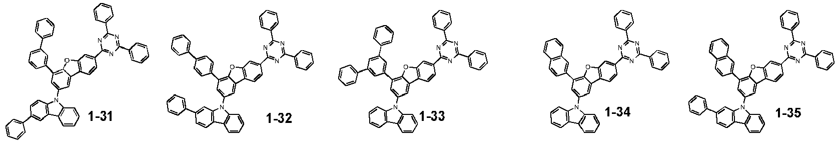

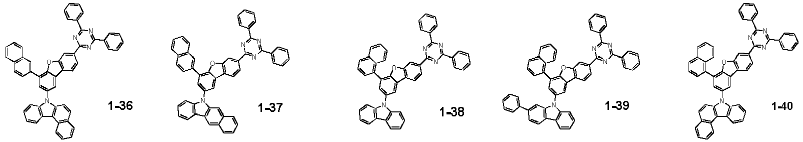

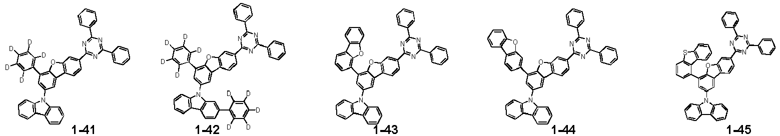

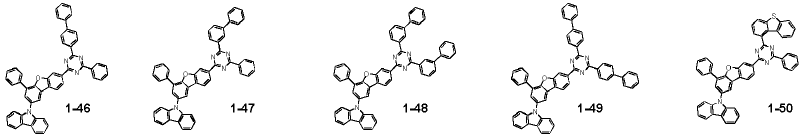

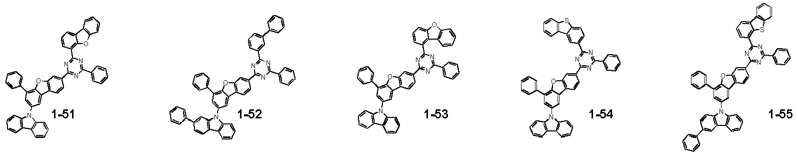

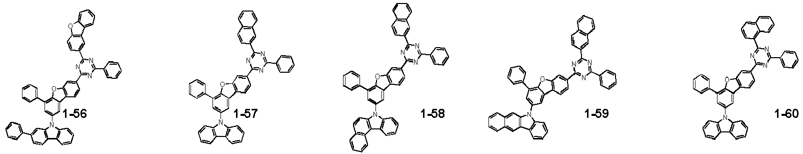

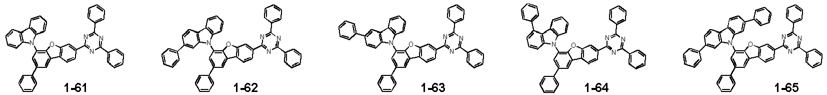

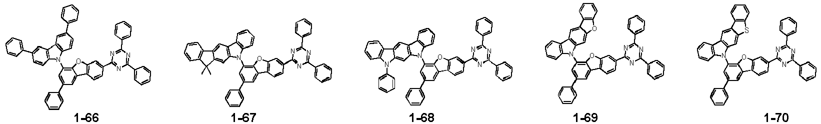

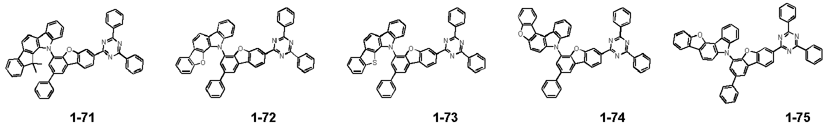

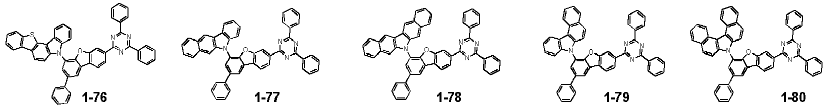

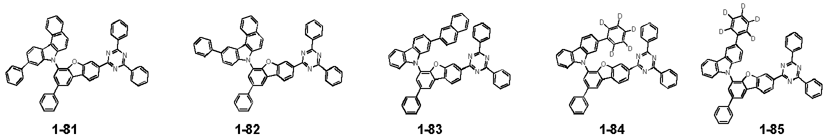

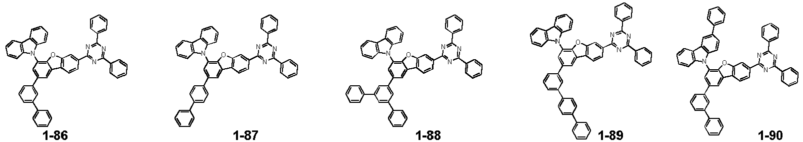

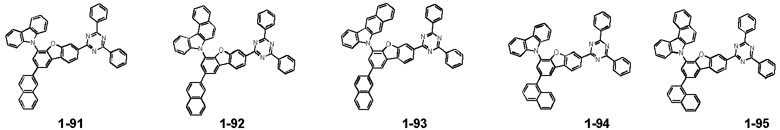

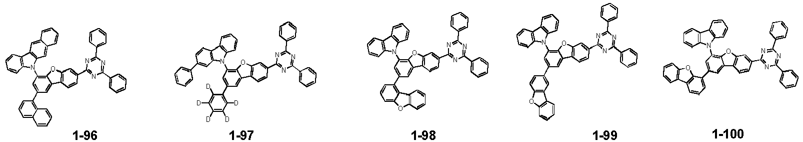

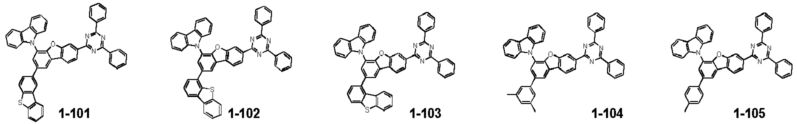

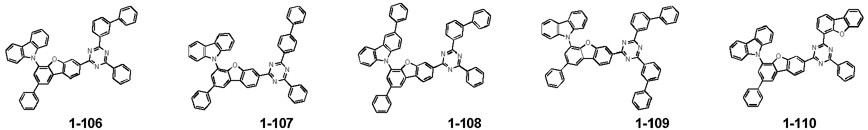

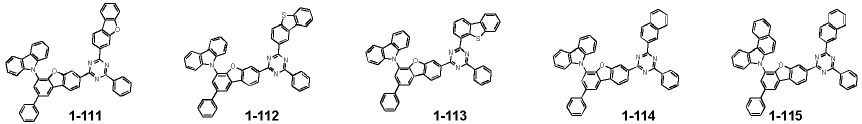

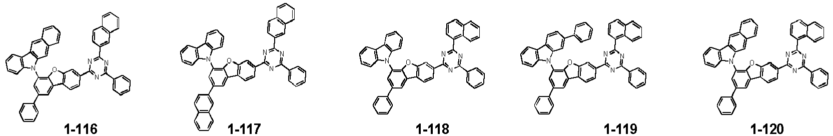

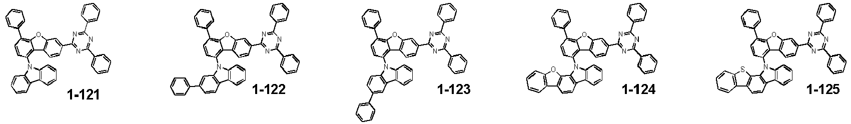

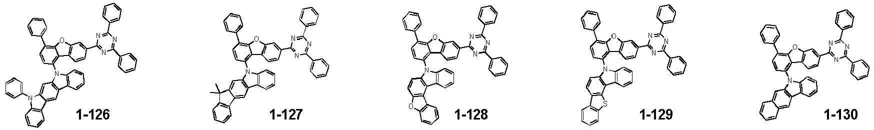

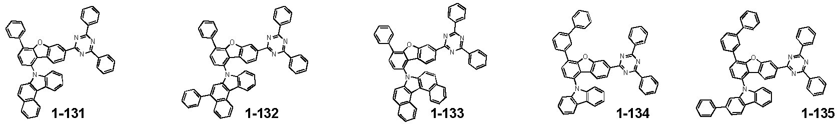

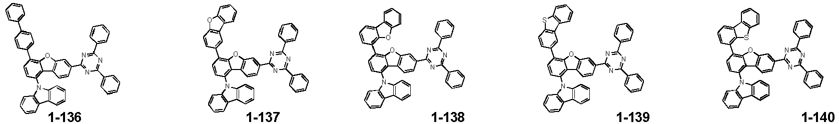

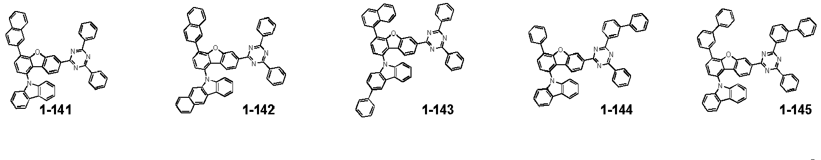

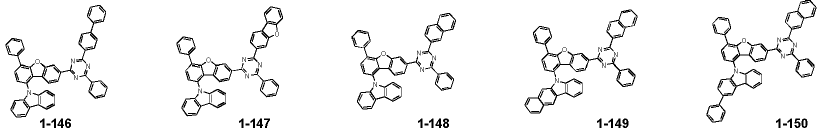

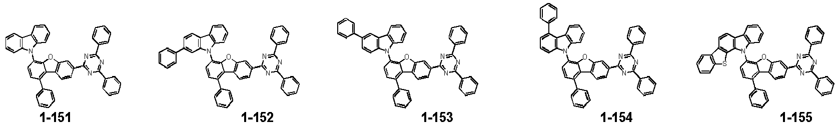

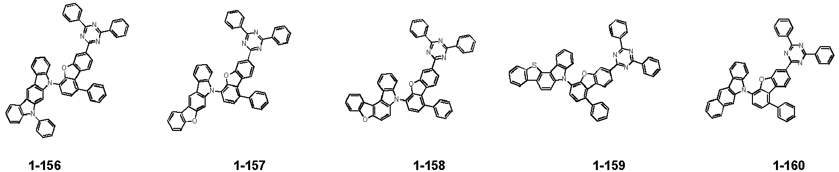

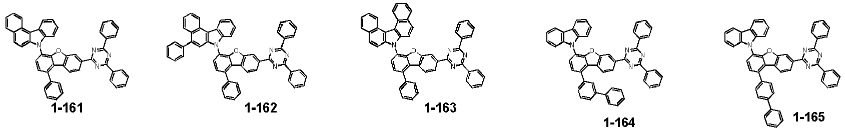

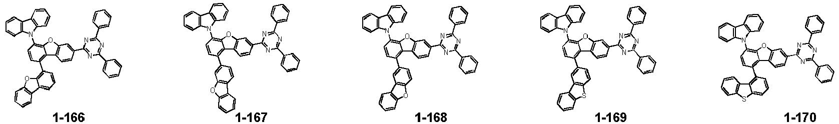

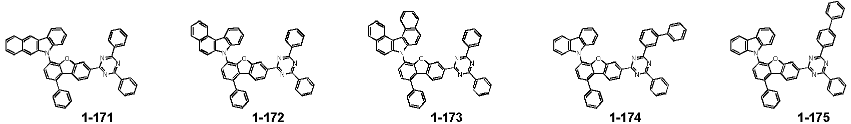

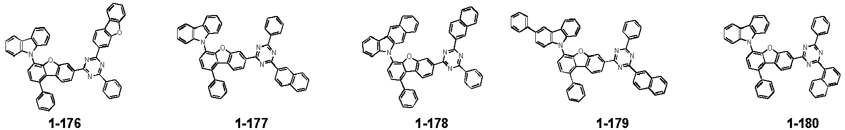

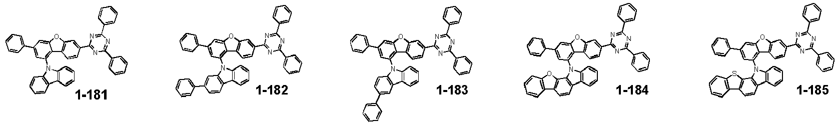

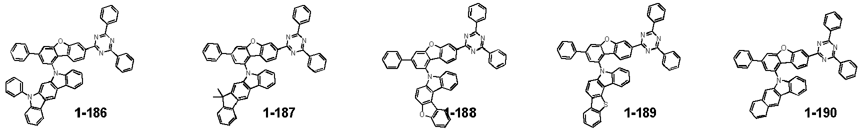

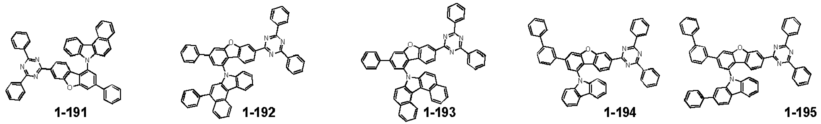

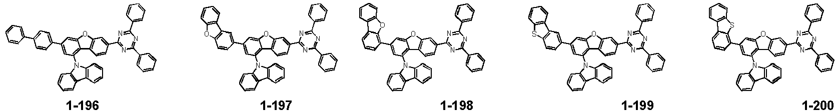

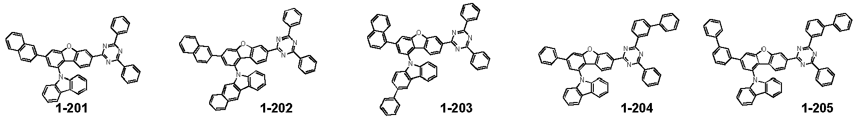

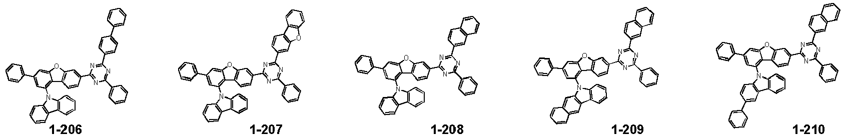

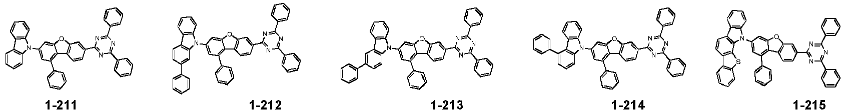

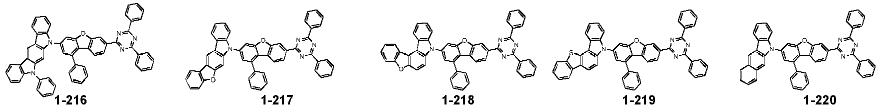

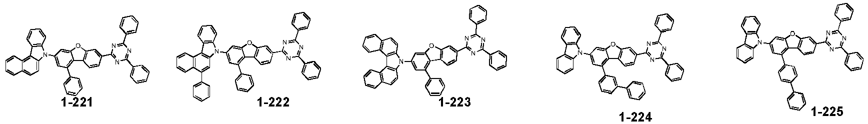

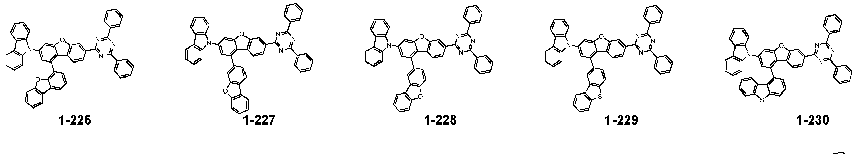

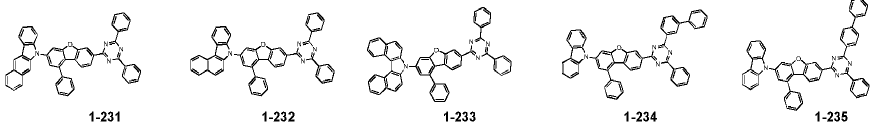

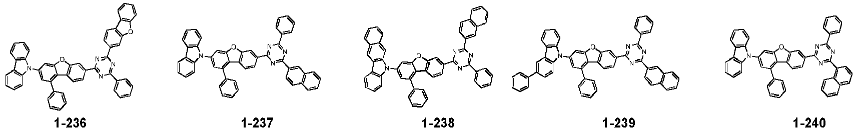

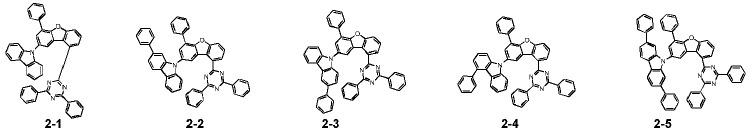

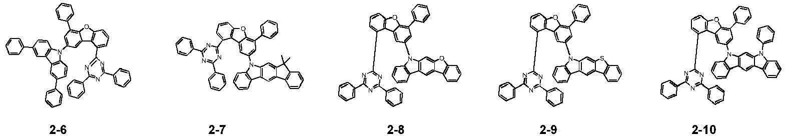

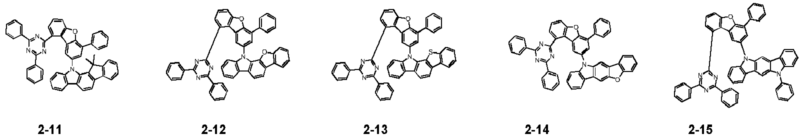

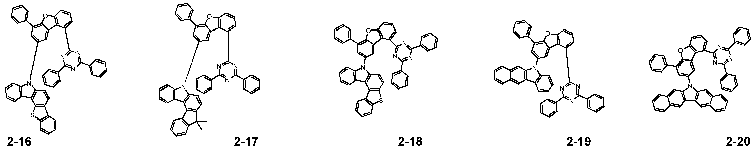

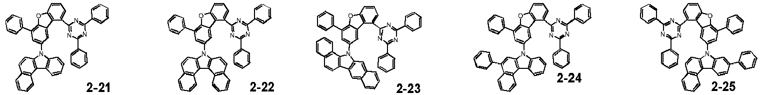

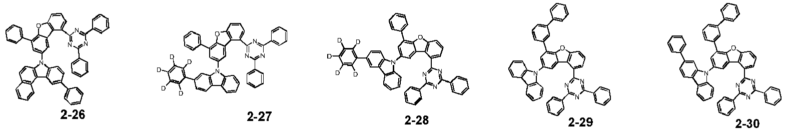

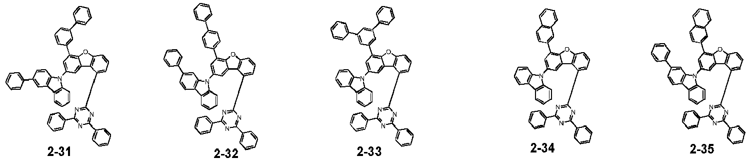

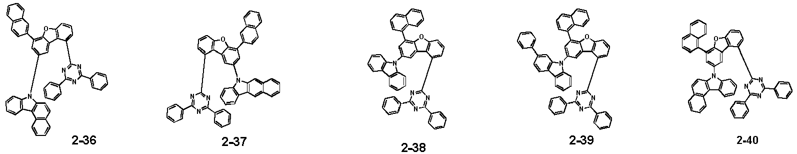

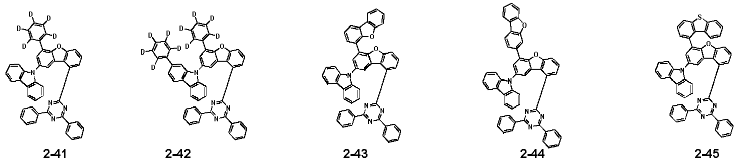

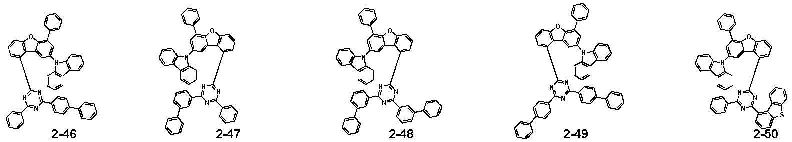

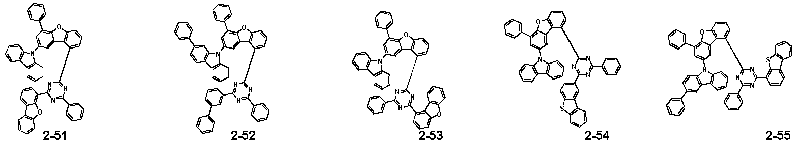

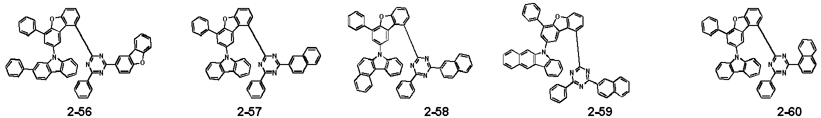

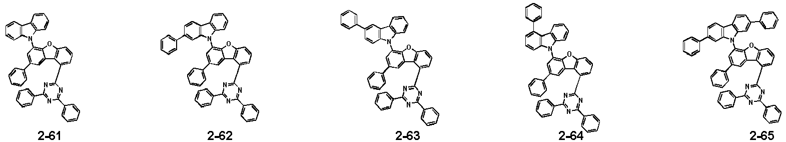

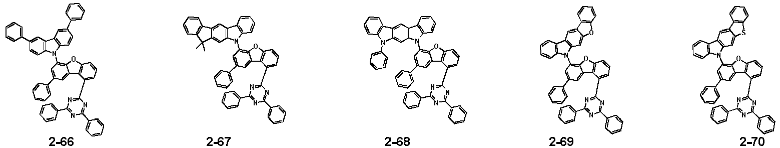

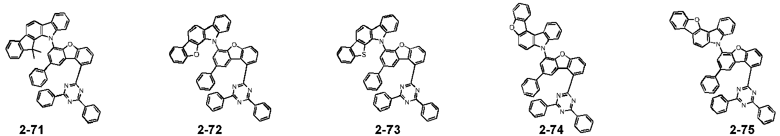

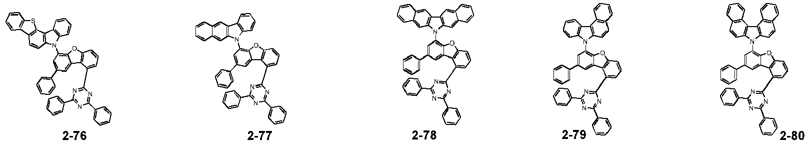

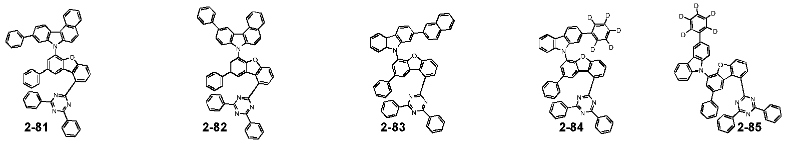

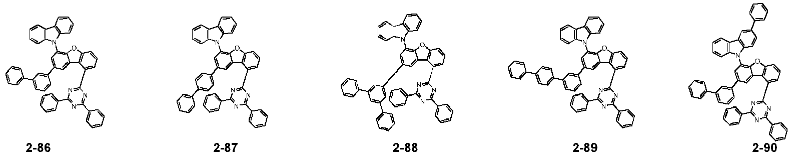

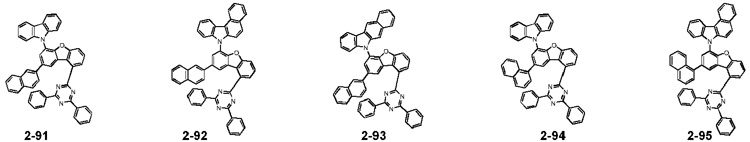

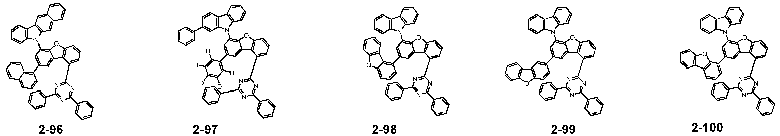

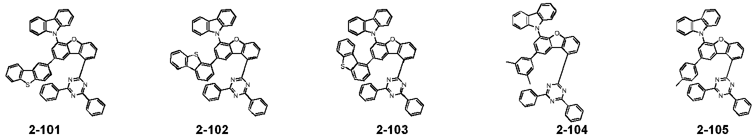

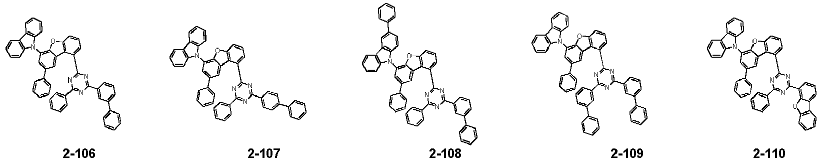

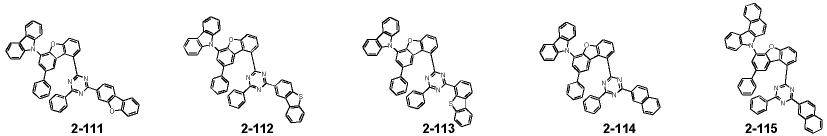

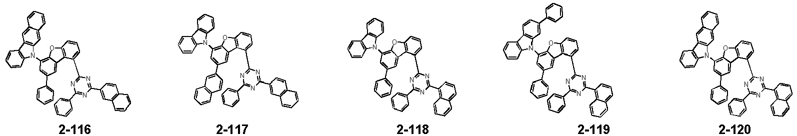

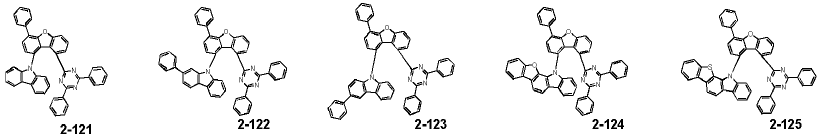

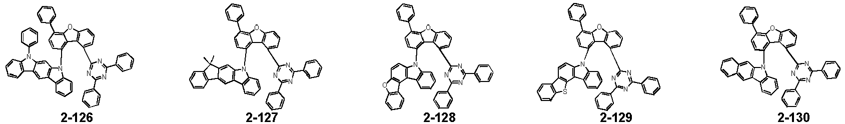

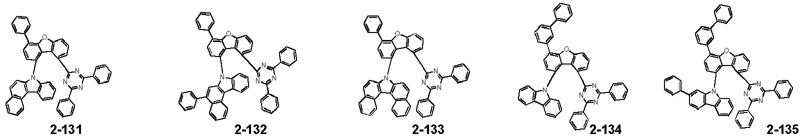

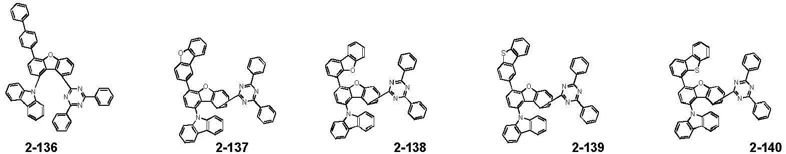

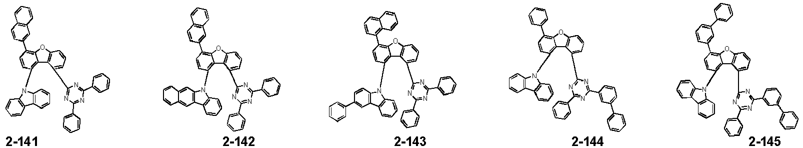

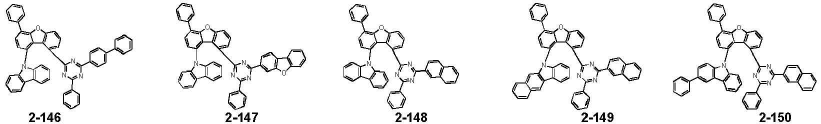

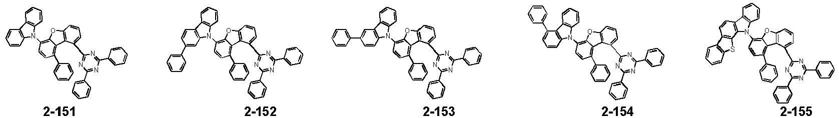

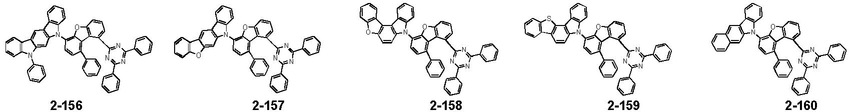

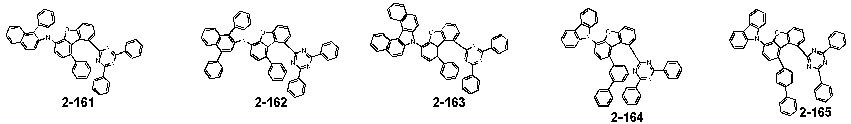

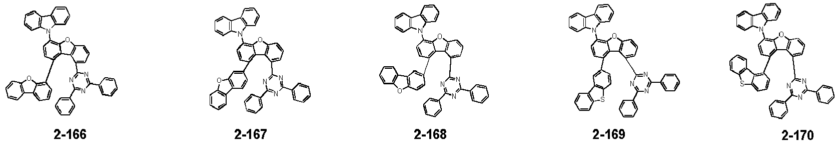

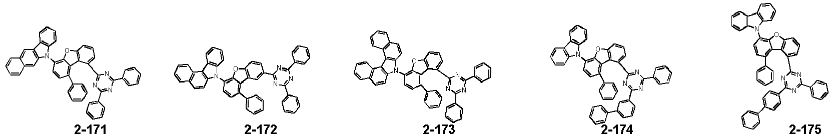

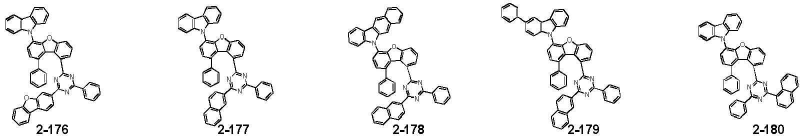

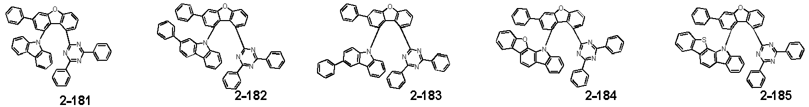

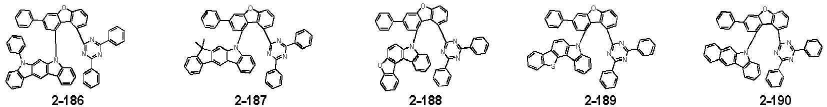

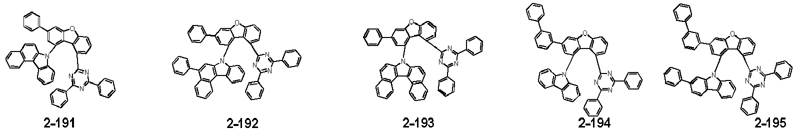

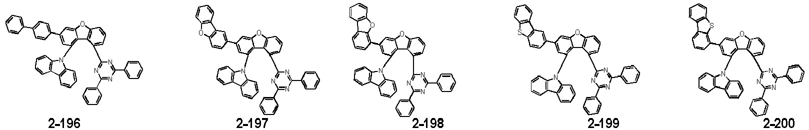

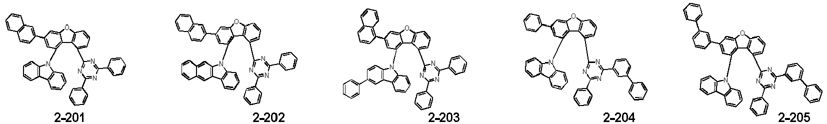

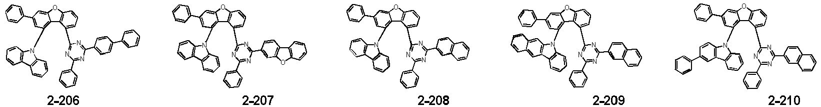

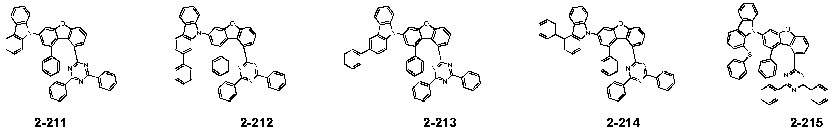

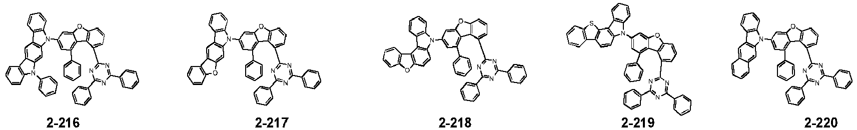

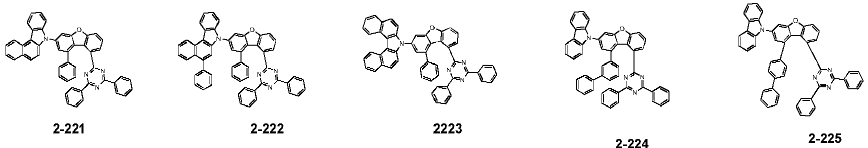

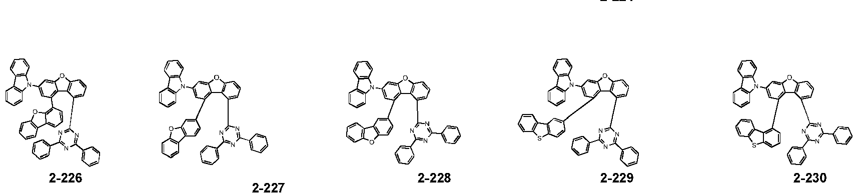

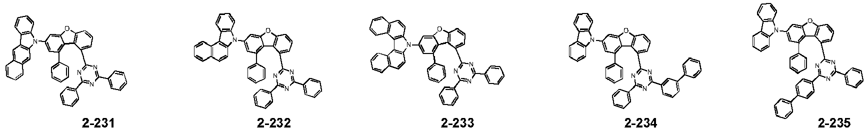

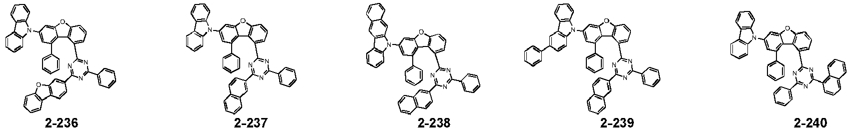

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

In addition, one embodiment of the present application provides an organic light emitting device comprising a first electrode; a second electrode provided opposite to the first electrode; and one or more organic material layers provided between the first electrode and the second electrode, wherein one or more layers of the organic material layers comprise the heterocyclic compound according to Chemical Formula 1.

Another embodiment of the present application provides an organic light emitting device comprising a first electrode; a second electrode provided opposite to the first electrode; and one or more organic material layers provided between the first electrode and the second electrode, wherein one or more layers of the organic material layers comprise one heterocyclic compound according to Chemical Formula 1.

Specific descriptions on the heterocyclic compound represented by Chemical Formula 1 are the same as the descriptions provided above.

In one embodiment of the present application, the first electrode may be an anode, and the second electrode may be a cathode.

In another embodiment, the first electrode may be a cathode, and the second electrode may be an anode.

In one embodiment of the present application, the organic light emitting device may be a blue organic light emitting device, and the heterocyclic compound according to Chemical Formula 1 may be used as a material of the blue organic light emitting device. For example, the heterocyclic compound according to Chemical Formula 1 may be included in a host material of a blue light emitting layer of the blue organic light emitting device.

In one embodiment of the present application, the organic light emitting device may be a green organic light emitting device, and the heterocyclic compound according to Chemical Formula 1 may be used as a material of the green organic light emitting device. For example, the heterocyclic compound according to Chemical Formula 1 may be included in a host material of a green light emitting layer of the green organic light emitting device.

In one embodiment of the present application, the organic light emitting device may be a red organic light emitting device, and the heterocyclic compound according to Chemical Formula 1 may be used as a material of the red organic light emitting device. For example, the heterocyclic compound according to Chemical Formula 1 may be included in a host material of a red light emitting layer of the red organic light emitting device.

The organic light emitting device of the present disclosure may be manufactured using common organic light emitting device manufacturing methods and materials except that one or more of the organic material layers are formed using the heterocyclic compound described above.

The heterocyclic compound may be formed into an organic material layer through a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present disclosure may be formed in a single layer structure, but may be formed in a multilayer structure in which two or more organic material layers are laminated. For example, the organic light emitting device of the present disclosure may have a structure comprising a hole injection layer, a hole transfer layer, a light emitting layer, an electron transfer layer, an electron injection layer and the like as the organic material layer. However, the structure of the organic light emitting device is not limited thereto, and may comprise a smaller number of organic material layers.

In the organic light emitting device of the present disclosure, the organic material layer may comprise a light emitting layer, and the light emitting layer may comprise the heterocyclic compound.

In another organic light emitting device, the organic material layer comprises a light emitting layer, the light emitting layer comprises a host material, and the host material may comprise the heterocyclic compound.

As another example, the organic material layer comprising the heterocyclic compound comprises the heterocyclic compound represented by Chemical Formula 1 as a host, and an iridiumbased dopant may be used therewith.

In the organic light emitting device of the present disclosure, the organic material layer comprises an electron injection layer or an electron transfer layer, and the electron transfer layer or the electron injection layer may comprise the heterocyclic compound.

In another organic light emitting device, the organic material layer comprises an electron blocking layer or a hole blocking layer, and the electron blocking layer or the hole blocking layer may comprise the heterocyclic compound.

The organic light emitting device of the present disclosure may further comprise one, two or more layers selected from the group consisting of a light emitting layer, a hole injection layer, a hole transfer layer, an electron injection layer, an electron transfer layer, an electron blocking layer and a hole blocking layer.

FIG 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

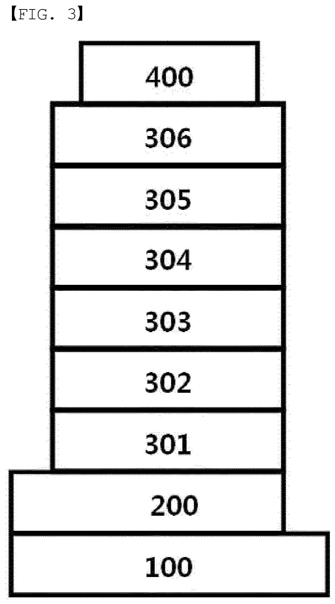

FIG. 3 illustrates a case of the organic material layer being a multilayer. The organic light emitting device according to FIG. 3 comprises a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be included, and other necessary functional layers may be further added.

The organic material layer comprising the compound of Chemical Formula 1 may further comprise other materials as necessary.

In the organic light emitting device according to one embodiment of the present application, the organic material layer may further comprise a heterocyclic compound of the following Chemical Formula 2.

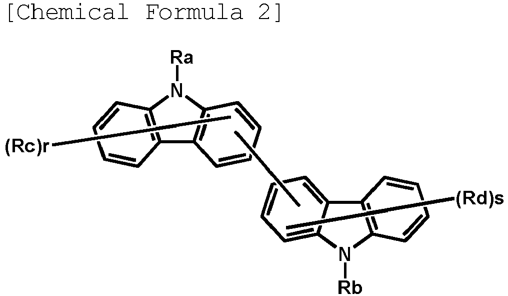

In Chemical Formula 2,
Ra and Rb are the same as or different from each other, and each independently a substituted or unsubstituted C6 to C60 aryl group; or a substituted or unsubstituted C2 to C60 heteroaryl group,
Rc and Rd are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; and a substituted or unsubstituted amine group, and
r and s are an integer of 0 to 7.

Effects of more superior efficiency and lifetime are obtained when comprising the compound of Chemical Formula 1 and the compound of Chemical Formula 2 at the same time in the organic material layer of the organic light emitting device. Such results may lead to a forecast that an exciplex phenomenon occurs when comprising the two compounds at the same time.

The exciplex phenomenon is a phenomenon of releasing energy having sizes of a donor (p-host) HOMO level and an acceptor (n-host) LUMO level due to electron exchanges between two molecules. When the exciplex phenomenon occurs between two molecules, reverse intersystem crossing (RISC) occurs, and as a result, internal quantum efficiency of fluorescence may increase up to 100%. When a donor (p-host) having a favorable hole transfer ability and an acceptor (n-host) having a favorable electron transfer ability are used as a host of a light emitting layer, holes are injected to the p-host and electrons are injected to the n-host, and therefore, a driving voltage may be lowered, which resultantly helps with enhancement in the lifetime.

In one embodiment of the present application, Chemical Formula 2 may be represented by any one of the following Chemical Formulae 5 to 12.

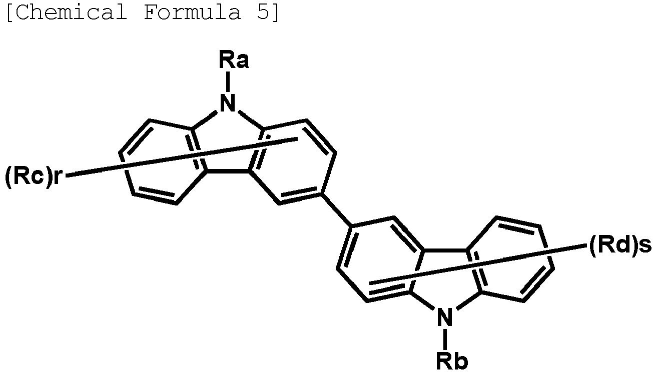

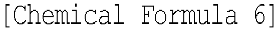

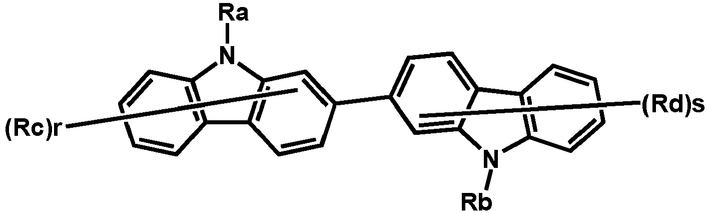

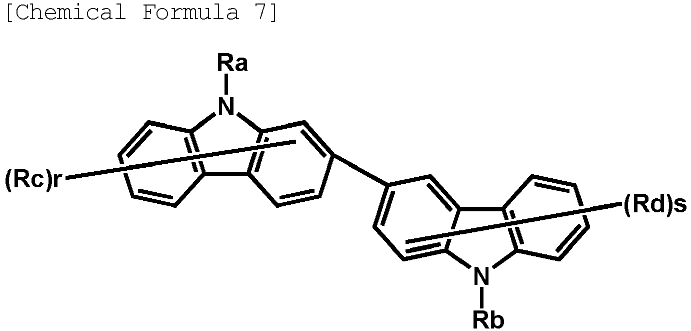

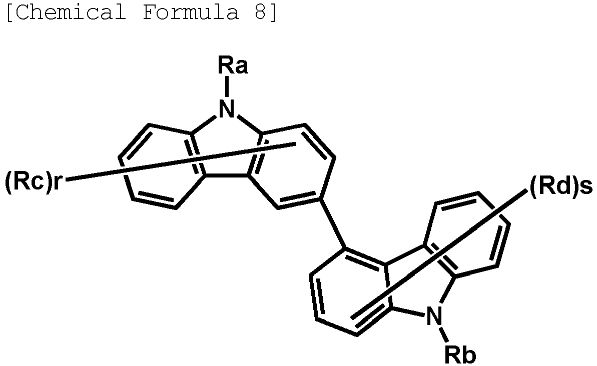

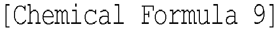

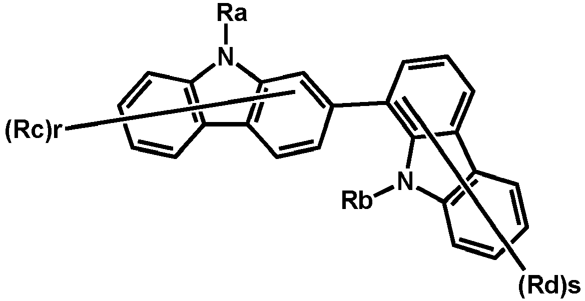

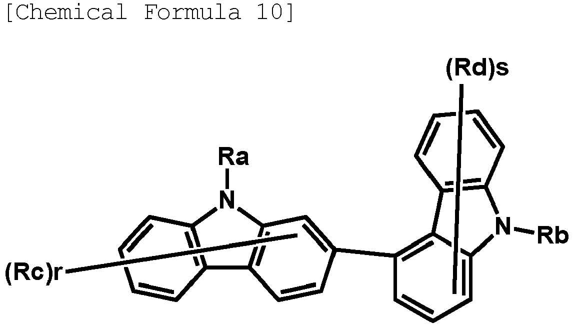

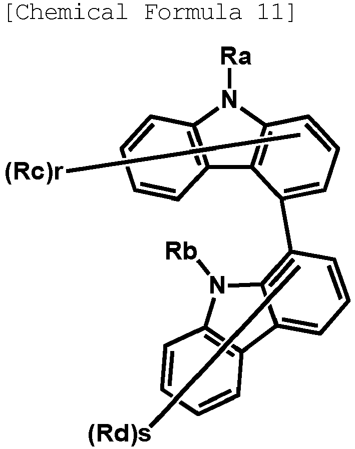

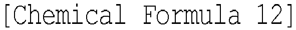

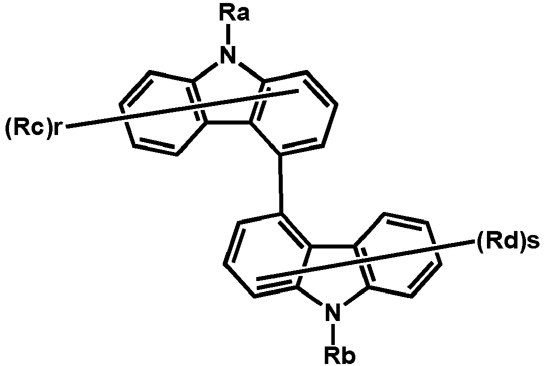

In Chemical Formulae 5 to 12,
Ra, Rb, Rc, Rd, r and s have the same definitions as in Chemical Formula 2.

In the organic light emitting device according to one embodiment of the present application, Ra and Rb of Chemical Formula 2 are the same as or different from each other, and may be each independently a substituted or unsubstituted C6 to C60 aryl group; or a substituted or unsubstituted C6 to C60 heteroaryl group.

In the organic light emitting device according to another embodiment, Ra and Rb of Chemical Formula 2 are the same as or different from each other, and may be each independently a substituted or unsubstituted C6 to C40 aryl group; or a substituted or unsubstituted C6 to C40 heteroaryl group.

In the organic light emitting device according to another embodiment, Ra and Rb of Chemical Formula 2 are the same as or different from each other, and may be each independently a C6 to C40 aryl group unsubstituted or substituted with one or more substituents selected from the group consisting of a C1 to C40 alkyl group, a C6 to C40 aryl group, -CN and -SiR101R102R103; or a C2 to C40 heteroaryl group unsubstituted or substituted with one or more substituents selected from the group consisting of a C6 to C40 aryl group and a C2 to C40 heteroaryl group.

In the organic light emitting device according to another embodiment, Ra and Rb of Chemical Formula 2 are the same as or different from each other, and may be each independently a phenyl group unsubstituted or substituted with a phenyl group, -CN or -SiR101R102R103; a biphenyl group unsubstituted or substituted with a phenyl group; a naphthyl group; a fluorene group unsubstituted or substituted with a methyl group or a phenyl group; a spirobifluorene group; a dibenzothiophene group unsubstituted or substituted with one or more substituents selected from the group consisting of a phenyl group, a biphenyl group, a naphthyl group, a dimethylfluorene group, a dibenzothiophene group and a dibenzofuran group; or a triphenylene group.

In the organic light emitting device according to one embodiment of the present application, R101, R102 and R103 of Chemical Formula 2 may be a C6 to C20 monocyclic aryl group.

In the organic light emitting device according to one embodiment of the present application, R101, R102 and R103 of Chemical Formula 2 may be a phenyl group.

In one embodiment of the present application, Rc and Rd may be hydrogen.

In one embodiment of the present application, Chemical Formula 2 may be represented by any one of the following compounds, but is not limited thereto.

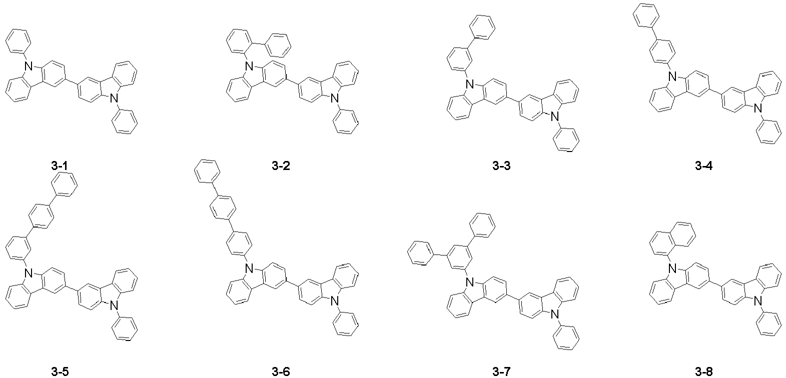

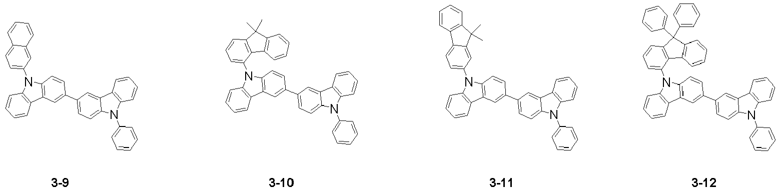

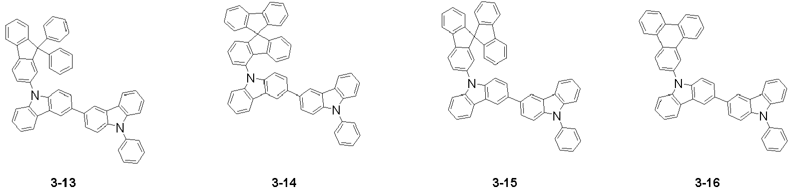

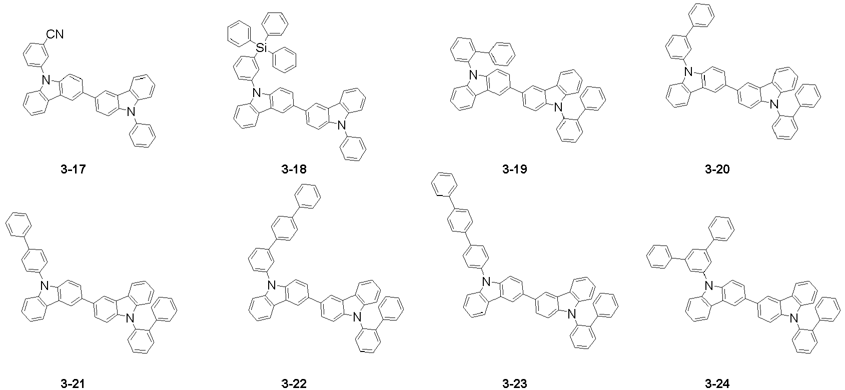

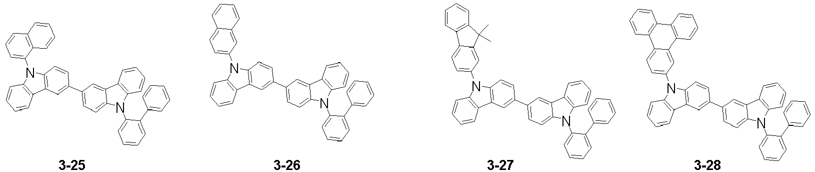

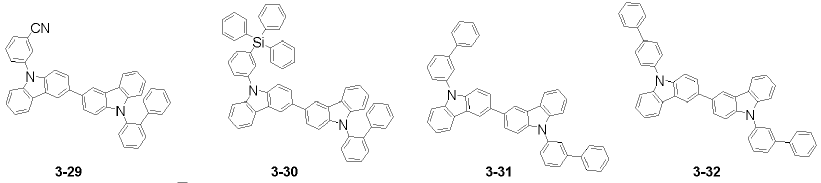

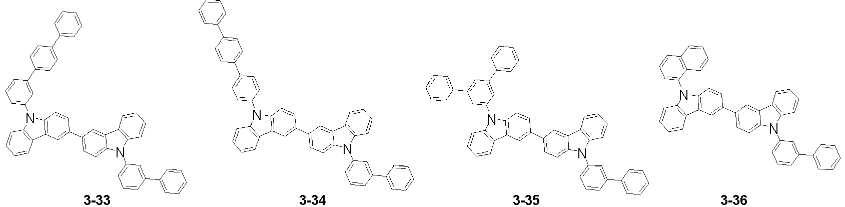

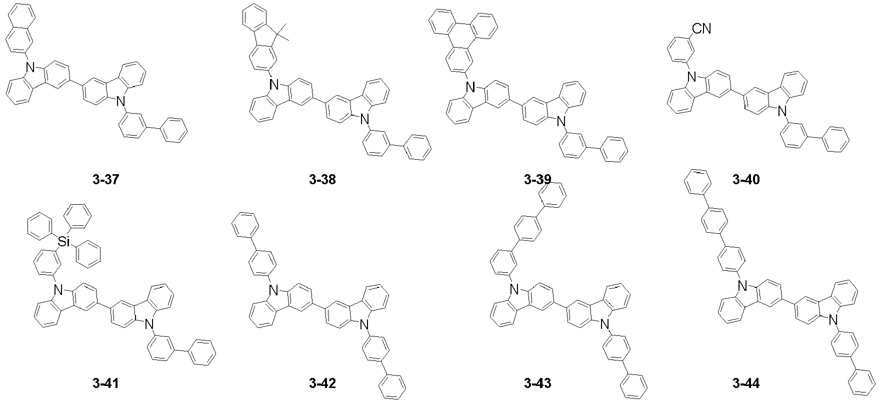

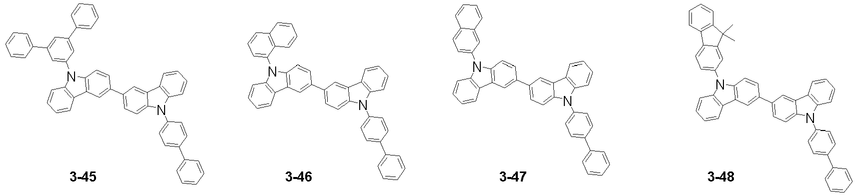

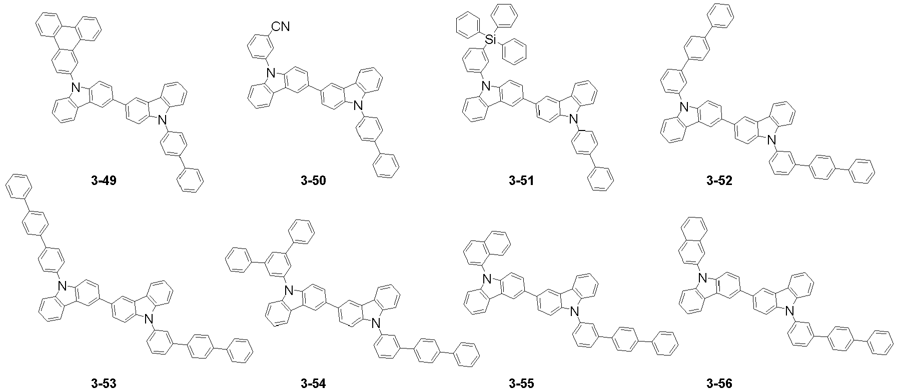

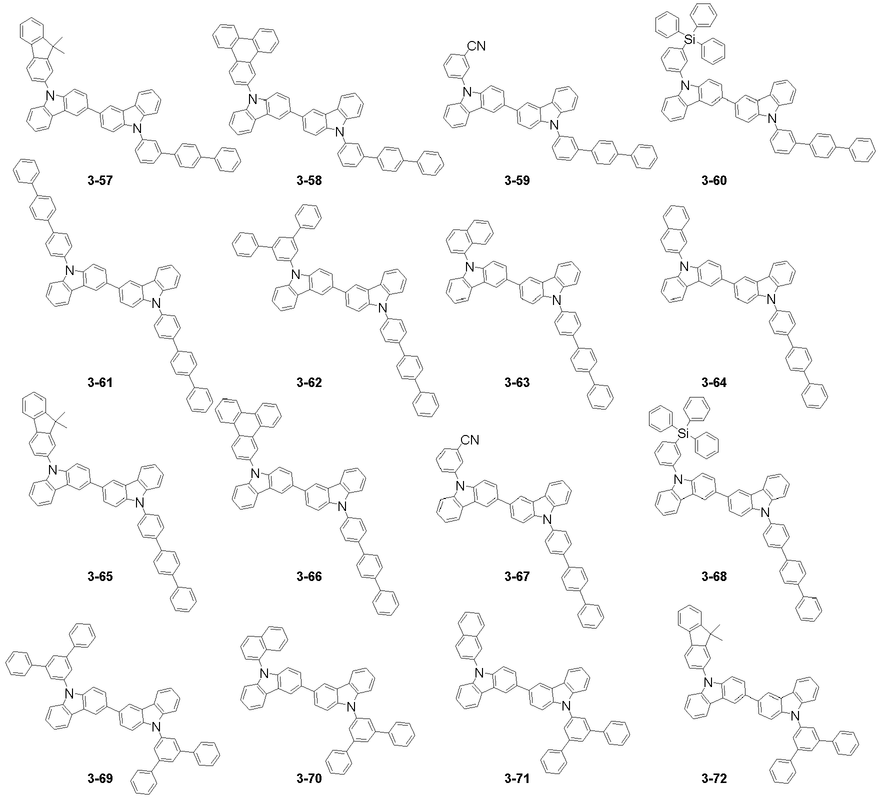

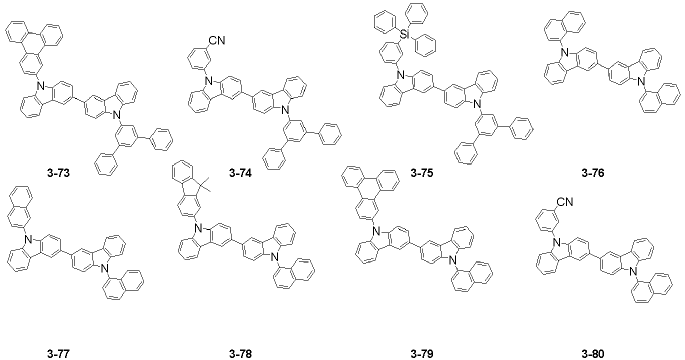

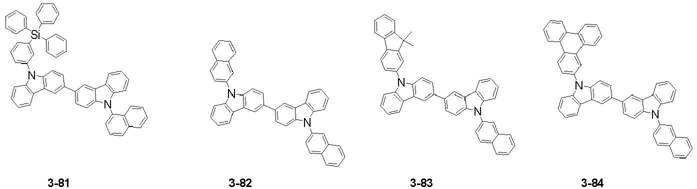

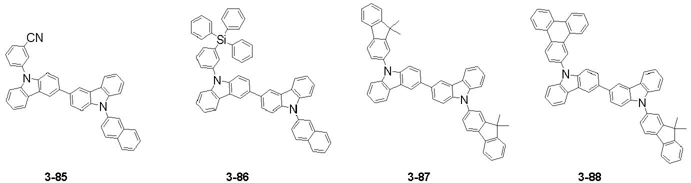

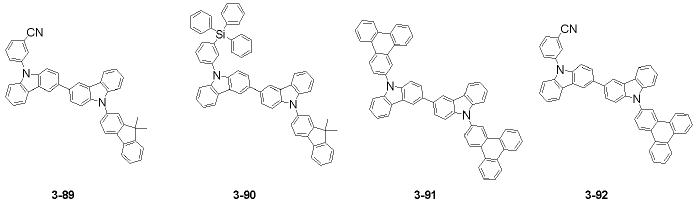

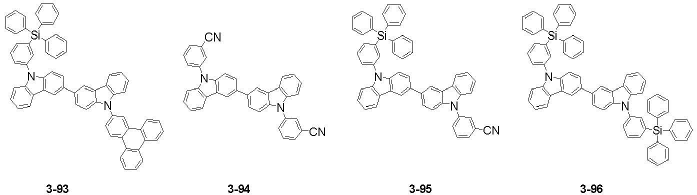

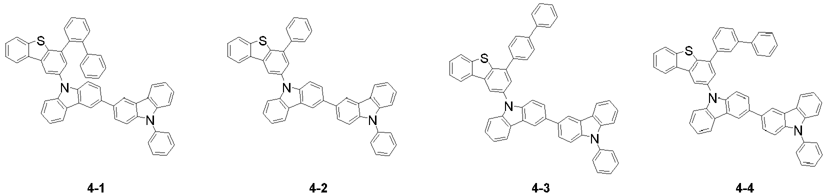

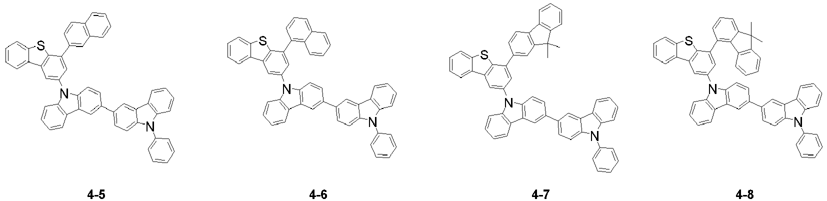

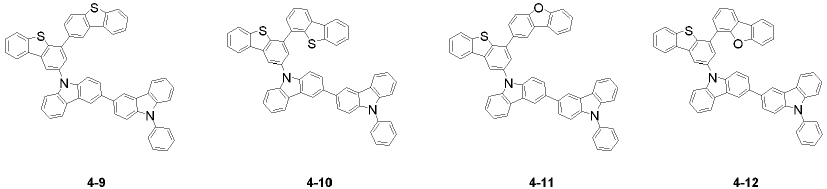

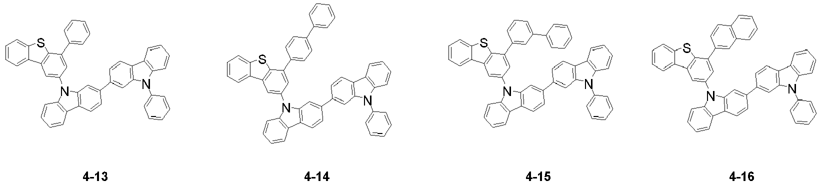

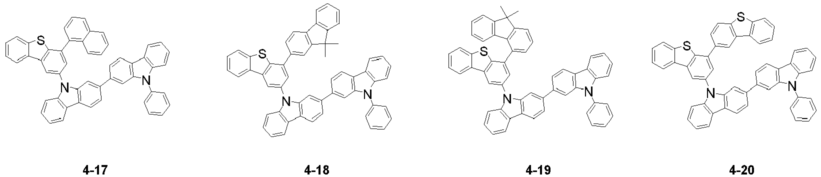

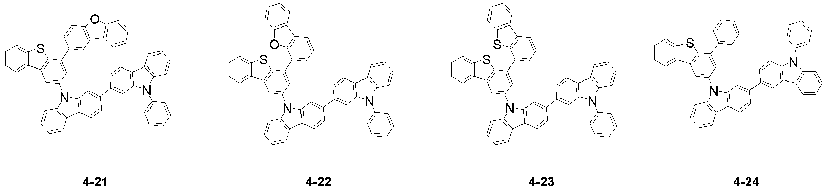

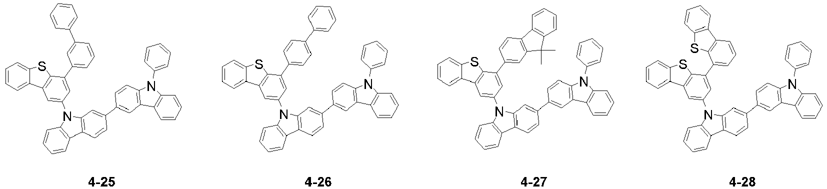

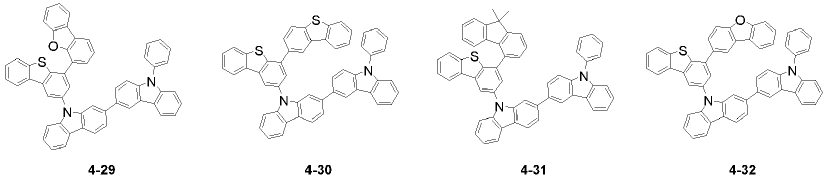

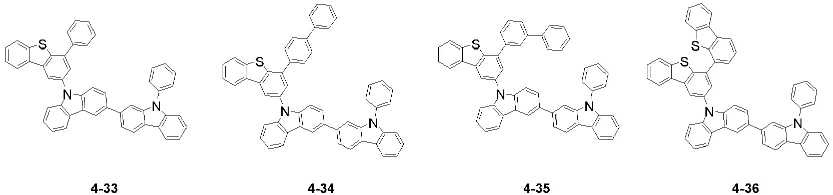

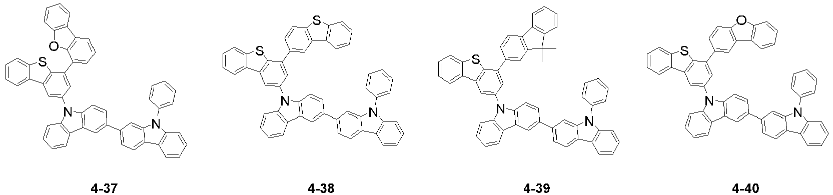

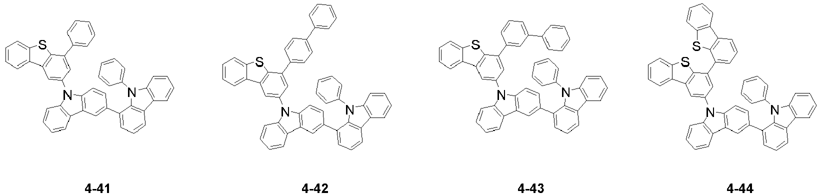

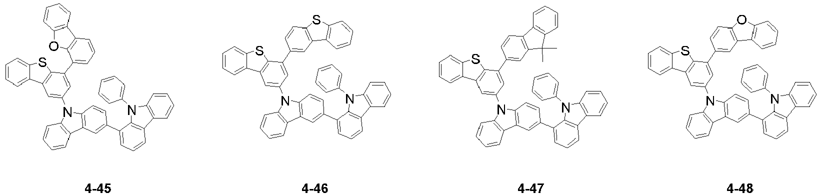

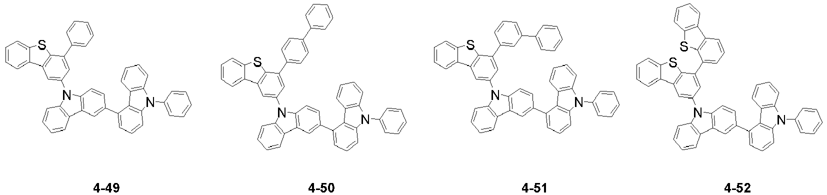

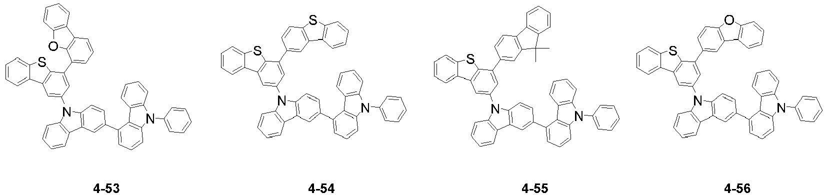

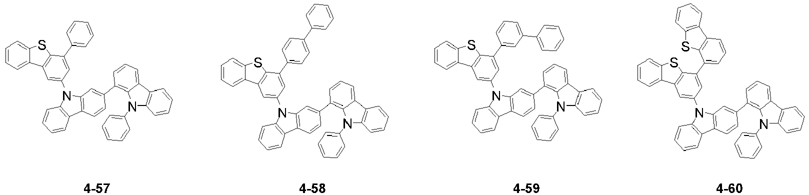

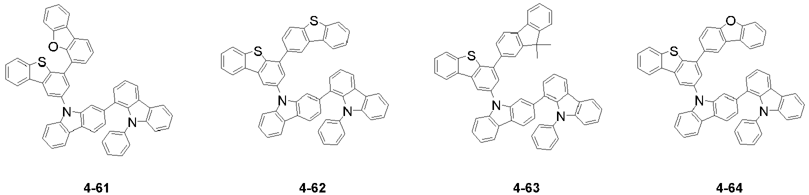

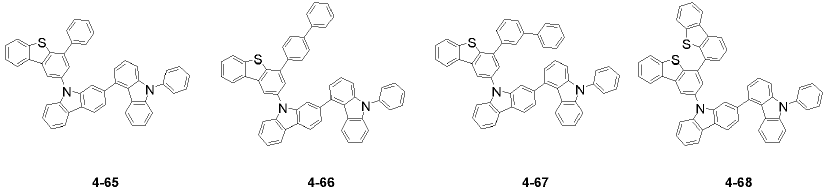

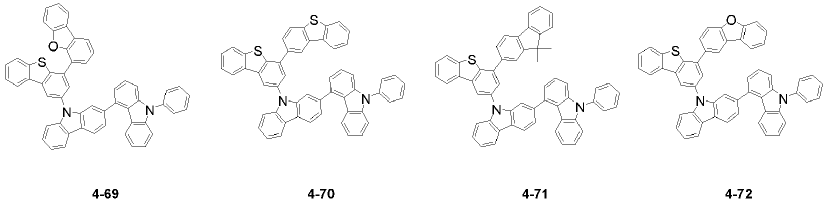

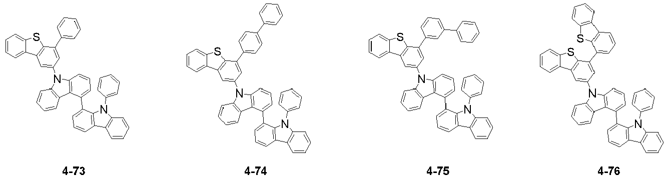

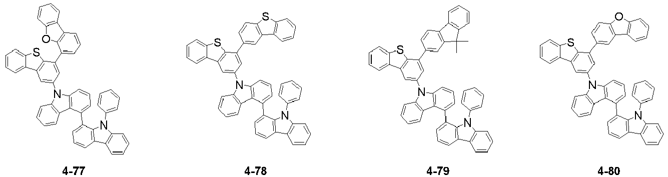

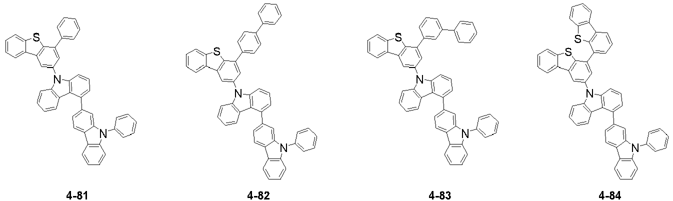

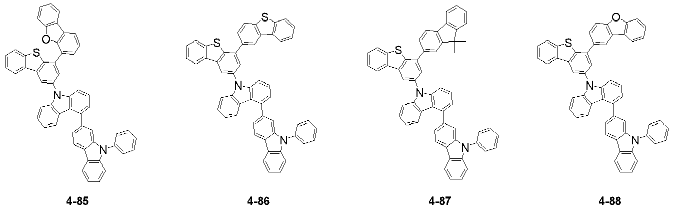

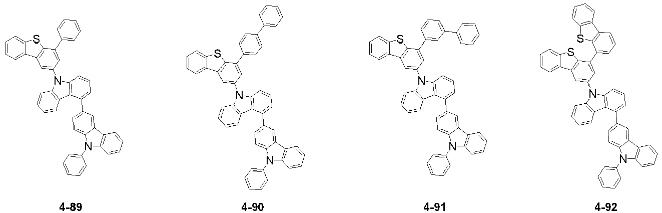

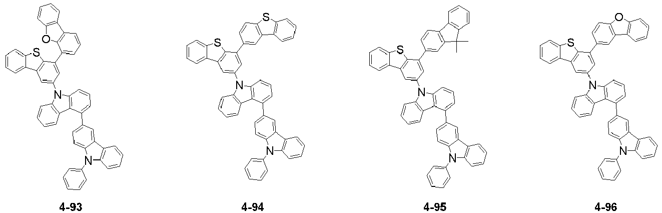

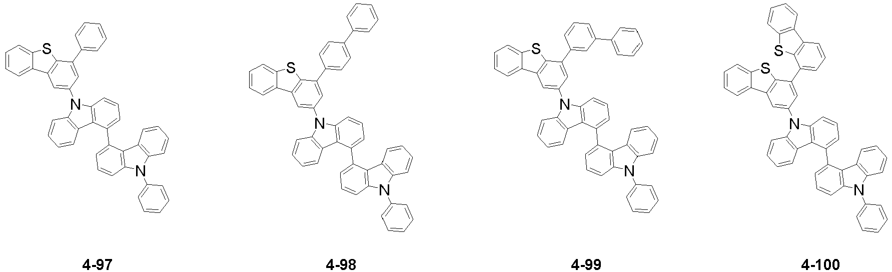

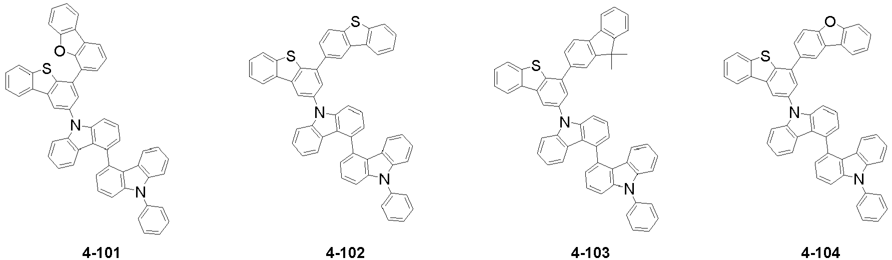

In the organic light emitting device according to one embodiment of the present application, Chemical Formula 2 may be included in a light emitting layer of the organic material layer.

In the organic light emitting device according to one embodiment of the present application, Chemical Formula 2 may be included in a light emitting layer of the organic material layer, and may be specifically used as a host material of the light emitting layer.

In one embodiment of the present application, the host material of the light emitting layer of the organic light emitting device may include the heterocyclic compound of Chemical Formula 1 and the heterocyclic compound of Chemical Formula 2 at the same time.

One embodiment of the present application provides a composition for an organic material layer of an organic light emitting device, the composition comprising the heterocyclic compound represented by Chemical Formula 1 and the heterocyclic compound represented by Chemical Formula 2.

In the composition, the heterocyclic compound represented by Chemical Formula 1: the heterocyclic compound represented by Chemical Formula 2 may have a weight ratio of 1:10 to 10:1, and the weight ratio may be from 1:8 to 8:1, 1:5 to 5:1 or 1:2 to 2:1, but is not limited thereto.

One embodiment of the present application provides a method for manufacturing an organic light emitting device, the method comprising preparing a substrate; forming a first electrode on the substrate; forming one or more organic material layers on the first electrode; and forming a second electrode on the organic material layer, wherein the forming of organic material layers comprises forming one or more organic material layers using the composition for an organic material layer according to one embodiment of the present application.

In the method for manufacturing an organic light emitting device provided in one embodiment of the present application, the forming of organic material layers is forming the heterocyclic compound represented by Chemical Formula 1 using a thermal vacuum deposition method.

In the method for manufacturing an organic light emitting device provided in one embodiment of the present application, the forming of organic material layers is forming two types of the heterocyclic compound represented by Chemical Formula 1 and the heterocyclic compound represented by Chemical Formula 2 using a thermal vacuum deposition method after pre-mixing.

The pre-mixing means first mixing two types of the heterocyclic compound represented by Chemical Formula 1 and the heterocyclic compound represented by Chemical Formula 2 in one source of supply before depositing on the organic material layer.

The premixed material may be referred to as the composition for an organic material layer according to one embodiment of the present application.

In the organic light emitting device according to one embodiment of the present application, materials other than the compound of Chemical Formula 1 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material comprise metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material comprise metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4ethylenedioxythiophene)/poly(4-styrenesulfonate), polyaniline/camphor sulfonic acid or polyaniline/poly(4-styrene-sulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. Herein, two or more light emitting materials may be used by being deposited as individual sources of supply or by being premixed and deposited as one source of supply. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

When mixing light emitting material hosts, same series hosts may be mixed, or different series hosts may be mixed. For example, any two or more types of materials among n-type host materials or p-type host materials may be selected and used as a host material of a light emitting layer.

The organic light emitting device according to one embodiment of the present application may be a top-emission type, a bottom-emission type or a dual-emission type depending on the materials used.

The heterocyclic compound according to one embodiment of the present application may also be used in an organic electronic device comprising an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Preparation Example 1> Synthesis of Intermediate 1-1-iv

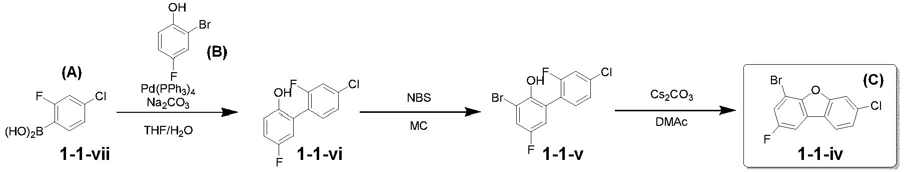

### Preparation of Compound 1-1-vi

In a one-neck round bottom flask (one neck r.b.f), (4-chloro-2-fluorophenyl)boronic acid (100 g/573.49 mmol), 2-bromo-4-fluorophenol (131.4 g/688.19 mmol), Pd(PPh₃)₄ (33.13 g/28.67 mmol) , Na₂CO₃ (121.57 g/1146.99 mmol) and THF/H₂O (1200 ml/200 ml) were introduced, and stirred under reflux for 10 hours at 70°C. After the reaction was finished, the result was extracted with dichloromethane, and then dried with MgSO₄. The result was column purified, and the filtrate was concentrated to obtain Compound 1-1-vi (138 g, 100%).

### Preparation of Compound 1-1-v

In a one-neck round bottom flask (one neck r.b.f), Compound 1-1-vi (138 g/573.49 mmol) and dichloromethane (1400 ml) were introduced and stirred at room temperature, and, while adding NBS (107.18 g/602.16 mmol) dropwise thereto, the result was stirred for 1 hour. The reaction was terminated using distilled water, and the organic layer was separated, extracted, and then dried with MgSO₄. The result was column purified, and the filtrate was concentrated to obtain Compound 1-1-v (134.2 g, 73%).

### Preparation of Compound 1-1-iv

In a one-neck round bottom flask (one neck r.b.f), Compound 1-1-v (134.2 g/420.00 mmol), Cs₂CO₃ (273.68 g/839.99 mmol) and DMAc (1400 ml) were introduced, and stirred under reflux for 3 hours at 170°C. The result was cooled to room temperature (25°C), then filtered to remove the salts, and then the solvent of the filtrate was removed. The result was washed with distilled water, then extracted with dichloromethane, and dried with MgSO₄. The result was column purified, and the filtrate was concentrated to obtain Intermediate Compound 1-1-iv (45 g, 40%).

The following target Compound C was prepared in the same manner as in Preparation of Compound 1-1-vi of Preparation Example 1 except that A and B of the following Table 1 were used as the intermediates.

**[Table 1]**

| Compound | A | B | C | Yield (Overall Yield) |
|---|---|---|---|---|
| 1-1-vi | 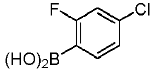 | 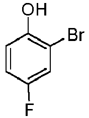 | 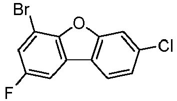 | 43% |
| 1-61-vi | 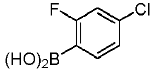 | 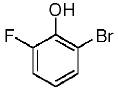 | 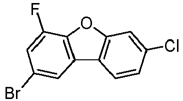 | 61% |
| 2-1-vi | 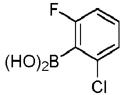 | 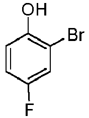 | 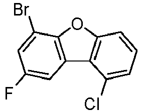 | 37% |
| 2-61-vi | 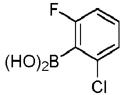 | 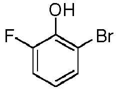 | 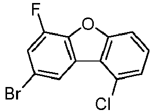 | 55% |

### <Preparation Example 2> Synthesis of Intermediate 1-121-iv

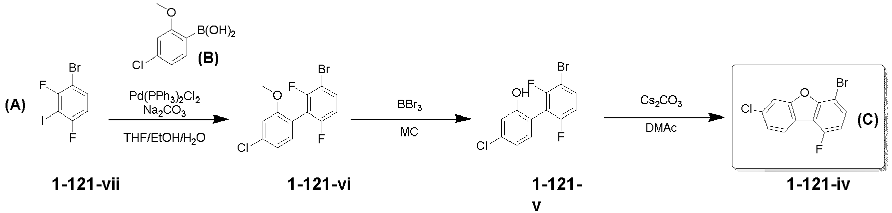

### Preparation of Compound 1-121-vi

In a one-neck round bottom flask (one neck r.b.f), 1-bromo-2,4-difluoro-3-iodobenzene (40 g/125.44 mmol), (4-chloro-2-methoxyphenyl)boronic acid (30.40 g/163.07 mmol), Pd(PPh₃)₂Cl₂ (7.04 g/10.03 mmol), Na₂CO₃ (26.59 g/250.87 mmol) and THF/H₂O (400 ml/120 ml) were introduced, and stirred under reflux for 10 hours at 70°C. After the reaction was finished, the result was extracted with dichloromethane, and then dried with MgSO₄. The result was column purified, and the filtrate was concentrated to obtain Compound 1-121-vi (27.2 g, 65%).

### Preparation of Compound 1-121-v

In a one-neck round bottom flask (one neck r.b.f), Compound 1-121-vi (27.20 g/81.54 mmol) and dichloromethane (300 ml) were introduced and stirred at room temperature, and, while adding BBr₃ (40.85 g/163.08 mmol) dropwise thereto, the result was stirred for 1 hour. The reaction was terminated using distilled water, and the result was filtered to remove the salts. Then, the organic layer was separated, extracted, and then dried with MgSO₄. The result was column purified, and the filtrate was concentrated to obtain Compound 1-121-v (24 g, 92%) .

### Preparation of Compound 1-121-iv

In a one-neck round bottom flask (one neck r.b.f), Compound 1-121-v (24.0 g/75.02 mmol), Cs₂CO₃ (48.87 g/150.04 mmol) and DMAc (250 ml) were introduced, and stirred under reflux for 3 hours at 170°C. The result was cooled to room temperature (25°C), then filtered to remove the salts, and then the solvent of the filtrate was removed. The result was washed with distilled water, then extracted with dichloromethane, and dried with MgSO₄. The result was column purified, and the filtrate was concentrated to obtain Compound 1-121-iv (20 g, 89%) .

The following target Compound C was prepared in the same manner as in Preparation of Compound 1-121-vi of Preparation Example 2 except that A and B of the following Table 2 were used as the intermediates.

**[Table 2]**

| Compound | A | B | C | Yield (Overall yield) |
|---|---|---|---|---|
| 1-121-vi | 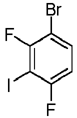 | 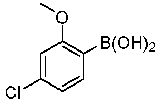 | 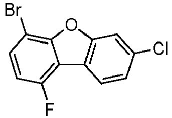 | 53% |
| 1-151-vi | 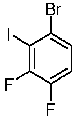 | 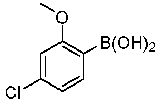 | 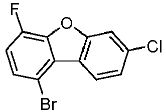 | 39% |
| 1-181-vi | 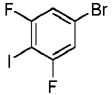 | 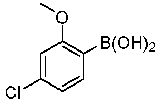 | 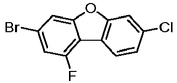 | 60% |
| 1-211-vi | 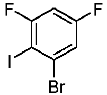 | 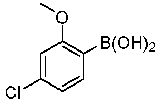 | 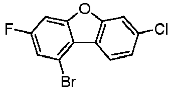 | 41% |
| 2-121-vi | 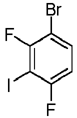 | 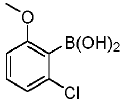 | 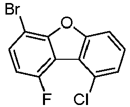 | 45% |
| 2-151-vi | 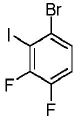 | 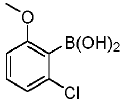 | 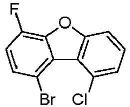 | 33% |
| 2-181-vi | 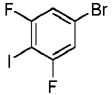 | 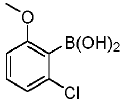 | 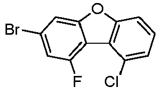 | 55% |
| 2-211-vi | 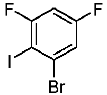 | 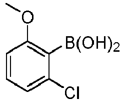 | 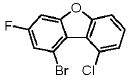 | 39% |

### <Preparation Example 3> Synthesis of Compound 1-1

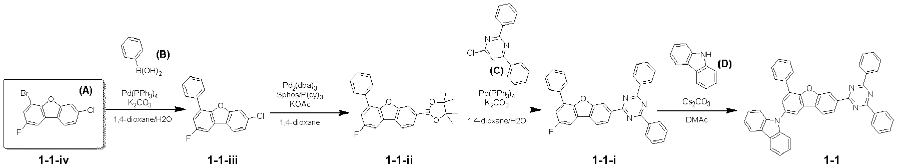

### Preparation of Compound 1-1-iii

In a one-neck round bottom flask (one neck r.b.f), Compound 1-1-iv (20 g/66.77 mmol), phenylboronic acid (A) (8.96 g/73.45 mmol), Pd (PPh₃)₄ (3.86 g/3.34 mmol), K₂CO₃ (18.46 g/133.55 mmol) and 1,4-dioxane/H₂O (200 ml/40 ml) were introduced, and stirred under reflux for 3 hours at 110°C. The organic layer was extracted with dichloromethane, and then dried with MgSO₄. The result was column purified, and the filtrate was concentrated to obtain Compound 1-1-iii (14 g, 71%) .

### Preparation of Compound 1-1-ii

In a one-neck round bottom flask (one neck r.b.f), Compound 1-1-iii (12 g/40.44 mmol), bispinacolatodiboron (17.46 g/68.75 mmol), Pd₂(dba)₃ (3.70 g/4.04 mmol), P(cy)₃ (3.40 g/12.13 mmol), KOAc (9.92 g/101.10 mmol) and 1,4-dioxane (120 ml) were introduced, and stirred under reflux for 1 hour at 110°C. The reaction was terminated using distilled water, and the organic layer was extracted with dichloromethane and the dried with MgSO₄. The result was column purified, and the filtrate was concentrated to obtain Compound 1-1-ii (14.6 g, 93%) .

### Preparation of Compound 1-1-i

In a one-neck round bottom flask (one neck r.b.f), Compound 1-1-ii (14,6 g/37.60 mmol), 2-chloro-4,6-diphenyl-1,3,5-triazine (B) (11.08 g/41.37 mmol), Pd(PPh₃)₄ (2.17 g/1.88 mmol), K₂CO₃ (10.39 g/75.21 mmol) and 1,4-dioxane/H₂O (150 ml/30 ml) were introduced, and stirred under reflux for 6 hours at 110°C. The result was cooled to room temperature, then filtered, and the solids were stirred for 1 hour in distilled water and MeOH. Then, the result was dissolved in DCB, filtered using silica gel, and then the filtrate was concentrated to obtain Compound 1-1-i (14.7 g, 80%).

### Preparation of Compound 1-1

In a one-neck round bottom flask (one neck r.b.f), Compound 1-1-i (5.0 g/10.13 mmol), 9H-carbazole (C) (2.20 g/13.17 mmol), Cs₂CO₃ (13.20 g/40.53 mmol) and DMAc (50 ml) were introduced, and stirred under reflux for 72 hours at 170°C. The result was cooled to room temperature, then filtered, and the solids were stirred for 1 hour in distilled water and MeOH. Then, the result was dissolved in DCB, filtered using silica gel, and then the filtrate was concentrated to obtain Compound 1-1 (4.54 g, 76%).

The following target Compound E was prepared in the same manner as in Preparation Example 3 except that B, C and D were used as the substituents with A of the following Table 3 as the starting material.

**[Table 3]**

| Compound | A | B | C | D | E | Yield |
|---|---|---|---|---|---|---|
| 1-1 | 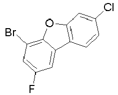 | 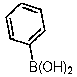 | 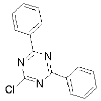 | 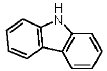 | 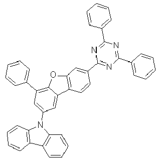 | 40% |
| 1-2 | 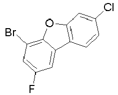 | 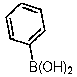 | 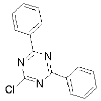 | 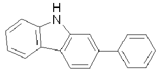 | 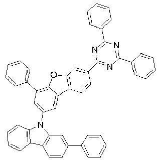 | 42% |
| 1-3 | 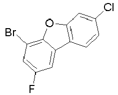 | 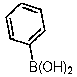 | 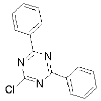 | 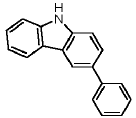 | 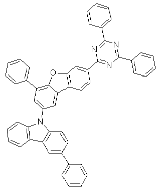 | 43% |
| 1-19 | 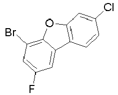 | 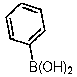 | 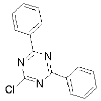 | 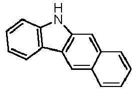 | 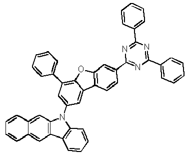 | 42% |
| 1-21 | 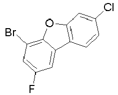 | 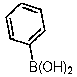 | 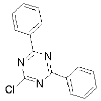 | 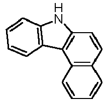 | 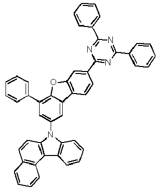 | 43% |
| 1-24 | 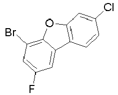 | 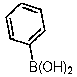 | 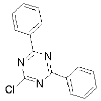 | 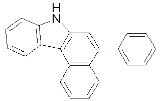 | 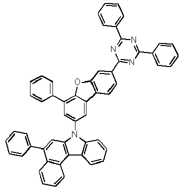 | 44% |
| 1-25 | 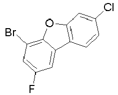 | 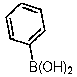 | 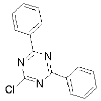 | 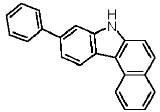 | 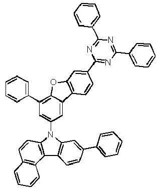 | 41% |
| 1-34 | 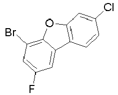 | 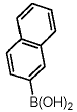 | 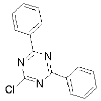 | 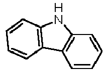 | 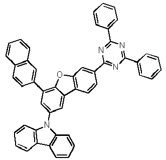 | 42% |
| 1-35 | 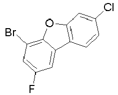 | 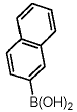 | 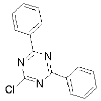 | 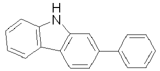 | 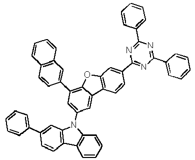 | 43% |
| 1-38 | 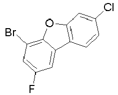 | 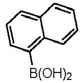 | 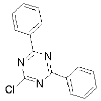 | 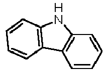 | 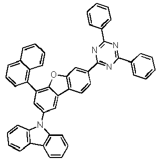 | 41% |
| 1-43 | 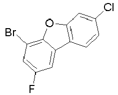 | 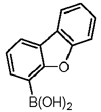 | 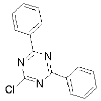 | 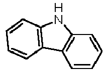 | 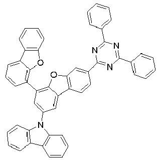 | 42% |
| 1-46 | 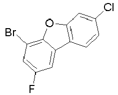 | 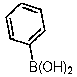 | 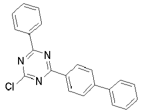 | 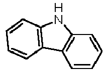 | 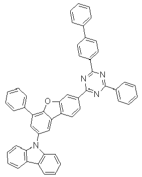 | 42% |
| 1-47 | 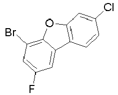 | 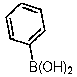 | 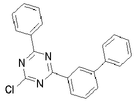 | 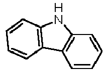 | 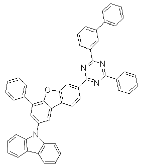 | 43% |
| 1-51 | 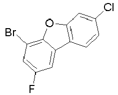 | 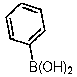 | 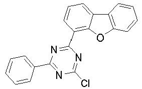 | 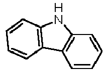 | 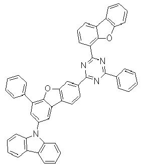 | 40% |
| 1-57 | 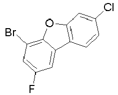 | 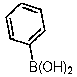 | 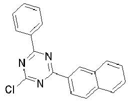 | 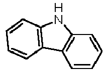 | 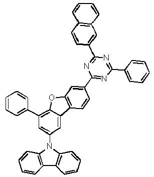 | 41% |
| 1-59 | 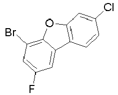 | 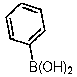 | 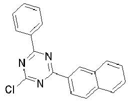 | 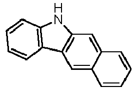 | 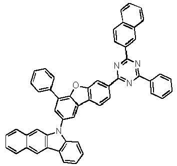 | 39% |
| 1-61 | 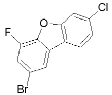 | 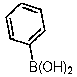 | 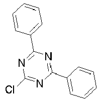 | 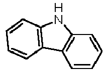 | 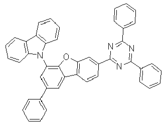 | 41% |
| 1-62 | 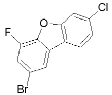 | 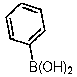 | 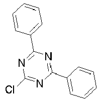 | 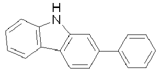 | 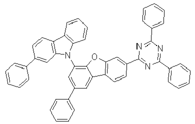 | 42% |
| 1-63 | 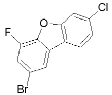 | 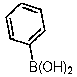 | 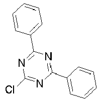 | 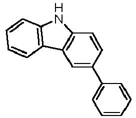 | 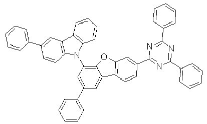 | 45% |
| 1-72 | 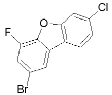 | 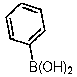 | 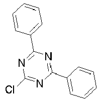 | 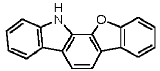 | 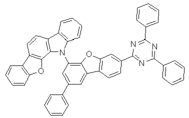 | 38% |
| 1-73 | 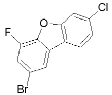 | 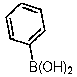 | 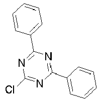 | 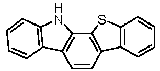 | 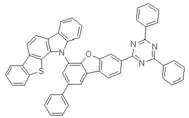 | 39% |
| 1-77 | 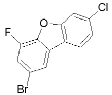 | 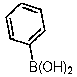 | 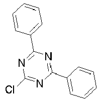 | 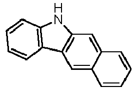 | 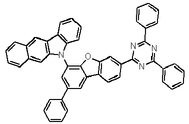 | 43% |
| 1-79 | 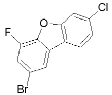 | 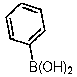 | 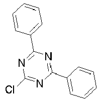 | 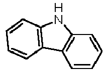 | 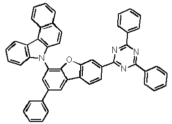 | 42% |
| 1-86 | 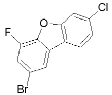 | 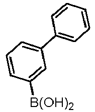 | 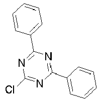 | 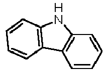 | 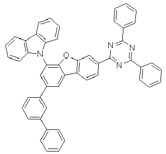 | 41% |
| 1-91 | 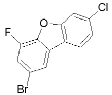 | 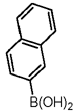 | 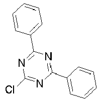 | 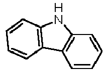 | 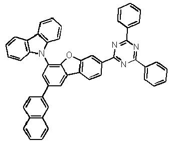 | 45% |
| 1-100 | 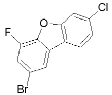 | 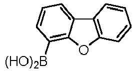 | 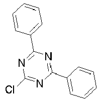 | 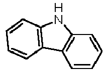 | 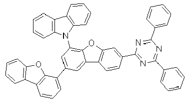 | 44% |
| 1-102 | 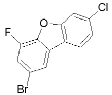 | 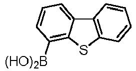 | 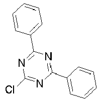 | 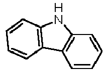 | 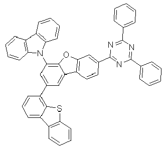 | 42% |
| 1-106 | 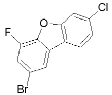 | 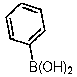 | 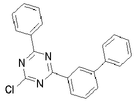 | 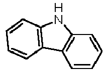 | 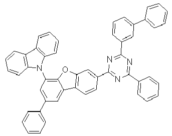 | 40% |
| 1-114 | 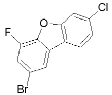 | 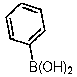 | 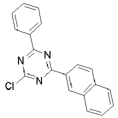 | 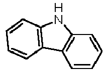 | 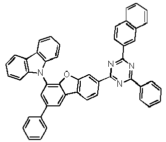 | 41% |
| 1-121 | 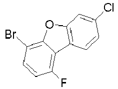 | 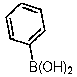 | 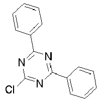 | 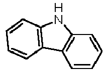 | 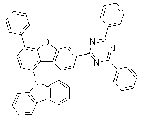 | 38% |
| 1-122 | 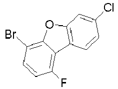 | 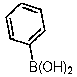 | 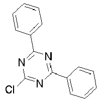 | 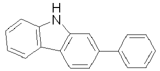 | 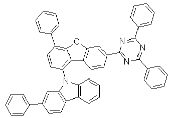 | 39% |
| 1-130 | 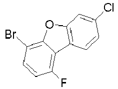 | 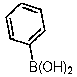 | 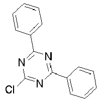 | 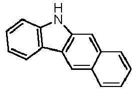 | 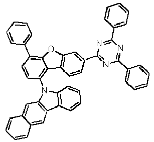 | 38% |
| 1-141 | 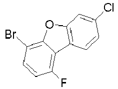 | 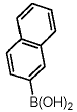 | 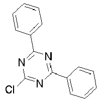 | 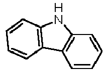 | 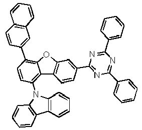 | 39% |
| 1-151 | 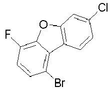 | 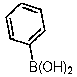 | 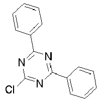 | 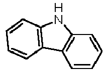 | 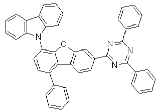 | 40% |
| 1-152 | 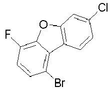 | 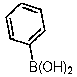 | 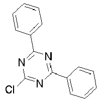 | 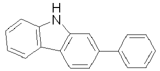 | 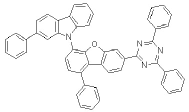 | 41% |
| 1-160 | 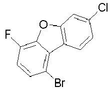 | 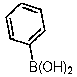 | 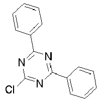 | 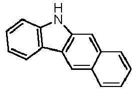 | 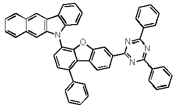 | 43% |
| 1-171 | 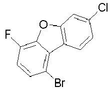 | 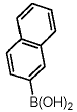 | 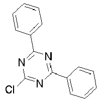 | 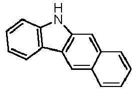 | 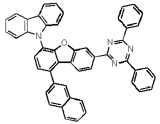 | 42% |
| 1-174 | 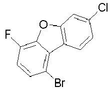 | 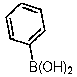 | 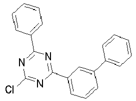 | 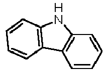 | 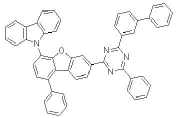 | 41% |
| 1-177 | 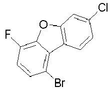 | 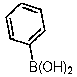 | 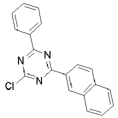 | 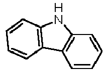 | 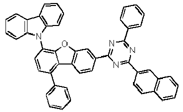 | 44% |
| 1-181 | 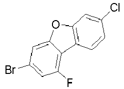 | 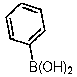 | 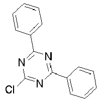 | 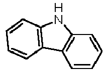 | 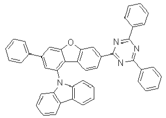 | 39% |
| 1-190 | 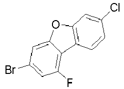 | 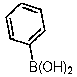 | 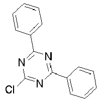 | 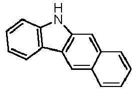 | 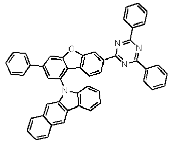 | 38% |
| 1-198 | 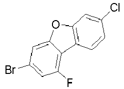 | 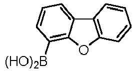 | 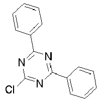 | 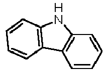 | 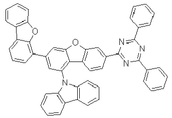 | 37% |
| 1-201 | 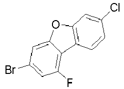 | 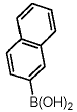 | 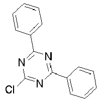 | 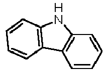 | 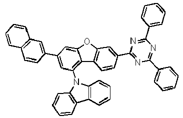 | 37% |
| 1-211 | 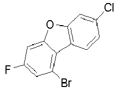 | 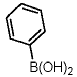 | 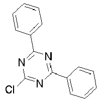 | 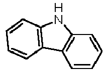 | 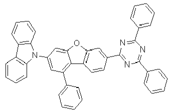 | 42% |
| 1-220 | 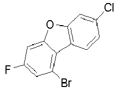 | 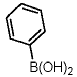 | 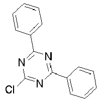 | 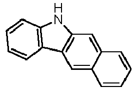 | 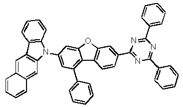 | 43% |
| 1-224 | 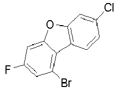 | 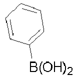 | 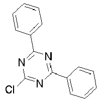 | 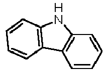 | 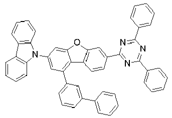 | 43% |
| 2-1 | 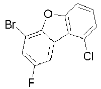 | 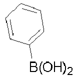 | 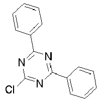 | 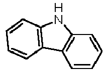 | 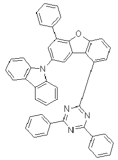 | 41% |
| 2-2 | 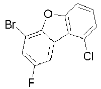 | 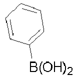 | 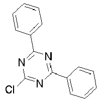 | 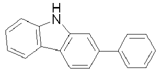 | 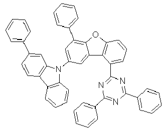 | 41% |
| 2-3 | 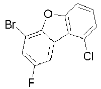 | 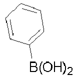 | 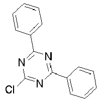 | 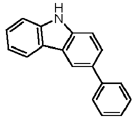 | 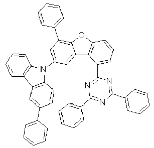 | 44% |
| 2-19 | 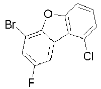 | 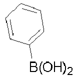 | 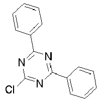 | 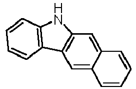 | 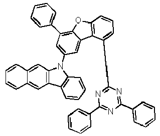 | 45% |
| 2-21 | 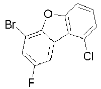 | 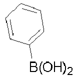 | 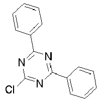 | 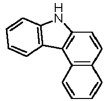 | 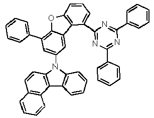 | 46% |
| 2-24 | 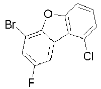 | 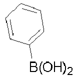 | 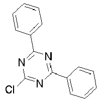 | 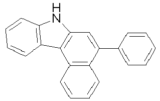 | 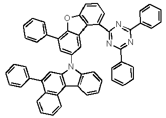 | 45% |
| 2-25 | 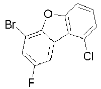 | 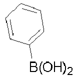 | 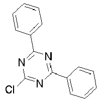 | 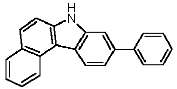 | 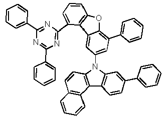 | 43% |
| 2-34 | 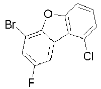 | 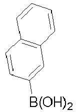 | 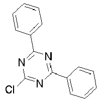 | 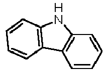 | 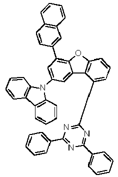 | 41% |
| 2-35 | 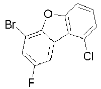 | 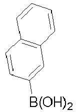 | 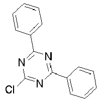 | 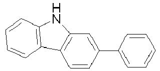 | 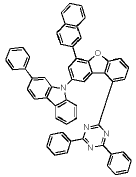 | 42% |
| 2-38 | 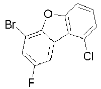 | 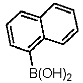 | 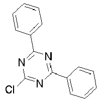 | 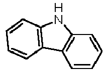 | 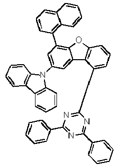 | 40% |
| 2-43 | 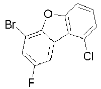 | 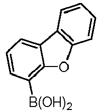 | 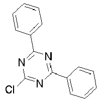 | 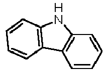 | 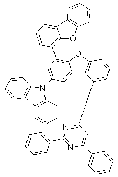 | 41% |
| 2-46 | 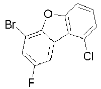 | 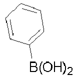 | 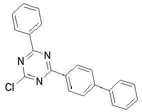 | 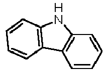 | 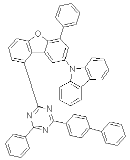 | 43% |
| 2-47 | 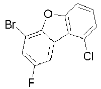 | 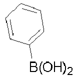 | 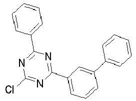 | 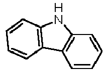 | 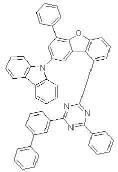 | 43% |
| 2-51 | 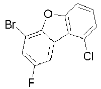 | 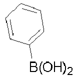 | 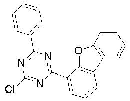 | 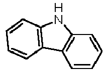 | 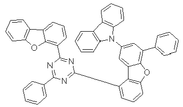 | 44% |
| 2-57 | 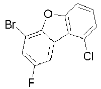 | 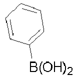 | 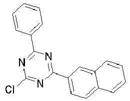 | 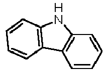 | 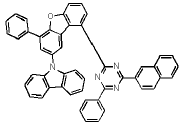 | 42% |
| 2-59 | 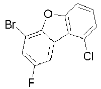 | 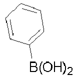 | 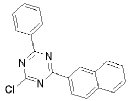 | 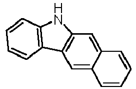 | 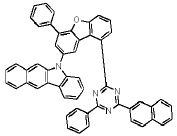 | 37% |
| 2-61 | 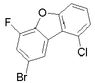 | 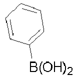 | 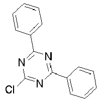 | 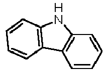 | 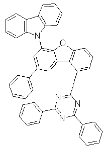 | 39% |
| 2-62 | 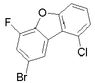 | 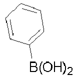 | 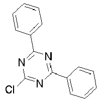 | 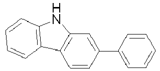 | 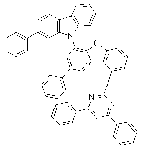 | 44% |
| 2-63 | 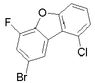 | 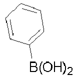 | 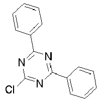 | 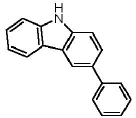 | 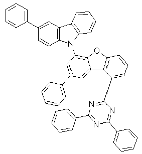 | 44% |
| 2-72 | 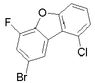 | 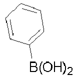 | 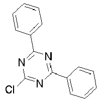 | 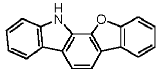 | 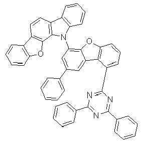 | 32% |
| 2-73 | 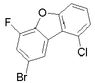 | 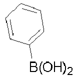 | 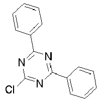 | 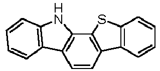 | 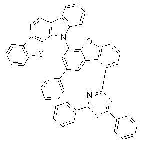 | 34% |
| 2-77 | 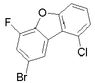 | 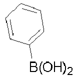 | 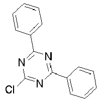 | 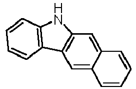 | 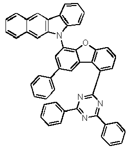 | 41% |
| 2-79 | 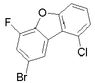 | 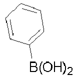 | 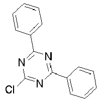 | 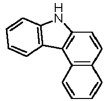 | 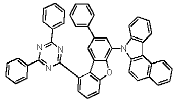 | 44% |
| 2-86 | 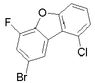 | 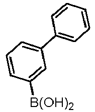 | 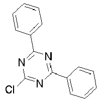 | 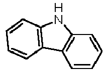 | 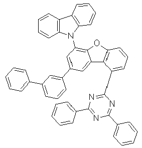 | 40% |
| 2-91 | 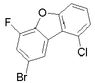 | 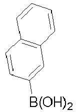 | 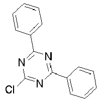 | 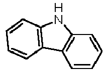 | 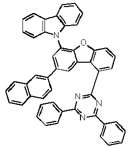 | 42% |
| 2-100 | 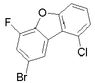 | 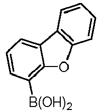 | 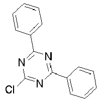 | 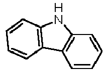 | 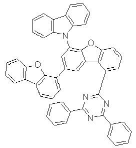 | 43% |
| 2-102 | 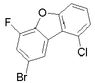 | 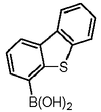 | 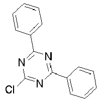 | 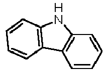 | 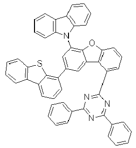 | 41% |
| 2-106 | 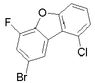 | 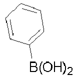 | 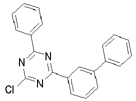 | 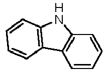 | 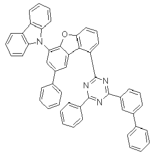 | 41% |
| 2-114 | 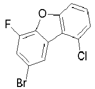 | 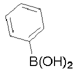 | 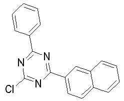 | 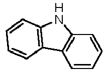 | 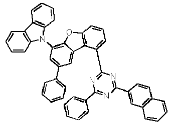 | 42% |
| 2-121 | 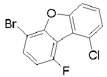 | 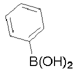 | 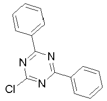 | 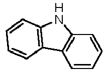 | 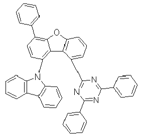 | 36% |
| 2-122 | 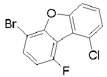 | 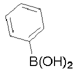 | 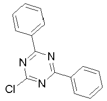 | 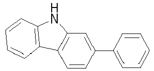 | 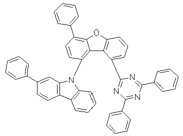 | 34% |
| 2-130 | 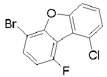 | 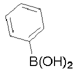 | 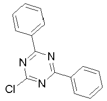 | 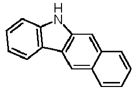 | 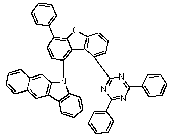 | 35% |
| 2-141 | 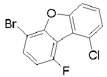 | 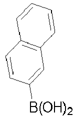 | 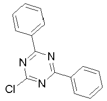 | 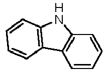 | 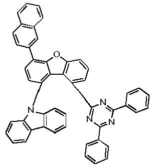 | 32% |
| 2-151 | 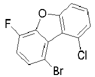 | 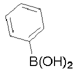 | 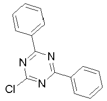 | 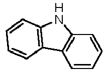 | 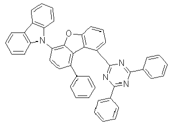 | 38% |
| 2-152 | 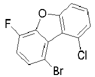 | 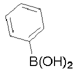 | 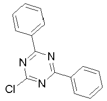 | 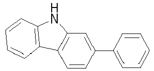 | 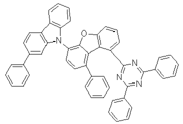 | 39% |
| 2-160 | 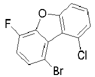 | 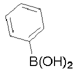 | 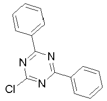 | 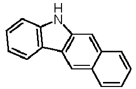 | 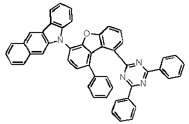 | 41% |
| 2-171 | 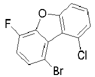 | 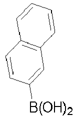 | 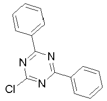 | 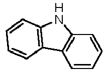 | 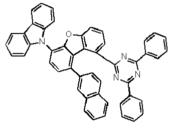 | 40% |
| 2-174 | 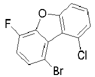 | 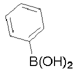 | 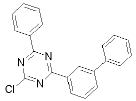 | 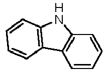 | 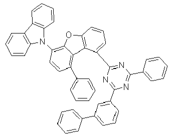 | 37% |
| 2-177 | 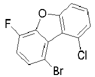 | 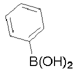 | 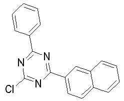 | 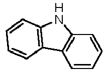 | 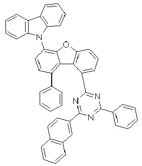 | 38% |
| 2-181 | 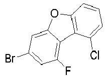 | 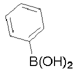 | 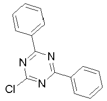 | 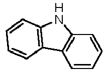 | 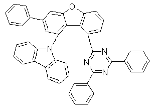 | 37% |
| 2-190 | 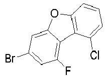 | 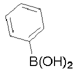 | 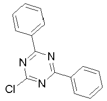 | 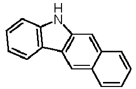 | 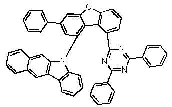 | 35% |
| 2-198 | 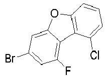 | 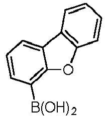 | 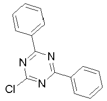 | 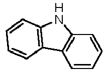 | 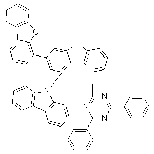 | 38% |
| 2-201 | 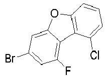 | 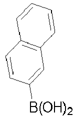 | 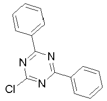 | 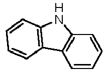 | 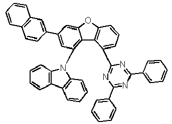 | 35% |
| 2-211 | 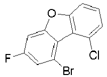 | 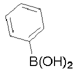 | 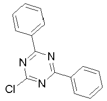 | 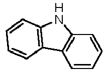 | 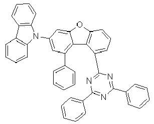 | 40% |
| 2-220 | 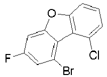 | 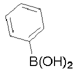 | 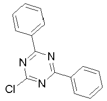 | 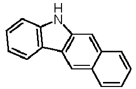 | 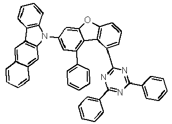 | 39% |
| 2-224 | 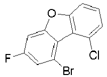 | 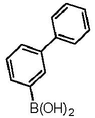 | 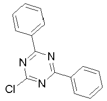 | 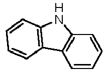 | 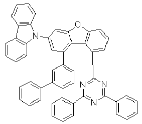 | 40% |
| 2-234 | 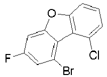 | 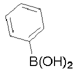 | 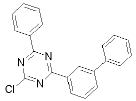 | 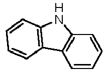 | 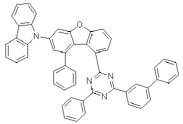 | 41% |

### <Preparation Example 4> Synthesis of Compound 3-3

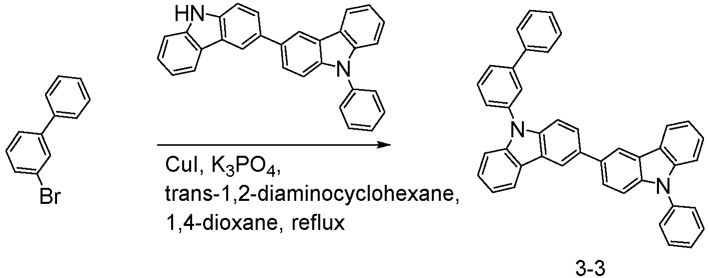

### 1) Preparation of Compound 3-3

After dissolving 3-bromo-1,1'-biphenyl (3.7 g, 15.8 mM), 9-phenyl-9H,9'H-3,3'-bicarbazole (6.5 g, 15.8 mM), CuI (3.0 g, 15.8 mM), trans-1,2-diaminocyclohexane (1.9 mL, 15.8 mM) and K₃PO₄ (3.3 g, 31.6 mM) in 1,4-dioxane (100 mL), the result was refluxed for 24 hours. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:Hex=1:3), and recrystallized with methanol to obtain target Compound 3-3 (7.5 g, 85%).

Target Compound A was synthesized in the same manner as in Preparation Example 4 except that Intermediate A of the following Table 4 was used instead of 3-bromo-1,1'-biphenyl, and Intermediate B of the following Table 4 was used instead of 9-phenyl-9H,9'H-3,3'-bicarbazole.

**[Table 4]**

| Compo und No. | Intermediate A | Intermediate B | Target Compound A | Overall Yield |
|---|---|---|---|---|
| 3-3 | 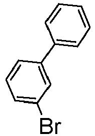 | 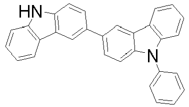 | 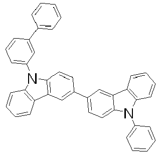 | 85% |
| 3-4 | 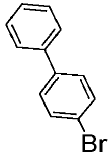 | | 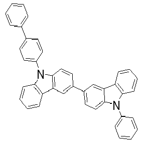 | 83% |
| 3-7 | 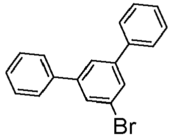 | | 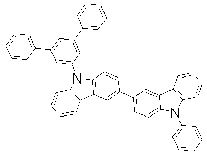 | 84% |
| 3-9 | 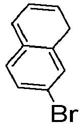 | | 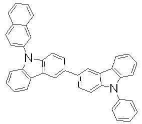 | 80% |
| 3-31 | 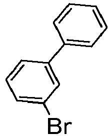 | 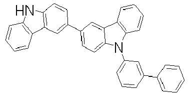 | 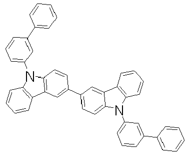 | 81% |
| 3-32 | 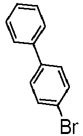 | | 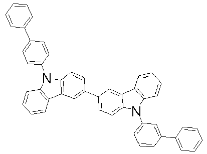 | 80% |
| 3-37 | 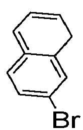 | | 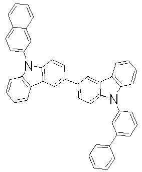 | 79% |

### <Preparation Example 5> Synthesis of Compound 4-2

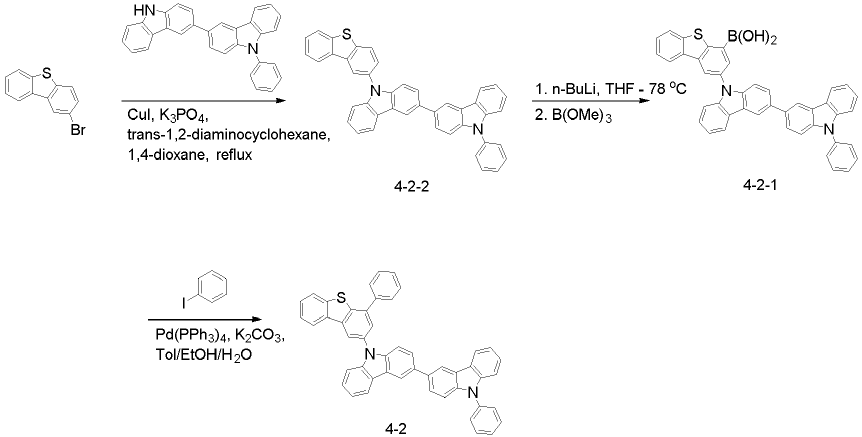

### 1) Preparation of Compound 4-2-2

After dissolving 2-bromodibenzo[b,d]thiophene (4.2 g, 15.8 mM), 9-phenyl-9H,9'H-3,3'-bicarbazole (6.5 g, 15.8 mM), CuI (3.0 g, 15.8 mM), trans-1,2-diaminocyclohexane (1.9 mL, 15.8 mM) and K₃PO₄ (3.3 g, 31.6 mM) in 1,4-dioxane (100 mL), the result was refluxed for 24 hours. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:Hex=1:3), and recrystallized with methanol to obtain target Compound 4-2-2 (7.9 g, 85%).

### 2) Preparation of Compound 4-2-1

To a mixture solution obtained by introducing Compound 4-2-1 (8.4 g, 14.3 mmol) and THF (100 mL), 2.5 M n-BuLi (7.4 mL, 18.6 mmol) was added dropwise at -78°C, and the result was stirred for 1 hour at room temperature. Trimethyl borate (4.8 mL, 42.9 mmol) was added dropwise to the reaction mixture, and the result was stirred for 2 hours at room temperature. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:MeOH=100:3), and recrystallized with DCM to obtain target Compound 4-2-1 (3.9 g, 70%).

### 3) Preparation of Compound 4-2

After dissolving Compound 4-2-1 (6.7 g, 10.5 mM), iodobenzene (2.1 g, 10.5 mM), Pd(PPh₃)₄ (606 mg, 0.52 mM) and K₂CO₃ (2.9 g, 21.0 mM) in toluene/EtOH/H₂O (100 mL/20 mL/20 mL), the result was refluxed for 12 hours. After the reaction was completed, the result was extracted by introducing distilled water and DCM thereto at room temperature. The organic layer was dried with MgSO₄, and then the solvent was removed using a rotary evaporator. The reaction material was purified by column chromatography (DCM:Hex=1:3), and recrystallized with methanol to obtain target Compound 4-2 (4.9 g, 70%).

The following target Compound B was obtained in the same manner as in Preparation Example 5 except that Compound A of the following Table 5 was used instead of iodobenzene.

**[Table 5]**

| Compo und No. | Intermediate 4-2-1 | Intermediat e A | Target Compound B | Overall Yield |
|---|---|---|---|---|
| 4-2 | 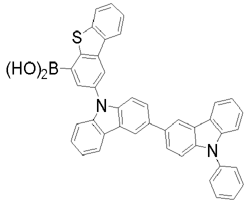 | 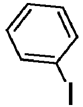 | 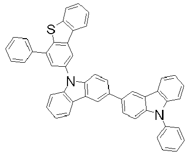 | 83% |
| 4-3 | | 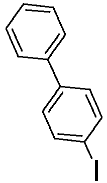 | 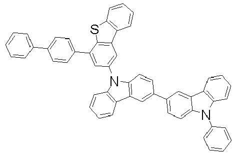 | 84% |
| 4-5 | | 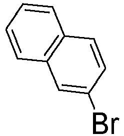 | 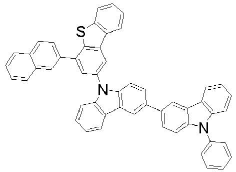 | 80% |

Heterocyclic compounds corresponding to Chemical Formula 1 and Chemical Formula 2 other than the compounds described in Preparation Examples 1 to 5 and Tables 1 to 5 were prepared in the same manner as in the preparation examples described above.

Synthesis identification data of the compounds prepared above are as described in the following [Table 6] and [Table 7] .

**[Table 6]**

| Compound | FD-Mass | Compound | FD-Mass |
|---|---|---|---|
| 1-1 | m/z=640.75(C45H28N4O=640.23) | 2-1 | m/z=640.75(C45H28N4O=640.23) |
| 1-2 | m/z=716.84(C51H32N4O=716.26) | 2-2 | m/z=716.84(C51H32N4O=716.26) |
| 1-3 | m/z=716.84(C51H32N4O=716.26) | 2-3 | m/z=716.84(C51H32N4O=716.26) |
| 1-19 | m/z=690.81(C49H30N4O=690.24) | 2-19 | m/z=690.81(C49H30N4O=690.24) |
| 1-21 | m/z=690.81(C49H30N4O=690.24) | 2-21 | m/z=690.81(C49H30N4O=690.24) |
| 1-24 | m/z=766.90(C55H34N4O=766.27) | 2-24 | m/z=766.90(C55H34N4O=766.27) |
| 1-25 | m/z=766.90(C55H34N4O=766.27) | 2-25 | m/z=766.90(C55H34N4O=766.27) |
| 1-34 | m/z=690.81(C49H30N4O=690.24) | 2-34 | m/z=690.81(C49H30N4O=690.24) |
| 1-35 | m/z=766.90(C55H34N4O=766.27) | 2-35 | m/z=766.90(C55H34N4O=766.27) |
| 1-38 | m/z=690.81(C49H30N4O=690.24) | 2-38 | m/z=690.81(C49H30N4O=690.24) |
| 1-43 | m/z=730.83(C51H30N4O2=730.24) | 2-43 | m/z=730.83(C51H30N4O2=730.24) |
| 1-46 | m/z=716.84(C51H32N4O=716.26) | 2-46 | m/z=716.84(C51H32N4O=716.26) |
| 1-47 | m/z=716.84(C51H32N4O=716.26) | 2-47 | m/z=716.84(C51H32N4O=716.26) |
| 1-51 | m/z=730.83(C51H30N4O2=730.24) | 2-51 | m/z=730.83(C51H30N4O2=730.24) |
| 1-57 | m/z=690.81(C49H30N4O=690.24) | 2-57 | m/z=690.81(C49H30N4O=690.24) |
| 1-59 | m/z=740.87(C53H32N4O=740.26) | 2-59 | m/z=740.87(C53H32N4O=740.26) |
| 1-61 | m/z=640.75(C45H28N4O=640.23) | 2-61 | m/z=640.75(C45H28N4O=640.23) |
| 1-62 | m/z=716.84(C51H32N4O=716.26) | 2-62 | m/z=716.84(C51H32N4O=716.26) |
| 1-63 | m/z=716.84(C51H32N4O=716.26) | 2-63 | m/z=716.84(C51H32N4O=716.26) |
| 1-72 | m/z=730.83(C51H30N4O2=730.24) | 2-72 | m/z=730.83(C51H30N4O2=730.24) |
| 1-73 | m/z=746.89(C51H30N4OS=746.21) | 2-73 | m/z=746.89(C51H30N4OS=746.21) |
| 1-77 | m/z=690.81(C49H30N4O=690.24) | 2-77 | m/z=690.81(C49H30N4O=690.24) |
| 1-79 | m/z=690.81(C49H30N4O=690.24) | 2-79 | m/z=690.81(C49H30N4O=690.24) |
| 1-86 | m/z=716.84(C51H32N4O=716.26) | 2-86 | m/z=716.84(C51H32N4O=716.26) |
| 1-91 | m/z=690.81(C49H30N4O=690.24) | 2-91 | m/z=690.81(C49H30N4O=690.24) |
| 1-100 | m/z=730.83(C51H30N4O2=730.24) | 2-100 | m/z=730.83(C51H30N4O2=730.24) |
| 1-102 | m/z=746.89(C51H30N4OS=746.21) | 2-102 | m/z=746.89(C51H30N4OS=746.21) |
| 1-106 | m/z=716.84(C51H32N4O=716.26) | 2-106 | m/z=716.84(C51H32N4O=716.26) |
| 1-114 | m/z=690.81(C49H30N4O=690.24) | 2-114 | m/z=690.81(C49H30N4O=690.24) |
| 1-121 | m/z=640.75(C45H28N4O=640.23) | 2-121 | m/z=640.75(C45H28N4O=640.23) |
| 1-122 | m/z=716.84(C51H32N4O=716.26) | 2-122 | m/z=716.84(C51H32N4O=716.26) |
| 1-130 | m/z=690.81(C49H30N4O=690.24) | 2-130 | m/z=690.81(C49H30N4O=690.24) |
| 1-141 | m/z=690.81(C49H30N4O=690.24) | 2-141 | m/z=690.81(C49H30N4O=690.24) |
| 1-151 | m/z=640.75(C45H28N4O=640.23) | 2-151 | m/z=640.75(C45H28N4O=640.23) |
| 1-152 | m/z=716.84(C51H32N4O=716.26) | 2-152 | m/z=716.84(C51H32N4O=716.26) |
| 1-160 | m/z=690.81(C49H30N4O=690.24) | 2-160 | m/z=690.81(C49H30N4O=690.24) |
| 1-171 | m/z=690.81(C49H30N4O=690.24) | 2-171 | m/z=690.81(C49H30N4O=690.24) |
| 1-174 | m/z=716.84(C51H32N4O=716.26) | 2-174 | m/z=716.84(C51H32N4O=716.26) |
| 1-177 | m/z=690.81(C49H30N4O=690.24) | 2-177 | m/z=690.81(C49H30N4O=690.24) |
| 1-181 | m/z=640.75(C45H28N4O=640.23) | 2-181 | m/z=640.75(C45H28N4O=640.23) |
| 1-190 | m/z=690.81(C49H30N4O=690.24) | 2-190 | m/z=690.81(C49H30N4O=690.24) |
| 1-198 | m/z=730.83(C51H30N4O2=730.24) | 2-198 | m/z=730.83(C51H30N4O2=730.24) |
| 1-201 | m/z=690.81(C49H30N4O=690.24) | 2-201 | m/z=690.81(C49H30N4O=690.24) |
| 1-211 | m/z=640.75(C45H28N4O=640.23) | 2-211 | m/z=640.75(C45H28N4O=640.23) |
| 1-220 | m/z=690.81(C49H30N4O=690.24) | 2-220 | m/z=690.81(C49H30N4O=690.24) |
| 1-224 | m/z=716.84(C51H32N4O=716.26) | 2-224 | m/z=716.84(C51H32N4O=716.26) |
| | | 2-234 | m/z=716.84(C51H32N4O=716.26) |
| 3-3 | m/z=560.23 (C₄₂H₂₈N₂=560.70) | 3-4 | m/z=560.23 (C₄₂H₂₈N₂=560.70) |
| 3-7 | m/z=636.26 (C₄₈H₃₂N₂=636. 80) | 3-9 | m/z=534.21 (C₄₀H₂₆N₂=534.66) |
| 3-31 | m/z=636.26 (C₄₈H₃₂N₂=636. 80) | 3-32 | m/z=636.26 (C₄₈H₃₂N₂=636. 80) |
| 3-37 | m/z=610.24 (C₄₆H₃₀N₂=610. 76) | 3-42 | m/z=636.26 (C₄₈H₃₂N₂=636. 80) |
| 3-82 | m/z=584.23 (C₄₄H₂₈N₂=584.72) | | |
| 4-2 | m/z=666.84 (C₄₈H₃₀N₂=666.21) | 4-3 | m/z=742.24 (C₅₄H₃₄N₂S=742.94) |
| 4-5 | m/z=716.23 (C₅₂H₃₂N₂S=716.90) | | |

**[Table 7]**

| Compound | ¹H NMR (CDCl₃, 200 Mz) |
|---|---|
| 1-1 | δ=8.55(1H, d) 8.36(4H, d) 8.19(1H, d) 8.03(1H, d) 7.94(1H, d) 7.82(1H ,d) 7.76(1H, s) 7.71(1H, s), 7.65(1H, s) 7.58-7.35(14H, m) 7.20-7.16(2H, m) |
| 1-2 | δ=8.55(1H, d) 8.36-8.31(5H, m) 8.03(1H, d) 7.94(2H, t) 7.82(1H ,d) 7.76-7.74(5H, m) 7.65(1H, s) 7.51-7.35(15H, m) 7.16(2H, m) |
| 1-3 | δ=8.55(1H, d) 8.36(4H, d) 8.03-7.94(3H, m) 7.89(1H, s) 7.82-7.75(6H, m) 7.65(1H, s) 7.51-7.35(15H, m) 7.16(1H, t) |
| 1-19 | δ=8.55(1H, d) 8.36-8.28(5H, m), 8.11(1H, d) 8.03(1H, d) 7.94(1H, d) 7.82(1H ,d) 7.76-7.69(5H, m) 7.55-7.35(14H, m) 7.16(1H, t) |
| 1-21 | δ=8.55-8.54(2H, m) 8.36(4H, m) 8.03(1H, d) 7.99-7.94(2H, m) 7.82(1H, d) 7.76(1H ,s) 7.71(1H, s) 7.65-7.35(17H, m) 7.16(1H, t) |
| 1-24 | δ=8.97(2H, d) 8.55(1H, d) 8.36(4H, d) 8.03(1H, d) 7.94(1H, d) 7.82-7.76(5H, m) 7.65-7.35(19H, m) 7.16(1H, t) |
| 1-25 | δ=8.62(1H, d) 8.54(1H, d) 8.36(4H, d) 8.22(1H, d) 8.03-7.99(2H, m) 7.82(1H, d) 7.76-7.41(24H, m) |
| 1-34 | δ=8.55(1H, d) 8.36(4H, d) 8.19(1H, d) 8.09-7.74(5H, m) 7.82(1H, d) 7.76(1H, s) 7.71(1H, s) 7.63-7.50(12H, m) 7.35-7.38(2H, m) 7.20-7.16(2H, t) |
| 1-35 | δ=8.55(1H, d) 8.36-8.31(5H, m) 8.09-7.91(6H, m) 7.82(1H, d) 7.76-7.71(5H, m) 7.65-7.35(15H, m) 7.16(1H, t) |
| 1-38 | δ=8.95(1H, d) 8.55-8.50(2H, m) 8.36(4H, d) 8.19(2H, m) 8.09(1H, d) 8.03(1H, d) 7.94(1H, d) 7.82(1H, d) 7.77-7.71(3H, m) 7.65(1H, s) 7.58-7.50(9H, m) 7.39-7.35(2H, m) 7.20-7.16(2H, m) |
| 1-43 | δ=8.55(1H, d) 8.36(4H, d), 8.19(1H, d) 8.08-7.94(5H, m) 7.82(1H, d) 7.76(1H, s) 7.71(1H, s) 7.65(1H, s) 7.58-7.50(10H, m) 7.39-7.31(3H, m) 7.20-7.16(2H, m) |
| 1-46 | δ=8.55(1H, d) 8.46(2H, d), 8.19(1H s) 8.03(1H, s) 7.96-7.94(3H, m) 7.82(1H, d) 7.76-7.71(4H, m) 7.65(1H, s) 7.587.35(14H, m) 7.25-7.16(4H, m) |
| 1-47 | δ=8.55(1H, d) 8.36-8.35(3H, m) 8.19(1H, s) 8.03(1H, d) 7.94(2H, m) 7.82(1H, d) 7.767-74(5H, m) 7.65-7.35(16H, m) 7.20-7.16(2H, m) |
| 1-51 | δ=8.55(1H, d) 8.36(2H ,d) 8.19(1H, d) 8.08-7.88(5H, m) 7.82(1H, s) 7.76(1H, s) 7.71(1H, s)7.65(1H, s) 7.58-7.35(15H, m) 7.20-7.16(2H, m) |
| 1-57 | δ=9.09(1H, s) 8.55(1H, d) 8.49(1H ,d) 8.36(2H, d) 8.19-8.16(2H, m) 8.08-7.94(4H, m) 7.82(1H, d) 7.76(1H, s) 7.71(1H, s) 7/65(1H, s) 7.59-7.35(13H, m) 7.20-7.16(2H, m) |
| 1-59 | δ=9.09(1H, s) 8.55(1H, d) 8.49(1H, d) 8.36(2H, d) 8.28(1H, d) 8.11-7.94(6H, m) 7.82-7.35(19H, m) |
| 1-61 | δ=8.55(1H, d) 8.36(4H, d) 8.19(1H, d) 8.03(1H, d) 7.94(1H, d) 7.82-7.75(5H, m) 7.65(1H, s) 7.58-7.35(12H, m) 7.20-7.16(2H, m) |
| 1-62 | δ=8.55(1H, d) 8.36(4H, d) 8.31(1H, d) 8.03(1H, d) 7.94-7.91(2H, m) 7.82(7.74(8H, m) 7.65(1H, s) 7.50-7.35(13H, m) 7.16(1H, t) |
| 1-63 | δ=8.55(1H, d) 8.36(4H, d) 8.03-7.94(3H, m) 7.89(1H, s) 7.82-7.75(8H, m) 7.65(1H, s) 7.50-7.35(13H, m) 7.16(1H, t) |
| 1-72 | δ=8.55(1H, d) 8.36(4H, d) 8.03-7.94(3H, m) 7.84-7.75(5H, m) 7.65(1H, s) 7.54-7.35(14H, m) 7.16(1H, t) |
| 1-73 | δ=8.55(1H, d) 8.45(1H, d) 8.36(4H, d) 8.05(2H, d) 7.94(2H, d) 7.82-7.75(5H, m) 7.65(1H, s) 7.60-7.35(13H, m) 7.16(1H, t) |
| 1-77 | δ=8.55(1H, d) 8.36(4H, d) 8.28(1H, d) 8.11(1H, d) 8.03(1H, d) 7.94(1H, d), 7.82-7.75(7H, m) 7.65(1H, s) 7.55-7.35(12H, m), 7.16(1H, t) |
| 1-79 | δ=8.55(2H, d) 8.36(4H, d) 8.03-7.94(3H, m) 7.82-7.75(5h, m) 7.65-7.35(15H, m) 7.16(1H, t) |
| 1-86 | δ=8.55(1H, d) 8.36(4H, d), 8.19(1H, d) 8.03(1h, d) 7.94(2H, d) 7.82-7.73(6H, m) 7.65-7.35(15H, m) 7.20-7.16(2H, m) |
| 1-91 | δ=8.55(1H, d) 8.36(4H, d) 8.19(1H, d) 8.09-7.94(6H, m) 7.82-7.76(3H, m) 7.63-7.50(12H, m) 7.38-7.35(2H, m) 7.20-7.16(2H, m) |
| 1-100 | δ=8.55(1H, d) 8.36(4H, d) 8.19(1H, d) 8.08-7.94(5H, m) 7.82-7.76(3H, m) 7.65(1H, s) 7.58-7.50(10H, m) 7.39-7.31(3H, m) 7.20-7.16(2H, m) |
| 1-102 | δ=8.55(1H, d) 8.45(1H, d) 8.36-32(5H, m) 8.19(1H, d) 8.03(1H, d) 7.94(2H, d) 7.82-7.65(5H, m) 7.58-7.49(10H, m) 7.35(1H, t) 7.20-7.16(2H, m) |
| 1-106 | δ=8.55(1H, d) 8.38(3H, m) 8.19(1H, d) 8.03(1H, d) 7.93(2H, d) 7.82-7.75(8H, m) 7.65-7.35(14H, m) 7.20-7.16(2H, m) |
| 1-114 | 9.09(1H, s) 8.55(1H, d) 8.49(1H, d) 8.36(2H, d) 8.19-8.16(2H, m) 8.08-7.94(4H, m) 7.82-7.75(5h ,m) 7.65-7.35(12H, m) 7.20-7.16(2H ,m) |
| 1-121 | δ=8.55(1H, d) 8.36(4H, d) 8.19(1H, d) 8.04(2H, d) 7.94(1H, d) 7.82(1H, d) 7.76(1H, s) 7.58-7.35(15H, m) 7.20-7.16(2H, m) |
| 1-122 | δ=8.55(1H, d) 8.36-8.31(5H, m) 8.03(2H, d) 7.94-7.93(2H, m) 7.82(1H, d) 7.76(4H, d) 7.55-7.35(16H, m) 7.16(1H, t) |
| 1-130 | δ=8.55(1H, d) 8.36(4H, d) 8.28(1H, d) 8.11(1H, d) 8.03(2H, d) 7.94(1H, d) 7.82(1H, d) 7.76-7.69(3H, m) 7.55-7.35(15H, m) 7.16(1H, t) |
| 1-141 | δ=8.55(1H, d) 8.36(4H, d) 8.19(1H, d) 8.09-7.94(6H, m) 7.82(1H, s) 7.76(1H, s) 7.63-7.35(14H, m) 7.20-7.16(2H, m) |
| 1-151 | δ=8.55(1H, d) 8.36(4H, d) 8.19(1H, d) 8.09(1H, d) 7.96-7.94(2H, m) 7.82-7.76(4H, m) 7.58-7.35(13H, m) 7.20-7.16(2H, m) |
| 1-152 | δ=8.55(1H, d) 8.36-8.31(5H, m) 8.03(1H, d) 7.96-7.91(3H, m) 7.82-7.75(7H, m) 7.50-7.35(14H, m) 7.16(1H, t) |
| 1-160 | δ=8.55(1H, d) 8.36(4H, d) 8.28(1H, d) 8.11(1H, d) 8.03(1H, d) 7.96-7.94(2H, d) 7.82-7.75(6H m) 7.55-7.35(13H, m) 7.16(1H, t) |
| 1-171 | δ=8.55(1H, d) 8.36(4H, d) 8.19(1H, d) 8.09-7.94(6H, m) 7.82(1H, d) 7.76(1H, s) 7.63-7.50(12H, m) 7.38-7.35(2H, m) 7.20-7.16(2H, m) |
| 1-174 | δ=8.55(1H, d) 8.36-8.33(3H, m) 8.19(1H, d) 8.03(1H, d) 7.96-7.94(3H, m) 7.82-7.73(7h, m) 7.61-7.35(14H, m) 7.20-7.16(2H, m) |
| 1-177 | δ=9.09(1H, s) 8.55(1H, d) 8.49(1H, s) 8.36(2H, d) 8.19-8.16(2H, m) 8.08-7.94(5H, m) 7.82-7.76(4H, m) 7.61-7.35(12H ,m) 7.20-7.16(2H, m) |
| 1-181 | δ=8.55(1H, d) 8.36(4H, d) 8.19(1H, d) 8.03(1H, d) 7.94(1H, d) 7.82-7.72(5H, m) 7.59-7.35(13H, m) 7.20-7.16(2H, m) |
| 1-190 | δ=8.55(1H, d) 8.36(4H, d) 8.28(1H, d) 8.11(1H, d) 8.03(1H, d) 7.94(1H, d) 7.82-7.69(7H, m) 7.55-7.35(13H, m) 7.16(1H, t) |
| 1-198 | δ=8.55(1H, d) 8.36(4H, d) 8.19(1H, d) 8.08-7.94(5H, m) 7.82(1H, d) 7.76(1H, s) 7.72(1H, s) 7.58-7.50(11H, m) 7.39-7.31(3h, m) 7.20-7.16(2H, m) |
| 1-201 | δ=8.55(1H, d) 8.36(4H, d) 8.19(1H, d) 8.09-7.94(5H, m) 7.82(1H, d) 7.76(1H, s) 7.72(1H, s) 7.63-7.35(14H, m) 7.20-7.16(2H, m) |
| 1-211 | δ=8.55(1H, d) 8.36(4H, d) 8.19(1H, d) 8.03(1H, d) 7.94(1H, d) 7.82-7.76(4H, m) 7.64(1H, s) 7.59-7.35(13H, m) 7.20-7.16(2H, m) |
| 1-220 | δ=8.55(1H, d) 8.36(4H, d) 8.28(1H, d) 8.11(1H, d) 8.03(1H, d) 7.94(1H, d) 7.79-7.352(H, m) 7.16(1H, t) |
| 1-224 | δ=8.55(1H, d) 8.36(4H, d) 8.19(1H, d) 8.03(1H, d) 8.03(1H, d) 7.94(2H, d)7.82(1H, d) 7.76-7.73(4H, m) 7.64-7.35(16H, m) 7.20-7.16(2H, m) |
| 2-1 | δ=8.55(1H, d) 8.36(4H, d) 8.11(1H, d) 7.94(1H, d) 7.8(1H, d) 7.71-7.65(3H, m) 7.57-7.35(15H, m) 7.20-7.16(1H, t) |
| 2-2 | δ=8.55(1H, d) 8.36-8.31(5H, m) 7.94(2H, t) 7.82(1H, d) 7.75-7.65(6H, m) 7.50-7.35(16H, m) 7.16(1H, t) |
| 2-3 | δ=8.55(1H, d) 8.36(4H, d) 7.99-7.89(3H, m) 7.82-7.65(7H, m) 7.57-7.35(16H, m) 7.16(1H, t) |
| 2-19 | δ=8.55(1H, d) 8.36(4H, d) 8.28(1H, d) 8.11(1H, d) 7.94(1H, d) 7.82-7.69(6H, m) 7.57-7.35(15H, m) 7.16(1H, t) |
| 2-21 | δ=8.55(2H, d) 8.36(4H, d) 7.99(1H, d) 7.94(1H, d) 7.82(1H, d) 7.71-7.35(20H, m) 7.16(1H, t) |
| 2-24 | δ=8.97(2H, d) 8.55(1H, d) 8.36(4H, d) 7.94(1H, d) 7.82(7.79(3H, m) 7.71-7.35(22H, m) 7.16(1H, t) |
| 2-25 | δ=8.62(1H, d) 8.55(1H, d) 8.36(4H, d) 8.22(1H, d) 7.99(1H, d) 7.82(1H, d) 7.71-7.41(25H, m) |
| 2-34 | δ=8.55(1H, d) 8.36(4H, d)8.19(1H, d) 8.09-7.94(4H, m) 7.82(1H, d) 7.71-7.50(15H, m) 7.38-7.35(2H, m) 7.20-7.16(2H, m) |
| 2-35 | δ=8.55(1H, d) 8.36-8.31(5H, m) 8.09-7.91(5H, m) 7.82(1H, d) 7.71-7.35(21H, m) 7.16(1H, t) |
| 2-38 | δ=8.95(1H, d) 8.55(1H, d) 8.50(1H, d) 8.36(4H, d) 8.19(2H, d) 8.09(1h, d) 7.94(1H, d) 7.82-7.65(5H, m) 7.57-7.50(10H, m) 7.39-7.35(2H, m) 7.20-7.16(2H, m) |
| 2-43 | δ=8.55(1H, d) 8.36(4H, d) 8.19(1H, d) 8.08(1H, d) 8.02(1H, d) 7.94(1H, d) 7.82(1H, d) 7.71-7.65(3H, m) 7.57-7.50(11H, m) 7.39-7.31(3H, m) 7.20-7.16(2H, m) |
| 2-46 | δ=8.55(1H, d) 8.36(2H, d) 8.19(1H, d) 7.94(3H, d) 7.82(1H, d) 7.75-7.69(5H, m) 7.57-7.35(15H, m) 7.25-7.16(4H, m) |
| 2-47 | δ=8.55(1H, d) 8.36(3H, d) 8.19(1H, d) 7.94(2H, d) 7.82(1H, d) 7.75-7.35(22H, m) 7.20-7.16(2H, m) |
| 2-51 | δ=8.55(1H, d) 8.36(2H, d) 8.19(1H, d) 8.08(1H, d) 7.98-7.88(4H, m) 7.71-7.65(3H, m) 7.57-7.31(16H, m) 7.20-7.16(2H, m) |
| 2-57 | δ=9.09(1H, s) 8.55(1H, d) 8.49(1H, d) 8.36(1H, d) 8.19-8.16(2H, m) 8.08(1H, d) 8.00(1H, d) 7.94(1H, d) 7.82(1H, d) 7.71-7.35(17H, m) 7.20-7.16(2H, m) |
| 2-59 | δ=9.09(1H, s) 8.55(1H, d) 8.49(1H, d) 8.36(2H, d) 8.28(1H, d) 8.16-8.08(3H, m) 8.00(1H, d) 7.94(1H, d) 7.82-7.35(20H, m) 7.16(1H, t) |
| 2-61 | δ=8.55(1H, d) 8.36(4H, d) 8.19(1H, d) 7.94(1H, d) 7.82-7.65(6H, m) 7.57-7.35(13H, m) 7.20-7.16(2H, m) |
| 2-62 | δ=8.55(1H, d) 8.36-8.31(5H, m) 7.91(2H, m) 7.82-7.69(9H, m) 7.57-7.35(14H, m) 7.16(1H, t) |
| 2-63 | δ=8.55(1H, d) 8.36(4H, d) 7.99-7.89(3H, m) 7.82-7.65(9H, m) 7.57-7.35(14H, m) 7.16(1H, t) |
| 2-72 | δ=8.55(1H, d) 8.36(4H, d) 7.98(1H, d) 7.94(1H, d) 7.84-7.65(7H, m) 7.57-7.31(15H, m) 7.16(1H, t) |
| 2-73 | δ=8.55(1H, d) 8,45(1H, d) 8.36(4H, d) 8.05(1H, d) 7.94(2H, d) 7.82-7.35(20H, m) 7.16(1H, t) |
| 2-77 | δ=8.55(1H, d) 8.36(4H, d) 8.28(1H, d) 8.11(1H, d) 7.94(1H, d) 7.82-7.65(8H, m) 7.57-7.35(13H, m) 7.16(1H, t) |
| 2-79 | δ=8.55(2H, d) 8.36(4H, d) 7.99(1H, d) 7.94(1H, d) 7.82-7.35(21H, m) 7.16(1H, t) |
| 2-86 | δ=8.55(1H, d) 8.36(4H, d) 8.19(1H, d) 7.94(2H, m) 7.79-7.35(22H, m) 7.20-7.16(2H, m) |
| 2-91 | δ=8.55(1H, d) 8.36(4H, d) 8.19(1H, d) 8.09-7.94(4H, m) 7.82-7.79(2H, m) 7.69-7.50(14H, m) 7.37-7.31(3H, m) 7.20-7.16(2H, m) |
| 2-100 | δ=8.55(1H, d) 8.36(4H, d) 8.19(1H, d) 8.08(1H, d) 8.02-7.94(3H, m) 7.82-7.79(2H, m) 7.69-7.50(13H, m) 7.38-7.31(3H, m) 7.20-7.16(2H, m) |
| 2-102 | δ=8.55(1H, d) 8.45(1H, d) 8.36-8.32(5H, m) 8.19(1H, d) 7.94(2H, d) 7.82-7.79(2H, m) 7.70-7.65(3H, m) 7.58-7.50(11H, m) 7.35(1H, t) 7.20-7.16(2H, m) |
| 2-106 | δ=8.55(1H, d) 8.38-8.36(3H, m) 8.19(1H, d) 7.94(2H, m) 7.82-7.35(23H, m) 7.20-7.16(2H, m) |
| 2-114 | δ=9.09(1H, s) 8.55(1H, d) 8.49(1H, d) 8.36(2H, d) 8.19-8.16(2H, m) 8.08(1H, d) 8.00(1H d) 7.94(1H, d) 7.82-7.35(18H, m) 7.20-7.16(2H, m) |
| 2-121 | δ=8.55(1H, d) 8.36(4H, d) 8.19(1H, d) 8.02(1H, d) 7.94(1H, d) 7.69(1H, d) 7.58-7.35(16H, m) 7.21-7.20(2H, m) |
| 2-122 | δ=8.55(1H, d) 8.36-8.31(5H, m) 8.02(1H, d) 7.94-7.91(2H, m) 7.82(1H, d) 7.75-7.69(4H, m) 7.58-7.35(17H, m) 7.16(1H, t) |
| 2-130 | δ=8.55(1H, d) 8.36(4H, d) 8.28(1H, d) 8.11(1H, d) 8.02(1H, d) 7.94(1H, d) 7.82(1H, d) 7.75-7.69(3H, m) 7.58-7.35(16H, m) 7.16(1H, t) |
| 2-141 | δ=8.55(1H, d) 8.36(4H, d) 8.19(1H, d) 8.09-7.94(5H, m) 7.82(1H, d) 7.69-7.35(16H, m) 7.20-7.16(2H, m) |
| 2-151 | δ=8.55(1H, d) 8.36(4H, d) 8.19(1H, d) 7.94(2H, d) 7.82-7.79(3H, m) 7.69(1H, d) 7.58-735(14H, m) 7.20-7.16(2H, m) |
| 2-152 | δ=8.55(1H, d) 8.36-8.31(5H, m) 7.96-7.71(3h, m) 7.82-7.69(7H, m) 7.58-7.35(15H, m) 7.16(1H, t) |
| 2-160 | δ=8.55(1H, d) 8.36(4H, d) 8.28(1H, d) 8.11(1H, d) 7.96(2H, d) 7.82-7.69(6H, m) 7.58-7.35(14H, m) 7.16(1H, t) |
| 2-171 | δ=8.55(1H, d) 8.36(4H, d) 8.19(1H, d) 8.09-7.94(5H, m) 7.82(1H, d) 7.63-7.50(14H, m) 7.38-7.35(2H, m) 7.20-7.16(2H, m) |
| 2-174 | δ=8.55(1H, d) 8.38-8.36(3H, m) 8.19(1H, d) 7.96-7.94(3H, m) 7.82-7.69(7H, m) 7.58-7.35(15H, m) 7.20-7.16(2H, m) |
| 2-177 | δ=9.09(1H, s) 8.55(1H, d) 8.49(1H, s) 8.36(2H, d) 8.19-8.16(2H, m) 8.08(1H, d) 8.00-7.94(3H ,m) 7.82-7.79(3H, m) 7.69-7.35(14H, m) 7.20-7.16(2H, m) |
| 2-181 | δ=8.55(1H, d) 8.36(4H, d) 8.19(1H, d) 7.94(1H, d) 7.82-7.69(5H, m) 7.58-7.35(14H, m) 7.16(1H, t) |
| 2-190 | δ=8.55(1H, d) 8.36(4H, d) 8.28(1H, d) 8.11(1H, d) 7.94(1H, d) 7.82(1H, d) 7.75-7.69(5H, m) 7.58-7.35(14H, m) 7.16(1H, t) |
| 2-198 | δ=8.55(1H, d) 8.36(4H, d) 8.19(1H, d) 8.08(1H, d) 8.02-7.94(3H, m) 7.82(1H, d) 7.72-7.69(2H, m) 7.58-7.50(12H, m) 7.39-7.31(3H, m) 7.20-7.16(2H, m) |
| 2-201 | δ=8.55(1H, d) 8.36(4H, d) 8.19(1H, d) 8.09-8.06(2H, m) 7.99(1H, d) 7.94(1H, d) 7.82(1H, d) 7.69-7.50(15H, m) 7.39-7.35(2H, m)7.20-7.16(2H, m) |
| 2-211 | δ=8.55(1H, d) 8.36(4H, d)8.19(1H, d) 7.94(1H, d) 7.82-7.79(3H, m) 7.64-7.35(16H, m)7.20-7.16(2H, m) |
| 2-220 | δ=8.55(1H, d) 8.36(4H, d) 8.28(1H, d) 8.11(1H, d) 7.94(1H, d) 7.75-7.35(21H, m) 7.16(1H, t) |
| 2-224 | δ=8.55(1H, d) 8.36(4H, d) 8.19(1H, d)7.94(2H, d) 7.75-7.35(22H, m) 7.20-7.16(2H, m) |
| 2-234 | δ=8.55(1H, d) 8.38-36(3H, m) 8.19(1H, d) 7.94(2H, d) 7.79-7.35(23H, m) 7.20-7.16(2H, m) |
| 3-3 | δ=8.55 (1H, d), 8.30 (1H, d), 8.21-8.13 (3H, m), 7.99-7.89 (4H, m), 7.77-7.35 (17H, m), 7.20-7.16 (2H, m) |
| 3-4 | δ=8.55 (1H, d), 8.30(1H, d), 8.19-8.13(2H, m), 7.99-7.89(8H, m), 7.77-7.75 (3H, m), 7.62-7.35 (11H, m), 7.20-7.16 (2H, m) |
| 3-7 | δ=8.55 (1H, d), 8.31-8.30 (3H, d), 8.19-8.13 (2H, m), 7.99-7.89 (5H, m), 7.77-7.75 (5H, m), 7.62-7.35 (14H, m), 7.20-7.16 (2H, m) |
| 3-9 | δ=8.55 (1H, d), 8.30 (1H, d), 8.19-8.13 (2H, m), 8.03-7.77 (9H, m), 7.62-7.50 (9H, m), 7.36-7.35 (2H, m), 7.20-7.16 (2H, m) |
| 3-31 | δ=8.55 (1H, d), 8.30 (1H, d), 8.21-8.13 (4H, m), 7.99-7.89 (4H, m), 7.77-7.35 (20H, m), 7.20-7.16 (2H, m) |
| 3-32 | δ=8.55 (1H, d), 8.30 (1H, d), 8.21-8.13 (3H, m), 7.99-7.89 (8H, m), 7.77-7.35 (17H, m), 7.20-7.16 (2H, m) |
| 3-37 | δ=8.55 (1H, d), 8.30 (1H, d), 8.21-8.13 (3H, m), 8.03-7.35 (23H, m), 7.20-7.16 (2H, m) |
| 3-42 | δ=8.55 (1H, d), 8.30 (1H, d), 8.19(1H, d) 8.13 (1H, d), 7.99-7.89 (12H, m), 7.77-7.75 (5H, m), 7.50-7.35 (8H, m), 7.20-7.16 (2H, m) |
| 3-82 | δ=8.55 (1H, d), 8.30 (1H, d), 8.19-8.13 (2H, m), 8.03-7.77 (13H, m), 7.59-7.50 (6H, m) 7.36-7.35 (3H, m), 7.20-7.16 (2H, m) |
| 4-2 | δ=8.55(1H, d), 8.45(1H, d), 8.30(1H, d), 8.19(1H, d), 8.13(1H, d), 8.00~7.89(6H, m), 7.77(2H, m), 7.62∼7.35 (15H, m), 7.20~7.16(2H, m) |
| 4-3 | δ=8.55(1H, d), 8.45(1H, d), 8.30(1H, d), 8.19(1H, d), 8.13(1H, d), 8.00~7.89(6H, m), 7.77-7.75(4H, m), 7.62~7.35(13H, m), 7.25~7.16(6H, m) |
| 4-5 | δ=8.55(1H, d), 8.45(1H, d), 8.30(1H, d), 8.19-7.93 (11H, d), 7.77 (2H, d) 7.63-7.49 (12H, m) 7.38-7.35 (2H, m) 7.20~7.16(2H, m) |

### <Experimental Example 1-1>-Manufacture of Organic Light Emitting Device (Green, Single Host)

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl(phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. As the light emitting layer, a compound described as Chemical Formula 1 of the following Table 8 was deposited to 400 Å as a host, and, as a green phosphorescent dopant, Ir(ppy)₃ was doped and deposited by 7% with respect to the deposited thickness of the light emitting layer. After that, BCP was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 10⁻⁸ torr to 10⁻⁶ torr for each material to be used in the OLED manufacture.

For each of the organic electroluminescent devices manufactured as above, electroluminescent (EL) properties were measured using M7000 manufactured by McScience Inc., and with the measurement results, T90 was measured when standard luminance was 6,000 cd/m² through a lifetime measurement system (M6000) manufactured by McScience Inc.. Results of measuring driving voltage, light emission efficiency, color coordinate (CIE) and lifetime of the manufactured organic light emitting devices are as shown in the following Table 8.

**[Table 8]**

| | Light Emitting Layer Compound | Driving Voltage (V) | Efficiency (cd/A) | Light Emitting Color | Lifetime (T90) |
|---|---|---|---|---|---|
| Comparative Example 1 | A | 6.64 | 22.9 | Green | 22 |
| Comparative Example 2 | B | 6.58 | 23.2 | Green | 24 |
| Comparative Example 3 | C | 6.50 | 24.1 | Green | 20 |
| Comparative Example 4 | D | 5.92 | 35.7 | Green | 48 |
| Comparative Example 5 | E | 5.65 | 37.8 | Green | 65 |
| Comparative Example 6 | F | 6.04 | 30.4 | Green | 30 |
| Comparative Example 7 | G | 5.84 | 31.0 | Green | 25 |
| Comparative Example 8 | H | 5.80 | 33.2 | Green | 45 |
| Comparative Example 9 | I | 5.31 | 40.6 | Green | 85 |
| Comparative Example 10 | J | 5.02 | 44.8 | Green | 112 |
| Comparative Example 11 | K | 4.89 | 47.8 | Green | 131 |
| Comparative Example 12 | 3-3 | 5.21 | 47.0 | Green | 55 |
| Comparative Example 13 | 3-4 | 5.75 | 41.1 | Green | 49 |
| Comparative Example 14 | 3-7 | 5.48 | 45.3 | Green | 56 |
| Comparative Example 15 | 3-31 | 5.75 | 41.2 | Green | 41 |
| Comparative Example 16 | 3-32 | 5.48 | 40.2 | Green | 54 |
| Comparative Example 17 | 3-42 | 5.52 | 41.0 | Green | 53 |
| Comparative Example 18 | 4-2 | 5.83 | 42.9 | Green | 45 |
| Comparative Example 19 | 4-3 | 5.84 | 43.0 | Green | 45 |
| Example 1 | 1-1 | 4.45 | 83.2 | Green | 211 |
| Example 2 | 1-2 | 4.43 | 84.1 | Green | 223 |
| Example 3 | 1-3 | 4.45 | 83.2 | Green | 227 |
| Example 4 | 1-34 | 4.27 | 69.2 | Green | 115 |
| Example 5 | 1-35 | 4.24 | 70.2 | Green | 121 |
| Example 6 | 1-38 | 4.29 | 68.4 | Green | 125 |
| Example 7 | 1-43 | 4.36 | 90.6 | Green | 255 |
| Example 8 | 1-46 | 4.31 | 91.5 | Green | 276 |
| Example 9 | 1-47 | 4.28 | 90.8 | Green | 284 |
| Example 10 | 1-51 | 4.22 | 95.5 | Green | 311 |
| Example 11 | 1-61 | 4.39 | 85.6 | Green | 205 |
| Example 12 | 1-62 | 4.36 | 84.5 | Green | 223 |
| Example 13 | 1-63 | 4.38 | 83.2 | Green | 249 |
| Example 14 | 1-72 | 4.22 | 96.4 | Green | 283 |
| Example 15 | 1-73 | 4.17 | 98.6 | Green | 296 |
| Example 16 | 1-86 | 4.38 | 85.8 | Green | 230 |
| Example 17 | 1-91 | 4.23 | 66.6 | Green | 122 |
| Example 18 | 1-100 | 4.30 | 91.2 | Green | 237 |
| Example 19 | 1-102 | 4.25 | 94.1 | Green | 254 |
| Example 20 | 1-106 | 4.22 | 93.3 | Green | 269 |
| Example 21 | 1-121 | 4.51 | 78.6 | Green | 203 |
| Example 22 | 1-122 | 4.46 | 79.5 | Green | 211 |
| Example 23 | 1-141 | 4.15 | 64.0 | Green | 138 |
| Example 24 | 1-151 | 4.50 | 79.7 | Green | 205 |
| Example 25 | 1-152 | 4.42 | 80.2 | Green | 232 |
| Example 26 | 1-171 | 4.17 | 65.1 | Green | 139 |
| Example 27 | 1-174 | 4.30 | 88.8 | Green | 267 |
| Example 28 | 1-181 | 4.47 | 79.9 | Green | 253 |
| Example 29 | 1-198 | 4.38 | 88.2 | Green | 243 |
| Example 30 | 1-201 | 4.18 | 64.3 | Green | 122 |
| Example 31 | 1-211 | 4.43 | 80.4 | Green | 212 |
| Example 32 | 1-224 | 4.40 | 84.1 | Green | 229 |
| Example 33 | 2-1 | 4.31 | 84.7 | Green | 189 |
| Example 34 | 2-2 | 4.29 | 85.6 | Green | 202 |
| Example 35 | 2-3 | 4.30 | 84.0 | Green | 195 |
| Example 36 | 2-34 | 4.11 | 70.0 | Green | 111 |
| Example 37 | 2-35 | 4.09 | 71.3 | Green | 116 |
| Example 38 | 2-38 | 4.15 | 69.1 | Green | 119 |
| Example 39 | 2-43 | 4.23 | 92.9 | Green | 237 |
| Example 40 | 2-46 | 4.16 | 93.7 | Green | 248 |
| Example 41 | 2-47 | 4.13 | 92.6 | Green | 251 |
| Example 42 | 2-51 | 4.06 | 98.2 | Green | 280 |
| Example 43 | 2-61 | 4.19 | 86.8 | Green | 198 |
| Example 44 | 2-62 | 4.20 | 86.2 | Green | 210 |
| Example 45 | 2-63 | 4.11 | 84.3 | Green | 232 |
| Example 46 | 2-72 | 4.06 | 99.5 | Green | 263 |
| Example 47 | 2-73 | 4.01 | 100.7 | Green | 272 |
| Example 48 | 2-86 | 4.24 | 87.3 | Green | 209 |
| Example 49 | 2-91 | 4.10 | 67.5 | Green | 118 |
| Example 50 | 2-100 | 4.14 | 94.0 | Green | 212 |
| Example 51 | 2-102 | 4.09 | 96.3 | Green | 219 |
| Example 52 | 2-106 | 4.04 | 96.9 | Green | 240 |
| Example 53 | 2-121 | 4.31 | 79.8 | Green | 175 |
| Example 54 | 2-122 | 4.29 | 81.3 | Green | 182 |
| Example 55 | 2-141 | 3.99 | 65.1 | Green | 111 |
| Example 56 | 2-151 | 4.33 | 80.9 | Green | 177 |
| Example 57 | 2-152 | 4.29 | 82.1 | Green | 203 |
| Example 58 | 2-171 | 4.01 | 66.3 | Green | 121 |
| Example 59 | 2-174 | 4.16 | 90.5 | Green | 243 |
| Example 60 | 2-181 | 4.30 | 82.3 | Green | 222 |
| Example 61 | 2-198 | 4.14 | 90.6 | Green | 219 |
| Example 62 | 2-201 | 4.02 | 65.6 | Green | 112 |
| Example 63 | 2-211 | 4.28 | 83.1 | Green | 200 |
| Example 64 | 2-224 | 4.26 | 86.6 | Green | 214 |
| Example 65 | 2-234 | 4.10 | 90.1 | Green | 234 |

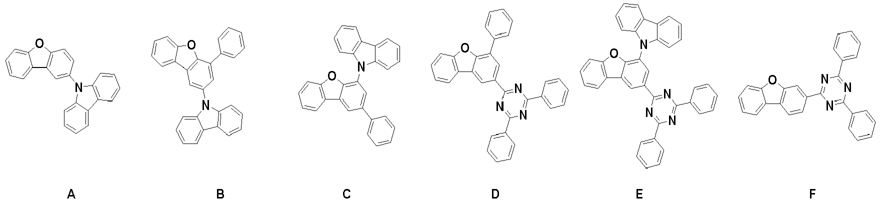

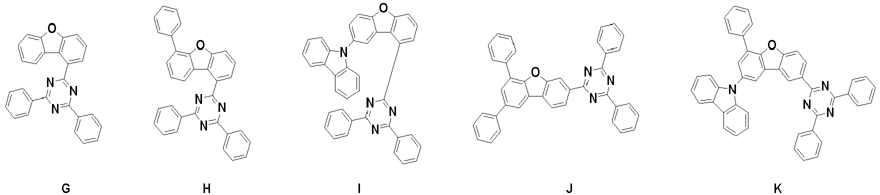

### <Experimental Example 1-2> Manufacture of Organic Light Emitting Device (Green, Pre-mixed Host)

A glass substrate on which ITO was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl(phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. As the light emitting layer, one type of compound described as Chemical Formula 1 described in the following Table 9 and one type of compound described as Chemical Formula 2 described in the following Table 9 were premixed and then deposited to 400 Å in one source of supply as a host, and, as a green phosphorescent dopant, Ir(ppy)₃ was doped and deposited by 7% with respect to the deposited thickness of the light emitting layer. After that, BCP was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 10⁻⁸ torr to 10⁻⁶ torr for each material to be used in the OLED manufacture.

For each of the organic electroluminescent devices manufactured as above, electroluminescent (EL) properties were measured using M7000 manufactured by McScience Inc., and with the measurement results, T90 was measured when standard luminance was 6,000 cd/m² through a lifetime measurement system (M6000) manufactured by McScience Inc..

Results of measuring driving voltage, light emission efficiency, color coordinate (CIE) and lifetime of the organic light emitting devices manufactured according to the present disclosure are as shown in the following Table 9.

**[Table 9]**

| | Light Emitting Layer Compound | | Ratio | Driving Voltage (V) | Efficiency (cd/A) | Light Emitting Color | Lifetime (T90) |
|---|---|---|---|---|---|---|---|
| Example 65 | 1-1 | 3-4 | 1:8 | 5.12 | 61.7 | Green | 88 |
| Example 66 | | | 1:5 | 4.75 | 73.6 | Green | 149 |
| Example 67 | | | 1:2 | 4.38 | 86.0 | Green | 402 |
| Example 68 | | | 1:1 | 4.32 | 87.2 | Green | 369 |
| Example 69 | | | 2:1 | 4.29 | 88.4 | Green | 344 |
| Example 70 | | | 5:1 | 4.40 | 83.9 | Green | 236 |
| Example 70-1 | | | 8:1 | 4.42 | 83.2 | Green | 211 |
| Example 71 | | 4-2 | 1:2 | 4.53 | 87.2 | Green | 432 |
| Example 72 | | | 1:1 | 4.39 | 90.1 | Green | 400 |
| Example 73 | | | 2:1 | 4.31 | 91.7 | Green | 374 |
| Example 74 | 1-2 | 3-31 | 1:3 | 4.53 | 84.1 | Green | 441 |
| Example 75 | | | 1:2 | 4.28 | 86.0 | Green | 424 |
| Example 76 | | | 1:1 | 4.22 | 87.2 | Green | 390 |
| Example 77 | | | 2:1 | 4.17 | 88.9 | Green | 364 |
| Example 78 | | | 3:1 | 4.26 | 76.1 | Green | 255 |
| Example 79 | 1-47 | 3-42 | 1:2 | 4.37 | 91.8 | Green | 451 |
| Example 80 | | | 1:1 | 4.19 | 93.6 | Green | 425 |
| Example 81 | | | 2:1 | 4.13 | 94.8 | Green | 393 |
| Example 82 | 1-61 | 3-3 | 1:2 | 4.29 | 87.6 | Green | 381 |
| Example 83 | | | 1:1 | 4.22 | 89.0 | Green | 362 |
| Example 84 | | | 2:1 | 4.16 | 89.6 | Green | 335 |
| Example 85 | 1-122 | 3-32 | 1:2 | 4.29 | 88.5 | Green | 372 |
| Example 86 | | | 1:1 | 4.20 | 89.7 | Green | 384 |
| Example 87 | | | 2:1 | 4.13 | 91.6 | Green | 326 |
| Example 88 | 2-1 | 3-3 | 1:2 | 4.21 | 87.9 | Green | 388 |
| Example 89 | | | 1:1 | 4.14 | 89.2 | Green | 350 |
| Example 90 | | | 2:1 | 4.08 | 90.5 | Green | 332 |
| Example 91 | 2-1 | 4-2 | 1:2 | 4.46 | 88.6 | Green | 417 |
| Example 92 | | | 1:1 | 4.37 | 91.2 | Green | 383 |
| Example 93 | | | 2:1 | 4.25 | 93.5 | Green | 350 |
| Example 94 | 2-3 | 3-7 | 1:2 | 4.20 | 87.7 | Green | 410 |
| Example 95 | | | 1:1 | 4.10 | 90.1 | Green | 375 |
| Example 96 | | | 2:1 | 4.04 | 91.4 | Green | 346 |
| Example 97 | 2-63 | 3-31 | 1:2 | 4.23 | 88.1 | Green | 384 |
| Example 98 | | | 1:1 | 4.11 | 91.3 | Green | 359 |
| Example 99 | | | 2:1 | 4.02 | 92.2 | Green | 342 |
| Example 100 | 2-73 | 4-3 | 1:2 | 4.43 | 91.9 | Green | 462 |
| Example 101 | | | 1:1 | 4.34 | 93.3 | Green | 430 |
| Example 102 | | | 2:1 | 4.22 | 95.1 | Green | 388 |
| Example 103 | 2-100 | 3-42 | 1:2 | 4.27 | 88.9 | Green | 414 |
| Example 104 | | | 1:1 | 4.16 | 91.6 | Green | 384 |
| Example 105 | | | 2:1 | 4.09 | 93.0 | Green | 352 |
| Example 106 | 2-106 | 3-31 | 1:2 | 4.17 | 92.2 | Green | 401 |
| Example 107 | | | 1:1 | 4.06 | 95.0 | Green | 361 |
| Example 108 | | | 2:1 | 3.98 | 97.5 | Green | 338 |

### <Experimental Example 2-1>-Manufacture of Organic Light Emitting Device (Red, Single Host)

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and ultraviolet ozone (UVO) treatment was conducted for 5 minutes using UV in an ultraviolet cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl(phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. The light emitting layer was deposited to 500 Å using a compound corresponding to Chemical Formula 1 described in the following Table 10 as a red host, and doping (piq)₂(Ir) (acac) to the host by 3% as a red phosphorescent dopant. After that, BCP was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 10⁻⁸ torr to 10⁻⁶ torr for each material to be used in the OLED manufacture.

For each of the organic electroluminescent devices manufactured as above, electroluminescent (EL) properties were measured using M7000 manufactured by McScience Inc., and with the measurement results, T90 was measured when standard luminance was 6,000 cd/m² through a lifetime measurement system (M6000) manufactured by McScience Inc.. Properties of the organic electroluminescent devices of the present disclosure are as shown in the following Table 10.

**[Table 10]**

| | Light Emitting Layer Compound | Driving Voltage (V) | Efficiency (cd/A) | Light Emitting Color | Lifetime (T90) |
|---|---|---|---|---|---|
| Comparative Example 20 | L | 5.83 | 15.7 | Red | 20 |
| Comparative Example 21 | M | 5.81 | 16.3 | Red | 41 |
| Comparative Example 22 | N | 5.77 | 18.4 | Red | 44 |
| Comparative Example 23 | O | 5.22 | 23.2 | Red | 88 |
| Comparative Example 24 | P | 4.95 | 28.6 | Red | 157 |
| Comparative Example 25 | Q | 5.34 | 20.5 | Red | 105 |
| Comparative Example 26 | R | 5.14 | 19.1 | Red | 97 |
| Comparative Example 27 | S | 5.10 | 23.4 | Red | 140 |
| Comparative Example 28 | T | 4.61 | 26.8 | Red | 182 |
| Comparative Example 29 | U | 4.32 | 28.7 | Red | 168 |
| Comparative Example 30 | V | 4.28 | 28.2 | Red | 203 |
| Comparative Example 31 | 3-9 | 5.19 | 17.0 | Red | 64 |
| Comparative Example 32 | 3-37 | 5.09 | 21.2 | Red | 50 |
| Comparative Example 33 | 3-82 | 5.03 | 22.4 | Red | 48 |
| Comparative Example 34 | 4-5 | 5.63 | 23.0 | Red | 51 |
| Example 109 | 1-19 | 3.70 | 42.3 | Red | 432 |
| Example 110 | 1-21 | 3.88 | 38.2 | Red | 388 |
| Example 111 | 1-24 | 3.85 | 40.9 | Red | 403 |
| Example 112 | 1-25 | 3.83 | 41.3 | Red | 415 |
| Example 113 | 1-34 | 3.64 | 38.5 | Red | 389 |
| Example 114 | 1-35 | 3.61 | 39.2 | Red | 401 |
| Example 115 | 1-38 | 3.65 | 37.9 | Red | 365 |
| Example 116 | 1-57 | 3.75 | 41.4 | Red | 424 |
| Example 117 | 1-59 | 3.76 | 40.8 | Red | 401 |
| Example 118 | 1-77 | 3.71 | 42.0 | Red | 420 |
| Example 119 | 1-79 | 3.92 | 37.9 | Red | 372 |
| Example 120 | 1-91 | 3.68 | 38.1 | Red | 375 |
| Example 121 | 1-114 | 3.74 | 41.8 | Red | 425 |
| Example 122 | 1-130 | 3.75 | 40.6 | Red | 411 |
| Example 123 | 1-141 | 3.66 | 36.5 | Red | 366 |
| Example 124 | 1-160 | 3.90 | 39.8 | Red | 404 |
| Example 125 | 1-171 | 3.67 | 36.6 | Red | 360 |
| Example 126 | 1-177 | 3.84 | 41.0 | Red | 413 |
| Example 127 | 1-190 | 3.86 | 41.0 | Red | 406 |
| Example 128 | 1-201 | 3.65 | 36.2 | Red | 351 |
| Example 129 | 1-220 | 3.79 | 40.3 | Red | 402 |
| Example 130 | 2-19 | 3.68 | 43.6 | Red | 420 |
| Example 131 | 2-21 | 3.86 | 39.5 | Red | 374 |
| Example 132 | 2-24 | 3.83 | 42.2 | Red | 388 |
| Example 133 | 2-25 | 3.80 | 42.6 | Red | 401 |
| Example 134 | 2-34 | 3.62 | 39.7 | Red | 373 |
| Example 135 | 2-35 | 3.58 | 40.4 | Red | 386 |
| Example 136 | 2-38 | 3.61 | 39.3 | Red | 352 |
| Example 137 | 2-57 | 3.73 | 43.1 | Red | 412 |
| Example 138 | 2-59 | 3.73 | 42.3 | Red | 387 |
| Example 139 | 2-77 | 3.68 | 43.3 | Red | 408 |
| Example 140 | 2-79 | 3.88 | 39.1 | Red | 360 |
| Example 141 | 2-91 | 3.62 | 39.5 | Red | 363 |
| Example 142 | 2-114 | 3.73 | 43.8 | Red | 414 |
| Example 143 | 2-130 | 3.71 | 42.0 | Red | 402 |
| Example 144 | 2-141 | 3.63 | 37.7 | Red | 352 |
| Example 145 | 2-160 | 3.85 | 40.9 | Red | 393 |
| Example 146 | 2-171 | 3.62 | 38.2 | Red | 349 |
| Example 147 | 2-177 | 3.79 | 42.7 | Red | 401 |
| Example 148 | 2-190 | 3.83 | 43.0 | Red | 393 |
| Example 149 | 2-201 | 3.61 | 37.8 | Red | 337 |
| Example 150 | 2-220 | 3.73 | 41.5 | Red | 388 |

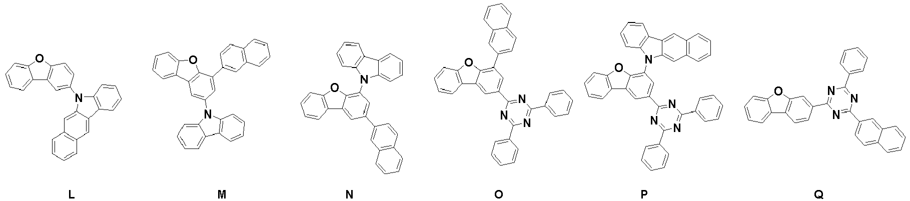

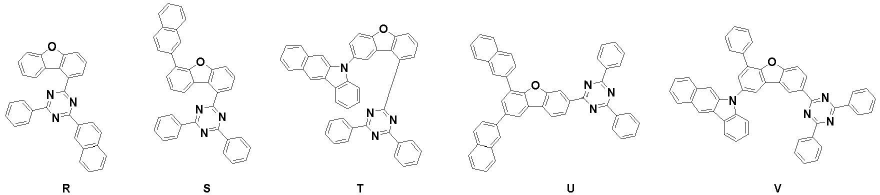

### <Experimental Example 2-2>-Manufacture of Organic Light Emitting Device (Red, Pre-mixed Host)

A glass substrate on which ITO was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), a hole injection layer 2-TNATA (4,4',4"-tris[2-naphthyl(phenyl)amino]triphenylamine) and a hole transfer layer NPB (N,N'-di(1-naphthyl)-N,N'-diphenyl-(1,1'-biphenyl)-4,4'-diamine), which are common layers, were formed.

A light emitting layer was thermal vacuum deposited thereon as follows. As the light emitting layer, two types corresponding to Compound 1 and Compound 2 as described in the following Table 11 were premixed and then deposited to 400 Å in one source of supply as a red host, and (piq)₂(Ir) (acac) was doped and deposited by 3% as a red phosphorescent dopant. After that, BCP was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

Meanwhile, all the organic compounds required to manufacture the OLED were vacuum sublimation purified under 10⁻⁸ torr to 10⁻⁶ torr for each material to be used in the OLED manufacture.

For each of the organic electroluminescent devices manufactured as above, electroluminescent (EL) properties were measured using M7000 manufactured by McScience Inc., and with the measurement results, T90 was measured when standard luminance was 6,000 cd/m² through a lifetime measurement system (M6000) manufactured by McScience Inc.. Properties of the organic electroluminescent devices of the present disclosure are as shown in the following Table 11.

**[Table 11]**

| | Light Emitting Layer Compound | | Ratio | Driving Voltage (V) | Efficien cy (cd/A) | Light Emitting Color | Lifetim e (T90) |
|---|---|---|---|---|---|---|---|
| Example 151 | 1-19 | 3-9 | 1:8 | 4.66 | 29.4 | Red | 175 |
| Example 152 | | | 1:5 | 3.86 | 40.3 | Red | 420 |
| Example 153 | | | 1:2 | 3.55 | 47.7 | Red | 507 |
| Example 154 | | | 1:1 | 3.52 | 46.8 | Red | 575 |
| Example 155 | | | 2:1 | 3.59 | 46.0 | Red | 520 |
| Example 156 | | | 5:1 | 3.72 | 43.0 | Red | 457 |
| Example 157 | | | 8:1 | 3.79 | 39.4 | Red | 373 |
| Example 158 | | 4-5 | 1:2 | 3.92 | 39.6 | Red | 499 |
| Example 159 | | | 1:1 | 3.74 | 45.6 | Red | 620 |
| Example 160 | | | 2:1 | 3.79 | 44.7 | Red | 551 |
| Example 161 | 1-21 | 3-9 | 1:3 | 3.81 | 37.0 | Red | 320 |
| Example 162 | | | 1:2 | 3.66 | 41.6 | Red | 467 |
| Example 163 | | | 1:1 | 3.63 | 42.2 | Red | 522 |
| Example 164 | | | 2:1 | 3.65 | 41.9 | Red | 501 |
| Example 165 | | | 3:1 | 3.80 | 39.2 | Red | 410 |
| Example 166 | 1-34 | 3-82 | 1:2 | 3.45 | 44.3 | Red | 581 |
| Example 167 | | | 1:1 | 3.46 | 43.1 | Red | 530 |
| Example 168 | | | 2:1 | 3.52 | 42.4 | Red | 479 |
| Example 169 | 1-59 | 3-37 | 1:2 | 3.50 | 45.5 | Red | 566 |
| Example 170 | | | 1:1 | 3.52 | 45.0 | Red | 541 |
| Example 171 | | | 2:1 | 3.60 | 43.1 | Red | 472 |
| Example 172 | 1-77 | 3-9 | 1:2 | 3.66 | 43.6 | Red | 490 |
| Example 173 | | | 1:1 | 3.56 | 45.2 | Red | 537 |
| Example 174 | | | 2:1 | 3.57 | 44.9 | Red | 511 |
| Example 175 | 1-130 | 3-9 | 1:2 | 3.51 | 45.7 | Red | 556 |
| Example 176 | | | 1:1 | 3.60 | 43.2 | Red | 491 |
| Example 177 | | | 2:1 | 3.69 | 42.5 | Red | 459 |
| Example 178 | 1-190 | 3-37 | 1:2 | 4.02 | 34.1 | Red | 294 |
| Example 179 | | | 1:1 | 3.68 | 41.7 | Red | 441 |
| Example 180 | | | 2:1 | 3.60 | 44.2 | Red | 520 |
| Example 181 | 2-19 | 3-9 | 1:2 | 3.40 | 49.5 | Red | 519 |
| Example 182 | | | 1:1 | 3.45 | 47.7 | Red | 500 |
| Example 183 | | | 2:1 | 3.57 | 45.9 | Red | 447 |
| Example 184 | 2-21 | 4-5 | 1:2 | 4.10 | 42.9 | Red | 490 |
| Example 185 | | | 1:1 | 3.90 | 43.8 | Red | 531 |
| Example 186 | | | 2:1 | 3.92 | 43.4 | Red | 467 |
| Example 187 | 2-34 | 3-82 | 1:2 | 3.61 | 43.9 | Red | 501 |
| Example 188 | | | 1:1 | 3.39 | 44.1 | Red | 462 |
| Example 189 | | | 2:1 | 3.47 | 42.5 | Red | 401 |
| Example 190 | 2-57 | 3-37 | 1:2 | 3.64 | 44.0 | Red | 539 |
| Example 191 | | | 1:1 | 3.49 | 48.2 | Red | 510 |
| Example 192 | | | 2:1 | 3.56 | 46.8 | Red | 460 |
| Example 193 | 2-77 | 3-9 | 1:2 | 3.55 | 45.4 | Red | 488 |
| Example 194 | | | 1:1 | 3.35 | 47.8 | Red | 480 |
| Example 195 | | | 2:1 | 3.42 | 46.0 | Red | 458 |

The compounds from Example 1 to Example 32 of Table 8 are materials having triazine bonding to a No. 3 position of the dibenzofuran core. In this case, overall linearity of the material increases, and a dipole moment of the material is more strengthened. As a result, an electron withdrawing effect, a property that a strong ET unit comprising triazine has, more strongly reveals, and delocalized electrons of the dibenzofuran that is a core structure are further withdrawn toward the ET unit.

Herein, the LUMO site of the host material is positioned centering on the triazine that is the ET unit, and when electrons are strongly attracted, an area overlapping with the HOMO site significantly decreases around the core. In addition, electron density in the HOMO site decreases since electrons present in the area are localized toward the LUMO site. Accordingly, charge transfer in the molecule caused by the overlap between LUMO-HOMO also decreases leading to more increased stability of the molecular structure, and as a result, it was seen that the device lifetime significantly increased.

However, the material having triazine bonding to a No. 3 position of the dibenzofuran core has more reduced degree of electron delocalization, and tends to have a slightly increased driving voltage compared to when substituting the No. 1 position of the dibenzofuran core. This may be adjusted to a certain level depending on the position of the carbazole-based substituent or Ar of Chemical Formula 1 on the opposite side of the core structure or the intensity of the donor effect.

The carbazole-based substituent, another substituent forming the bipolar host system, is a good host material having a high T1 energy level based on a strong donor effect (T1=3.00 eV). A major HOMO site is located, and the donor effect may be controlled through substitution/unsubstitution of the carbazole group itself. Moreover, substitution of the carbazole group may contribute to increasing stability of the molecule itself and thermal properties (Tg, Tm) by increasing the molecular weight.

Particularly, in maintaining a charge balance that is most important in an OLED, substitution of the carbazole group plays an important role in the corresponding host structure in order to control the flow of holes having faster mobility than electrons, and through this, the device structure may be diversified, and even with changes in the work function caused by the device thickness and changes in the hole injection layer and the hole transfer layer, the HOMO level of the material may be adjusted to a certain level through the substitution of the carbazole group. Through this, it was identified that the driving voltage was effectively maintained low by eliminating an energy barrier with the hole transfer layer and thereby improving the flow of holes in the device.

When comparing substitution between the carbazole-based substituent and Ar of Chemical Formula 1, it was identified that an overall driving voltage decreased when substituted with the carbazole group. This is due to the fact that resistance at the interface relatively decreases as the HOMO level that is work function of the carbazole group becomes similar to the HOMO level of the adjacent hole transfer layer. Through this, it was identified that the turn on voltage decreased as the flow of holes was improved, and the driving voltage decreased as well.

The compounds from Example 33 to Example 65 of Table 8 are materials having triazine bonding to a No. 1 position of the dibenzofuran core. In this case, relatively slightly decreased linearity is obtained, however, an effect of increasing or decreasing linearity to a certain level is obtained depending on the substituted position of the carbazole group or Ar of Chemical Formula 1 locating on the opposite side of the dibenzofuran.

Accordingly, some electron withdrawing effect is obtained, however, charge transfer in the molecule is more active since the degree of electron delocalization tends to be higher. From the experimental results, it was identified that the color coordinate in the same device structure was further red shifted meaning that the bandgap of the material became narrower due to the occurrence of charge transfer. It was identified that driving voltage and current efficiency in the device were more superior due to the presence of delocalized electrons and their active charge transfer effect.

Table 10 shows results of manufacturing and measuring red light emitting devices. A metal complex and an organic host material all had a low T1 energy level in the red devices compared to in the green devices. Accordingly, the red devices had properties of relatively low current efficiency and long lifetime compared to the green devices.

In order to prepare a material having a proper energy level used in the red device around the same core, a conjugation area of the material needs to be adjusted, and, by changing each of the carbazole-based substituent that is a HT unit and the substituents of the triazine that is an ET unit, which were in use, to a polycyclic fused cyclic group, the conjugation area of the material was expanded, and through this, a red phosphorescent host material having properties of relatively the same long lifetime and high efficiency was able to be designed.

Particularly, the polycyclic fusion introduced to the carbazole group maintains fast hole properties that the carbazole group has and enables adjustment to a T1 energy level at which red phosphorescence is expressed. At the same time, molecular stability may also be enhanced since a wider conjugation area may be secured by having a form of fused ring. Herein, the energy level of the HOMO level may be adjusted to a certain level by substituting surroundings of the carbazole group. Accordingly, it was identified that an advantage of lowering a driving voltage of the whole device was obtained by adjusting to have work function close to the hole transfer layer.

In addition, the fused substituent such as dibenzofuran, dibenzothiophene or a triphenylene group introduced to the triazine group is capable of strengthening electron withdrawing properties that the triazine has. Apart from the presence or absence of a heteroatom, the LUMO level may be expanded by securing a wide conjugation area through introducing a multiple fused ring having a stable structure. Through this, electron mobility is more strengthened serving to maintain sufficient current efficiency even at a low voltage, and an adjustment to a proper T1 energy level required for red light emission is also achieved.

Since various colors may be obtained in the same structure depending on the fused ring introduction of substituents with the existing structure as a base, expandability of the corresponding structure is endless, and there is an advantage of building a similar type of device even when introducing a RGB type or various device structures such as a 2-stack and a 3-stack.

Materials having proper work function may be prepared depending on the substituent position, and therefore, it may be used as a factor adjusting electron mobility of the whole device as well as adjusting an energy level even when building a new device in the future.

In Table 9 and Table 11, the heterocyclic compound of Chemical Formula 1 and the heterocyclic compound of Chemical Formula 2 go through a pre-mixing process of mixing the materials first in one source of supply before being deposited to the organic material layer. In the pre-mixing, one source of deposition is used instead of using 2 to 3 sources of deposition, which has an advantage of more simplifying the process.

In the pre-mixing as above, unique thermal properties of each of the materials need to checked before the mixing, and herein, when depositing the pre-mixed host material from one source of deposition, deposition conditions comprising a deposition rate may be greatly affected depending on the unique thermal properties of the material. Repeatability and reproducibility may not be maintained in the deposition process when thermal properties of pre-mixed two or more types of the materials are not similar and are different, which means that it may not be possible to manufacture an OLED that is all uniform in one deposition process.

In order to overcome such an issue, thermal properties may be adjusted depending on the form of a molecular structure as well as coordinating electrical properties of the material using a proper combination of a basic structure and substituents of each material. Accordingly, diversity of various pre-mixing deposition processes between host-host may be secured by adjusting thermal properties of each material while aiming to enhance device performance by using, as well as bonding with carbon that bonds to N of the carbazole as in Chemical Formula 2, various substituents in Chemical Formula 2 in addition to the basic skeleton. This also has an advantage of securing diversity of a pre-mixing deposition process using three, four or more host materials as well as two compounds as a host.

Mixing of the heterocyclic compound of Chemical Formula 1 and the heterocyclic compound of Chemical Formula 2 means that two or more types of materials may be mixed, and the mixing is not limited thereto with the experimental examples described above being just a representative example.

As seen from Table 9 and Table 11, it was identified that, through mixing the heterocyclic compound of Chemical Formula 1 and the heterocyclic compound of Chemical Formula 2, a device with properties of long lifetime was able to be built while obtaining an effect of partially improving current efficiency of the light emitting layer. From the results of Table 9 and Table 11, it was identified that effects of more superior efficiency and lifetime were obtained when comprising the heterocyclic compound of Chemical Formula 1 and the heterocyclic compound of Chemical Formula 2 at the same time. This is due to an exciplex phenomenon occurring when two or more materials are mixed. The exciplex phenomenon is a phenomenon of releasing energy having sizes of a donor (p-host) HOMO level and an acceptor (n-host) LUMO level due to electron exchanges between two molecules.

When the exciplex phenomenon occurs between two molecules, reverse intersystem crossing (RISC) occurs, and as a result, internal quantum efficiency of fluorescence may increase up to 100%. When a donor (p-host) having a favorable hole transfer ability and an acceptor (n-host) having a favorable electron transfer ability are used as a host of a light emitting layer, holes are injected to the p-host and electrons are injected to the n-host, and herein, the excitons are not quenched due to the electron exchanges between molecules, and a lifetime of the excitons that is capable of having energy increases. As a result, overall current efficiency may be improved, and the device lifetime may be enhanced. In the invention of the present application, it was identified that excellent device properties were obtained when the heterocyclic compound of Chemical Formula 2 performing a donor role and the heterocyclic compound of Chemical Formula 1 performing an acceptor role were used as the light emitting layer host.

## Claims

1. A heterocyclic compound represented by the following Chemical Formula 1: 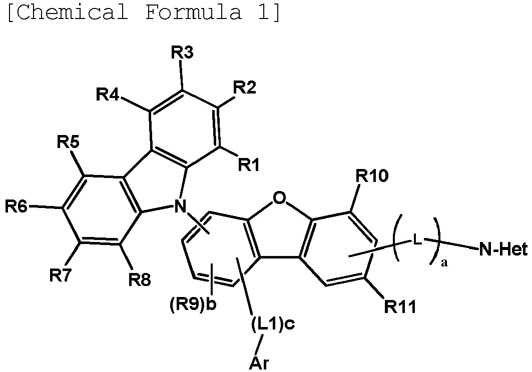 wherein, in Chemical Formula 1,
N-Het is a monocyclic or polycyclic C2 to C60 heterocyclic group substituted or unsubstituted, and comprising one or more Ns;
L and L1 are the same as or different from each other, and each independently a direct bond; a substituted or unsubstituted C6 to C60 arylene group; or a substituted or unsubstituted C2 to C60 heteroarylene group;
Ar is a substituted or unsubstituted C1 to C60 alkyl group; a substituted or unsubstituted C6 to C60 aryl group; or a substituted or unsubstituted C2 to C60 heteroaryl group;
R1 to R11 are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -P(=O)RR'; -SiRR'R"; and -NRR', or two or more groups adjacent to each other bond to each other to form a substituted or unsubstituted C6 to C60 aromatic hydrocarbon ring or a substituted or unsubstituted C2 to C60 heteroring;
R, R' and R" are the same as or different from each other, and each independently a substituted or unsubstituted C1 to C60 alkyl group; a substituted or unsubstituted C6 to C60 aryl group; or a substituted or unsubstituted C2 to C60 heteroaryl group;
a and c are an integer of 0 to 4; and
b is an integer of 0 to 2.

2. The heterocyclic compound of Claim 1, wherein Chemical Formula 1 is represented by the following Chemical Formula 3 or 4: 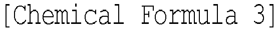 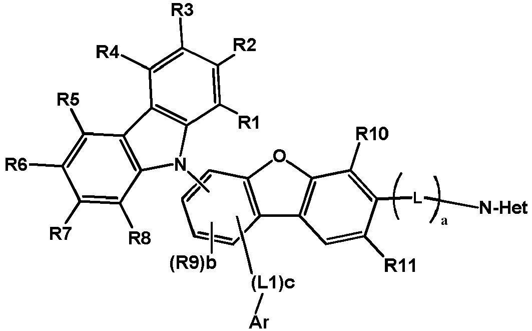 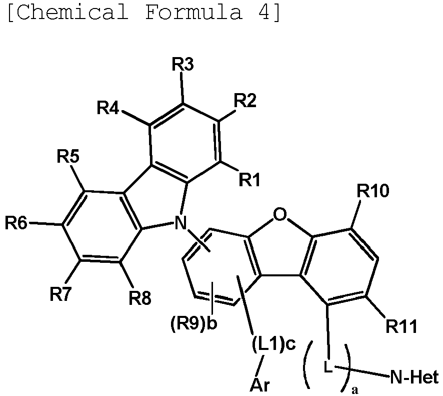 in Chemical Formulae 3 and 4,
R1 to R11, N-Het, L, L1, Ar, a, b and c have the same definitions as in Chemical Formula 1.

3. The heterocyclic compound of Claim 2, wherein Chemical Formula 3 is represented by any one of the following Chemical Formulae 3-1 to 3-6: 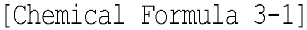 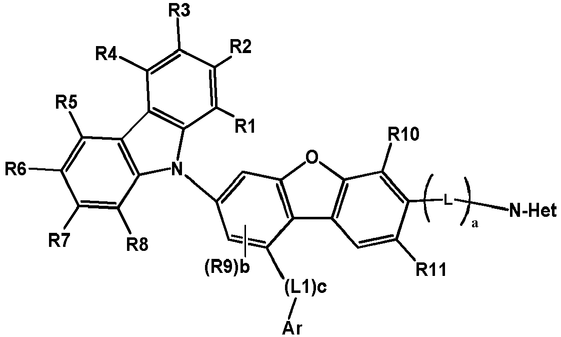 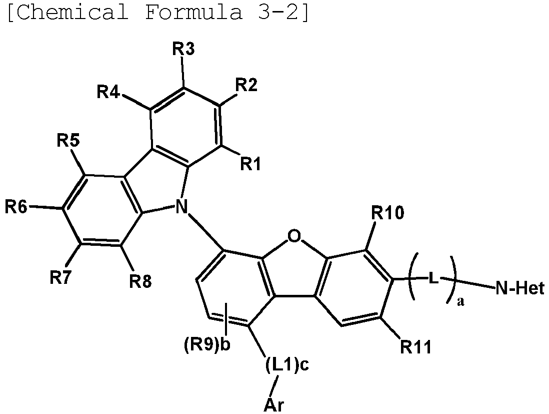 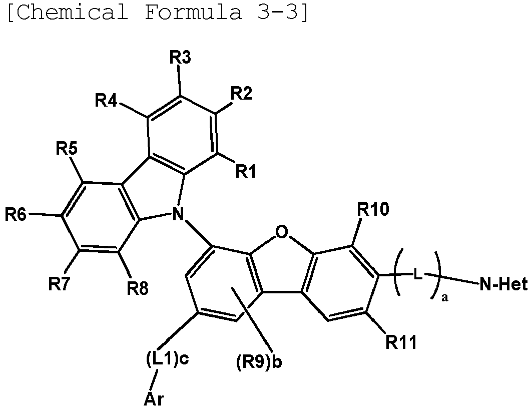 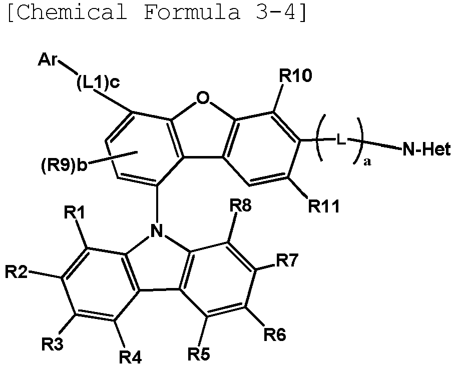 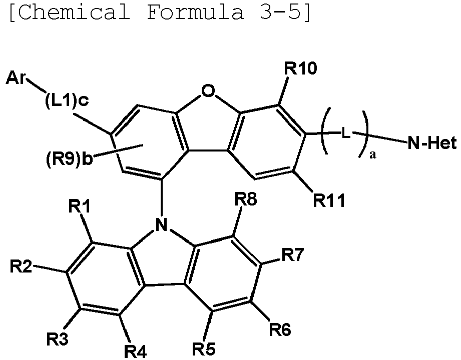 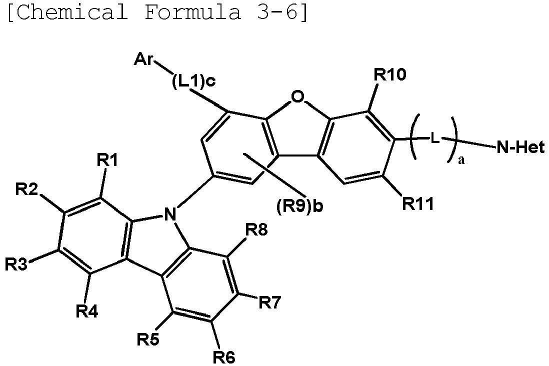 in Chemical Formulae 3-1 to 3-6,
R1 to R11, N-Het, L, L1, Ar, a, b and c have the same definitions as in Chemical Formula 3.

4. The heterocyclic compound of Claim 2, wherein Chemical Formula 4 is represented by any one of the following Chemical Formulae 4-1 to 4-6: 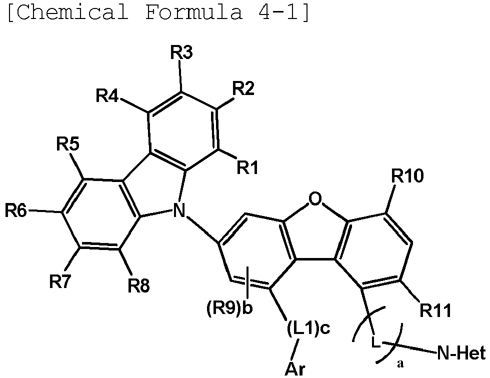 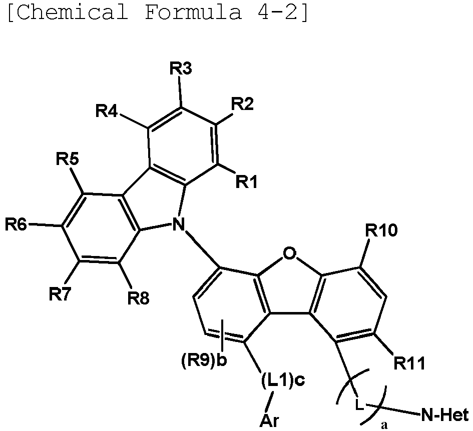 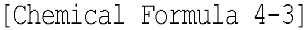 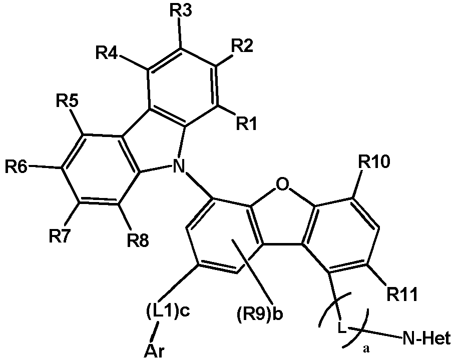 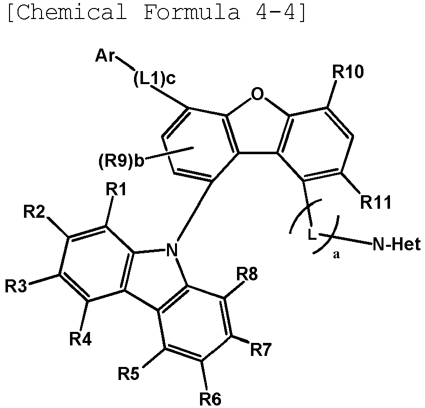 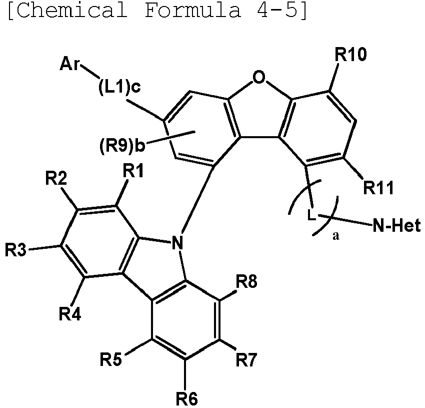 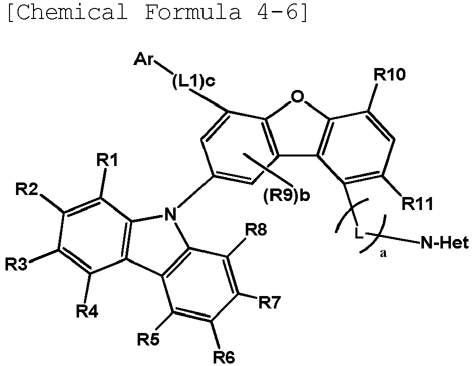 in Chemical Formulae 4-1 to 4-6,
R1 to R11, N-Het, L, L1, Ar, a, b and c have the same definitions as in Chemical Formula 4.

5. The heterocyclic compound of Claim 1, wherein Ar of Chemical Formula 1 is any one of the following structures: 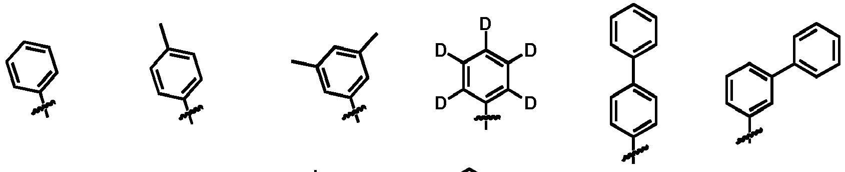 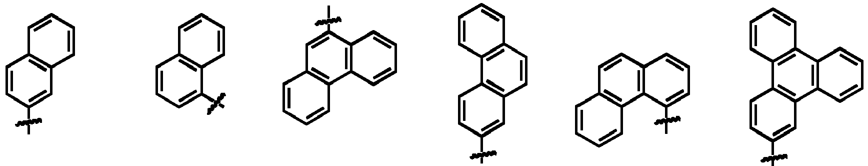 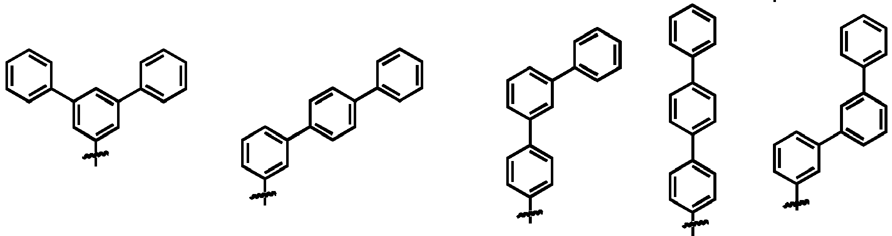 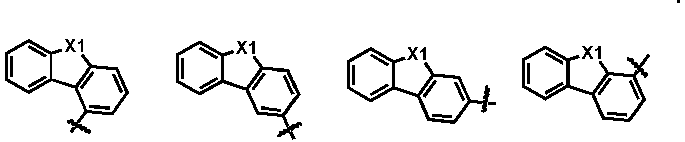 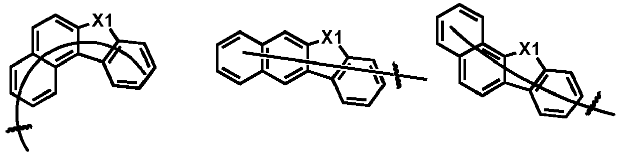 in the structural formulae,
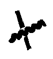 means a site linked to L1; and
X1 is O; or S.

6. The heterocyclic compound of Claim 1, wherein Chemical Formula 1 is represented by any one of the following compounds:

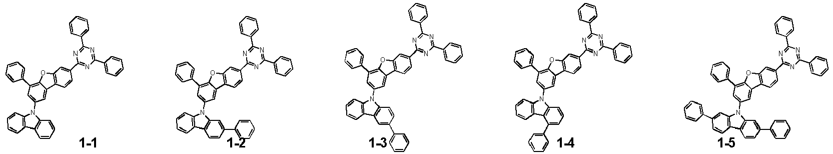

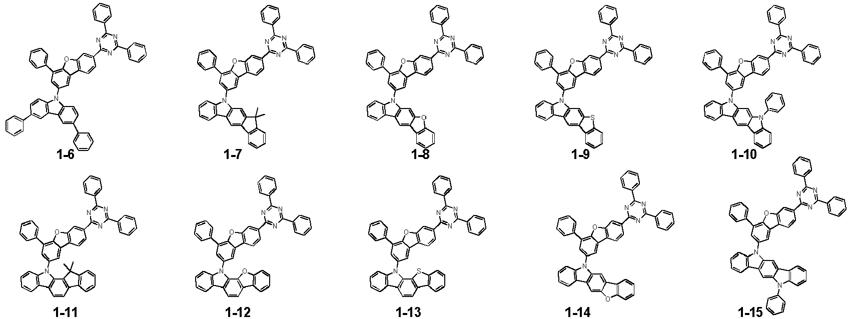

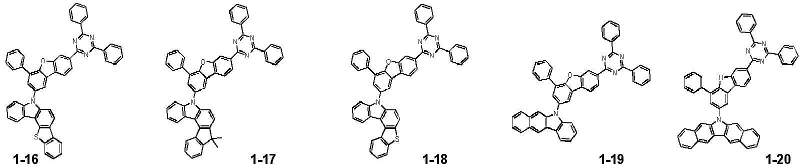

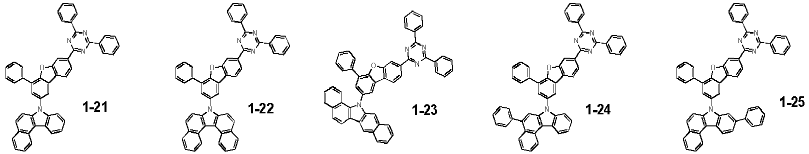

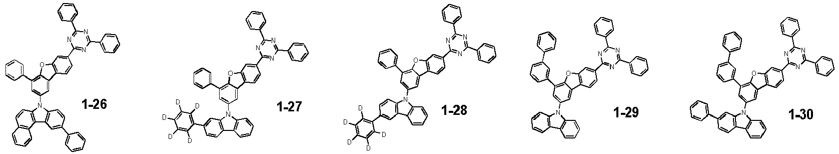

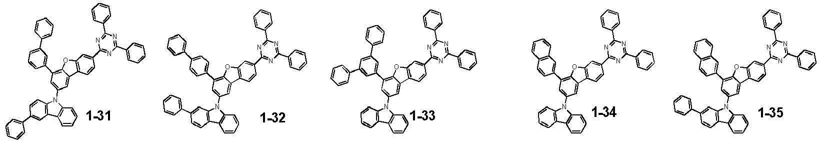

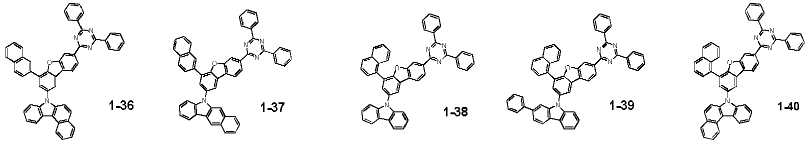

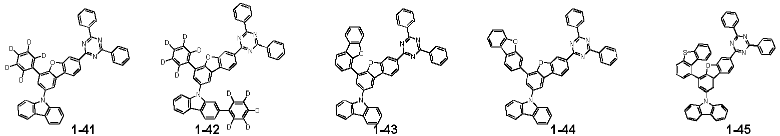

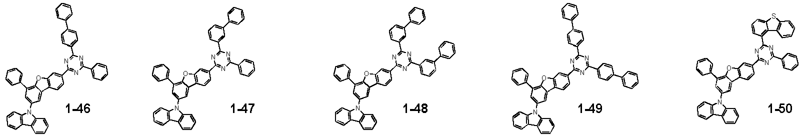

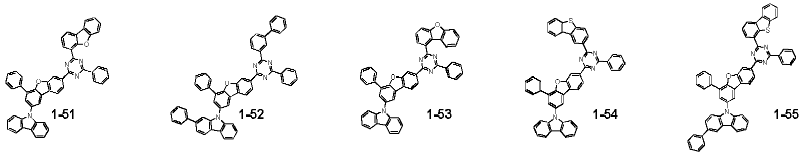

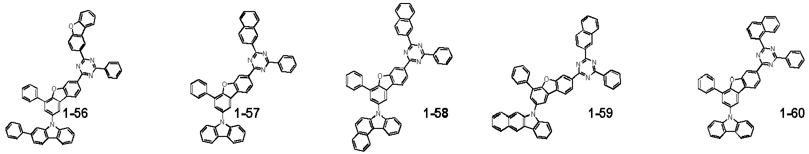

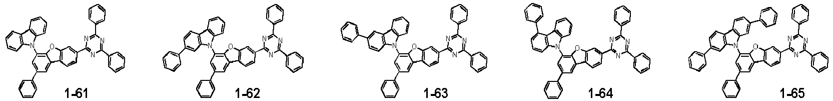

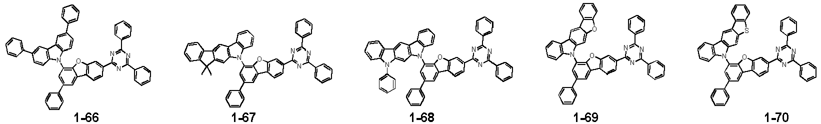

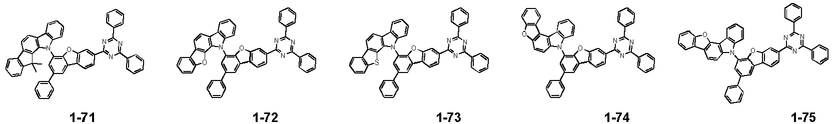

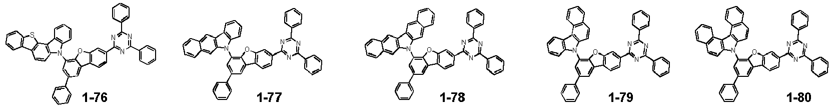

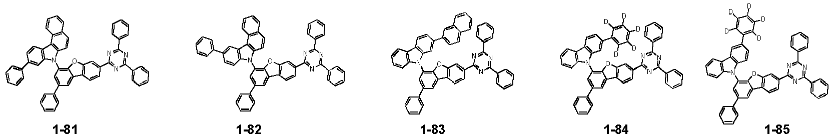

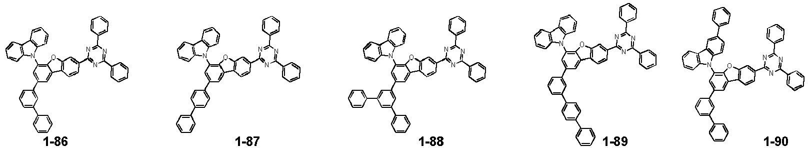

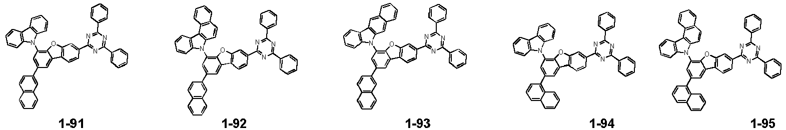

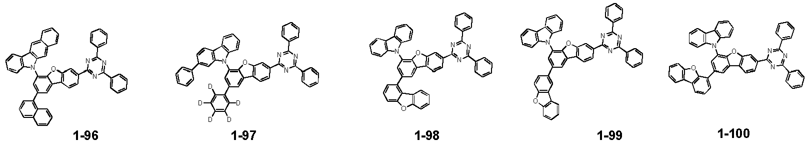

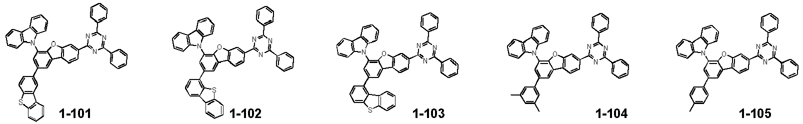

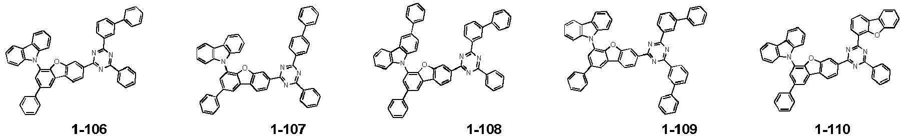

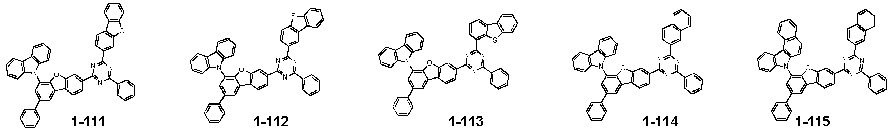

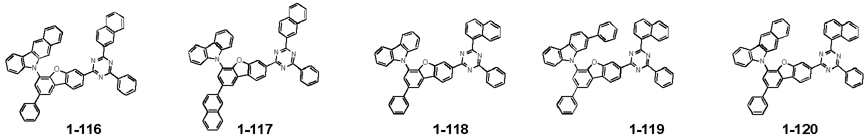

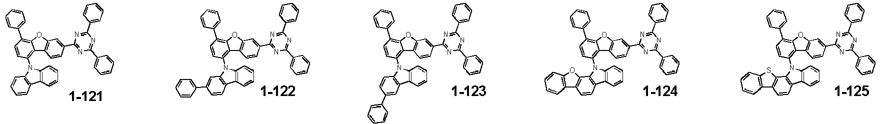

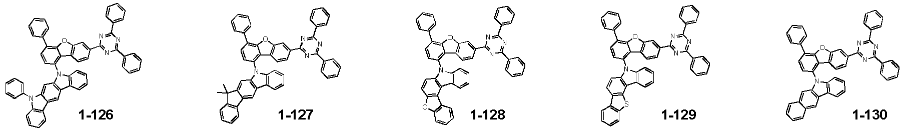

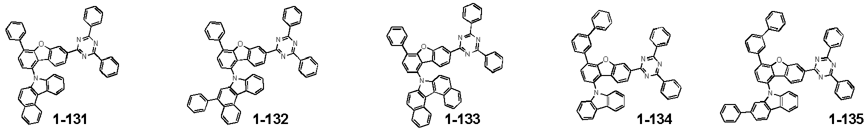

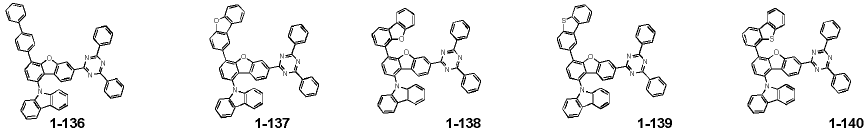

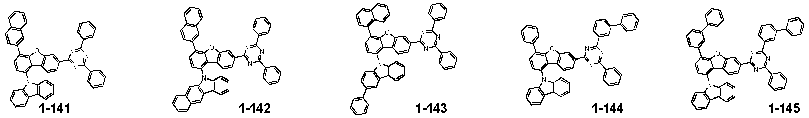

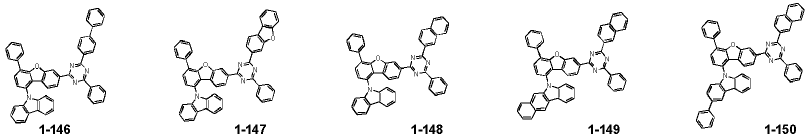

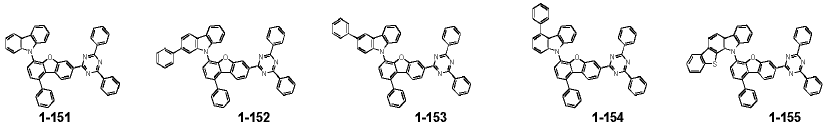

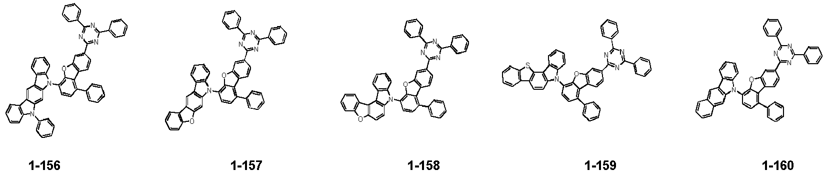

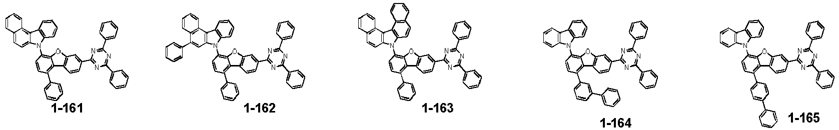

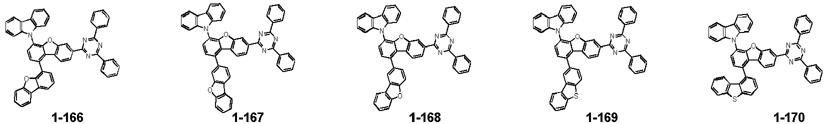

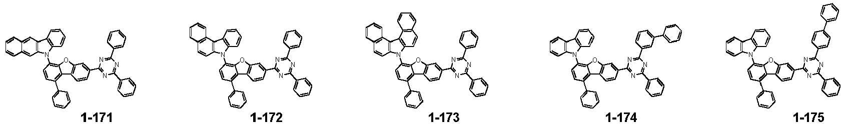

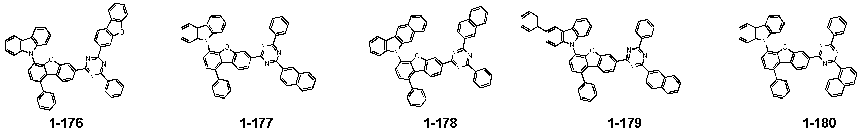

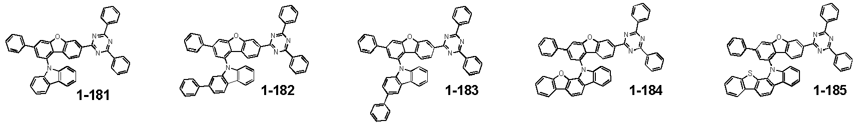

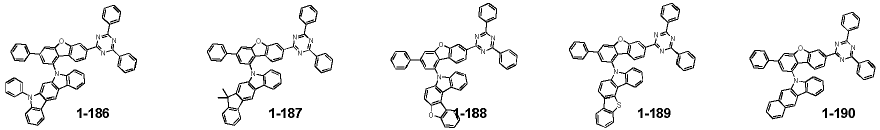

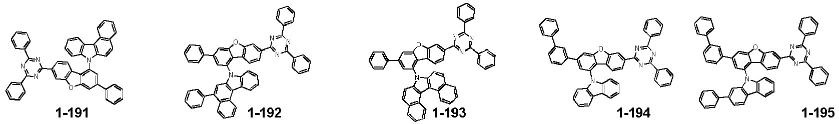

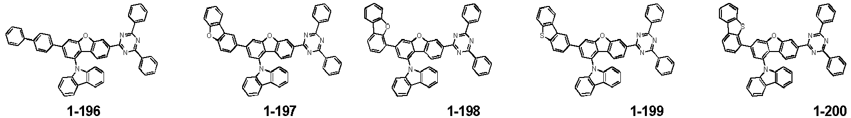

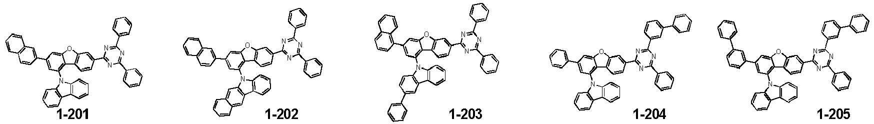

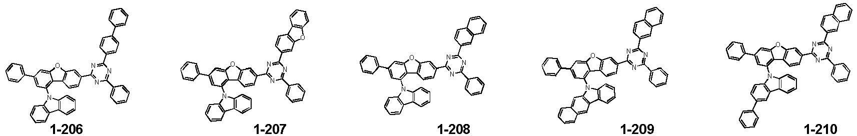

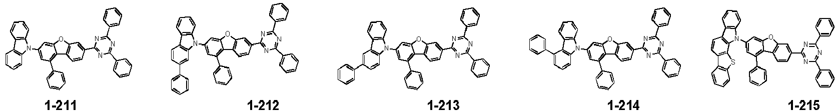

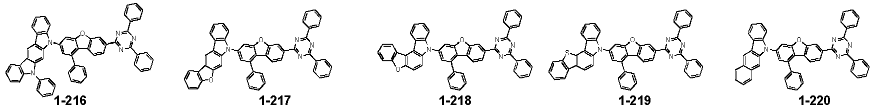

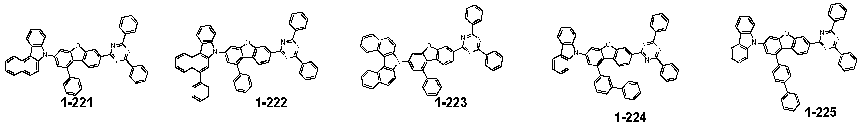

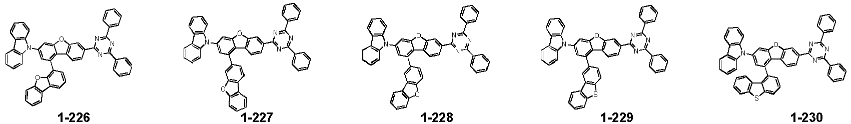

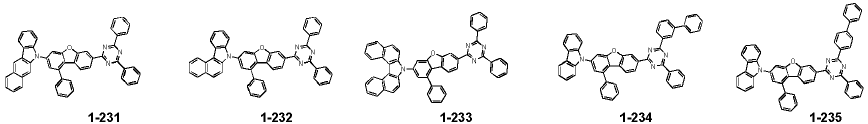

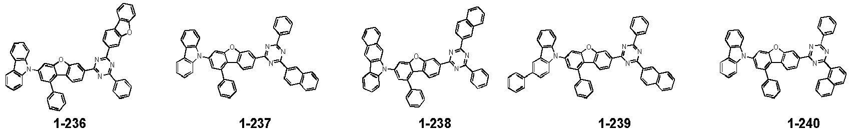

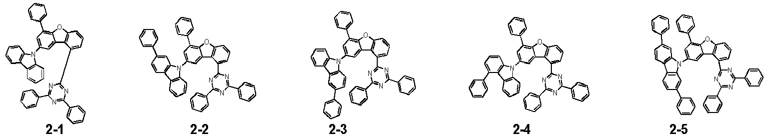

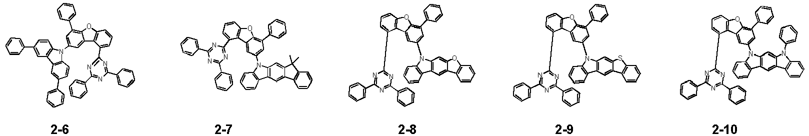

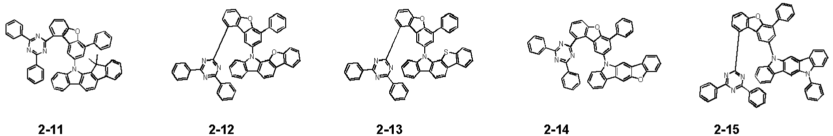

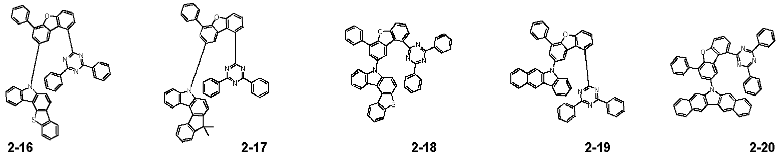

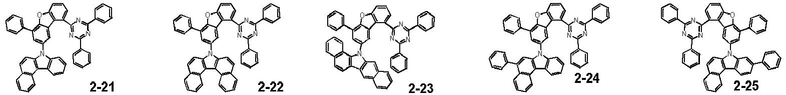

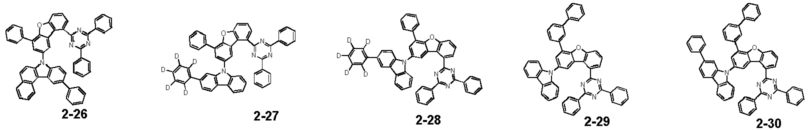

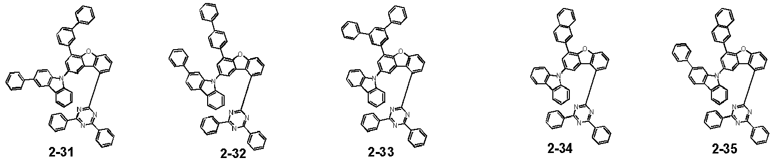

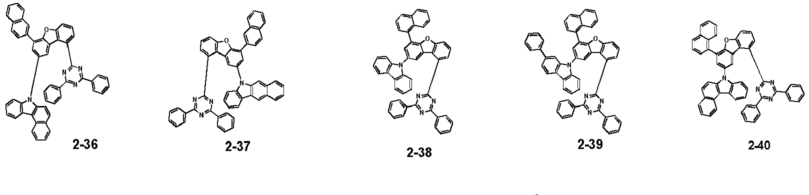

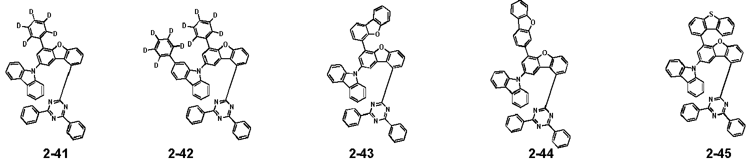

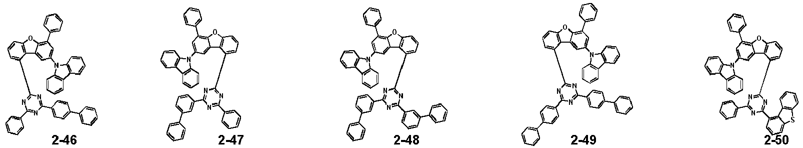

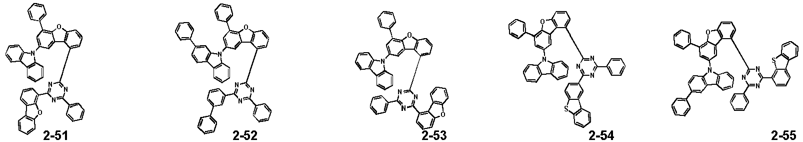

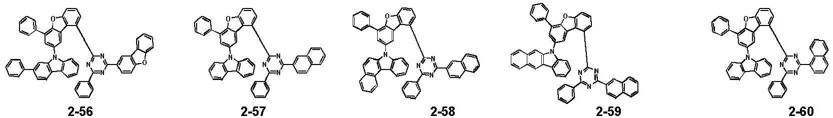

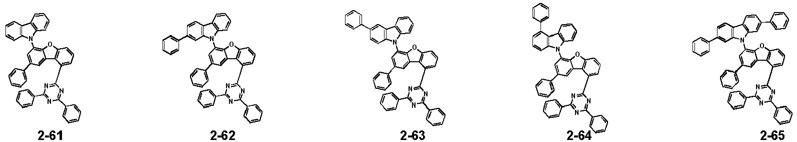

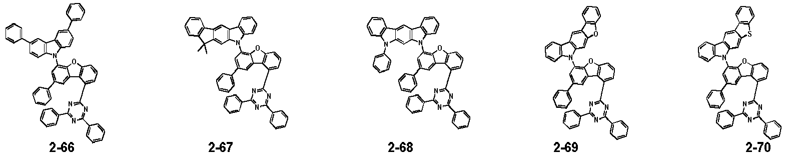

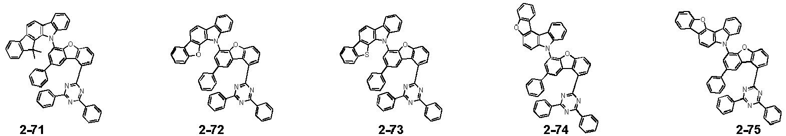

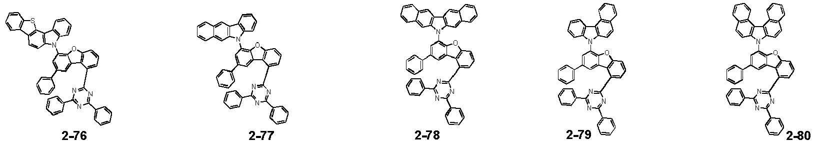

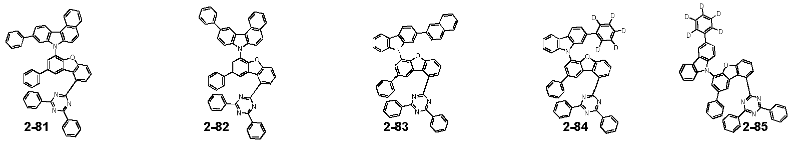

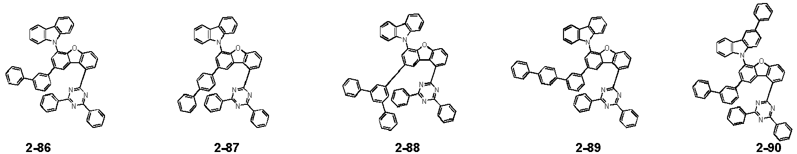

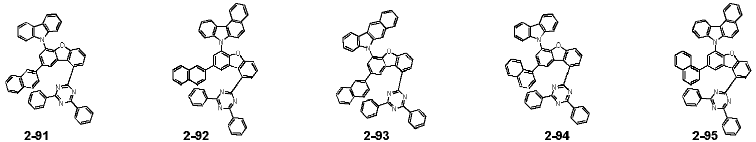

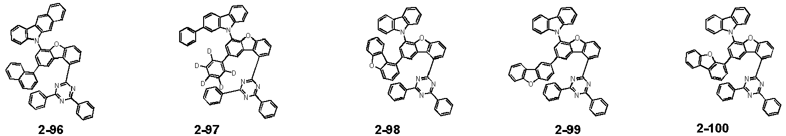

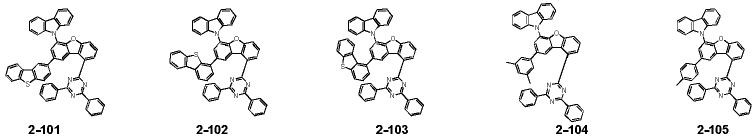

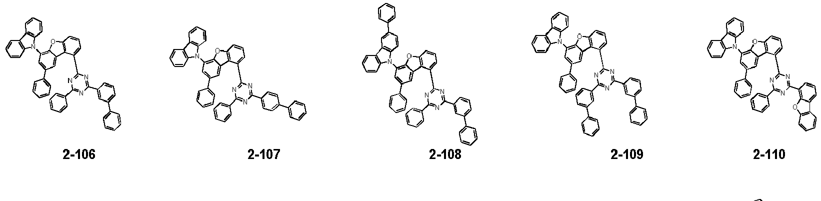

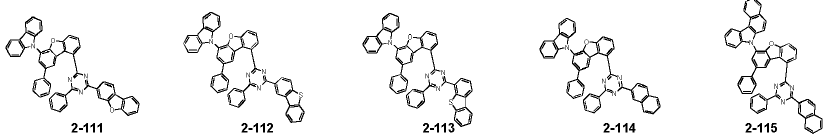

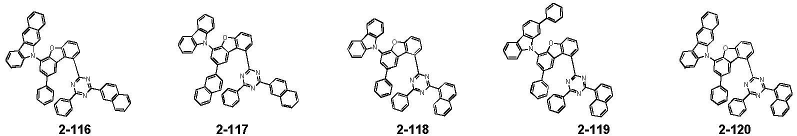

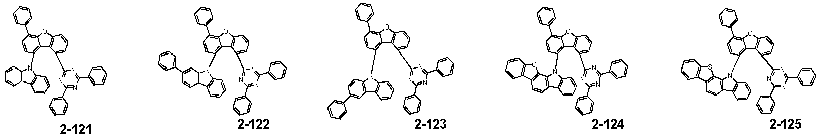

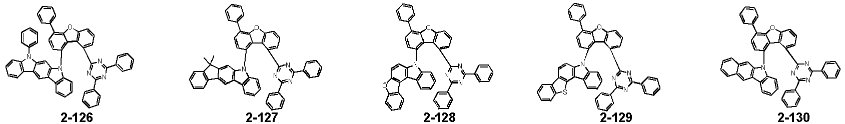

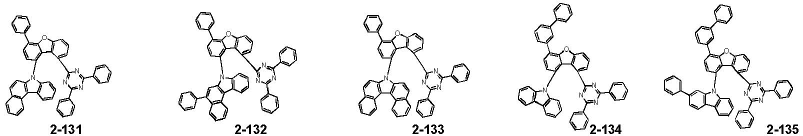

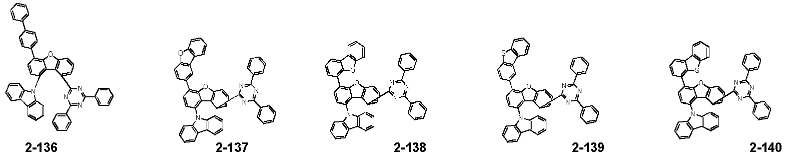

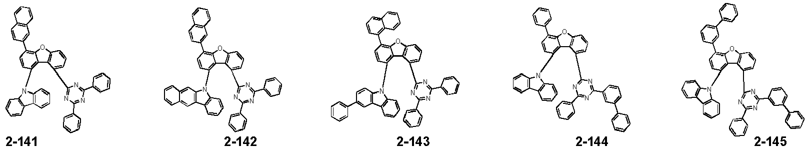

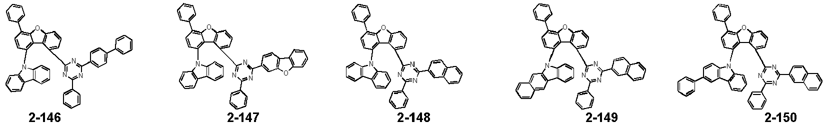

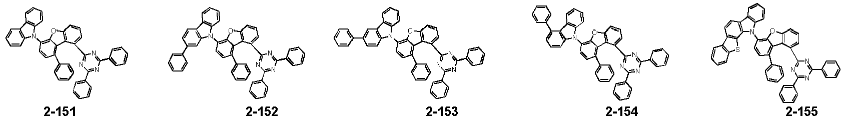

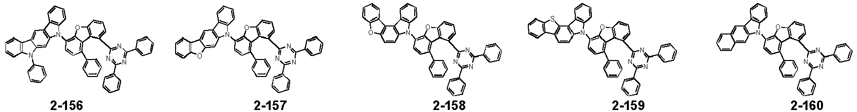

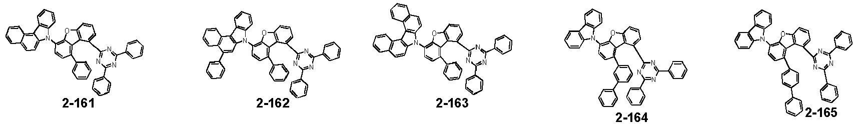

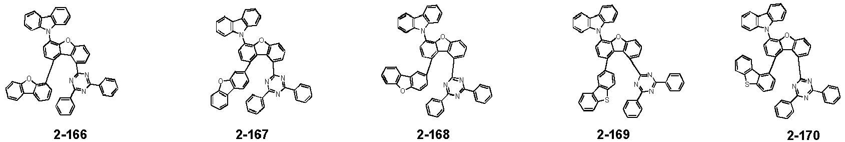

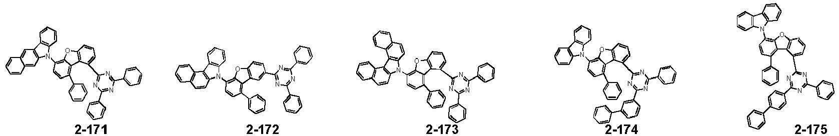

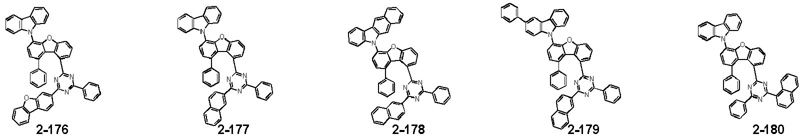

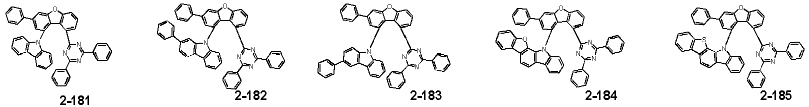

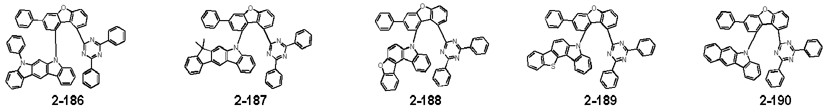

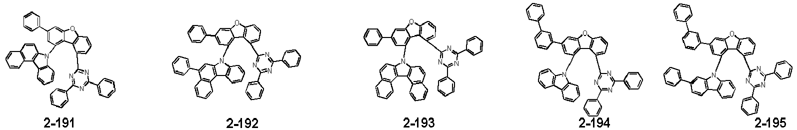

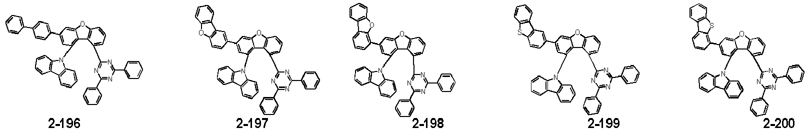

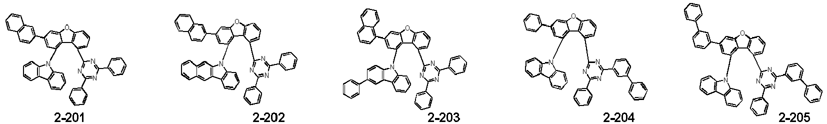

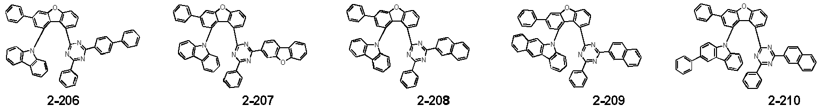

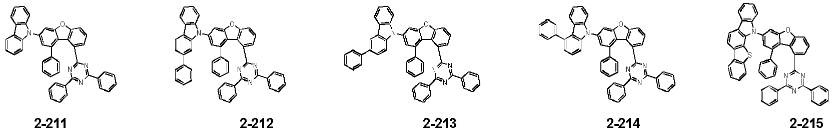

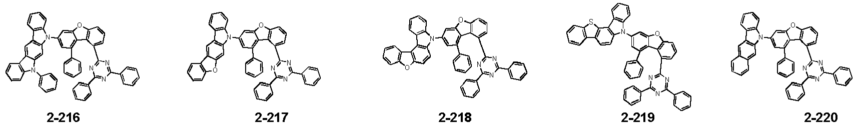

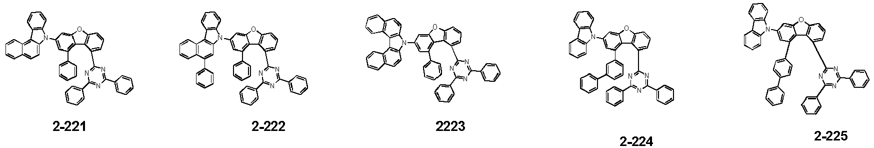

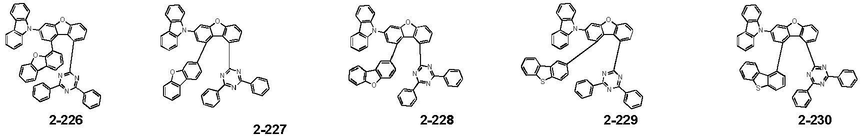

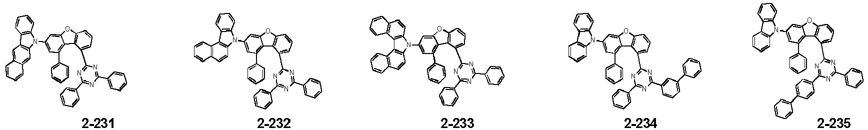

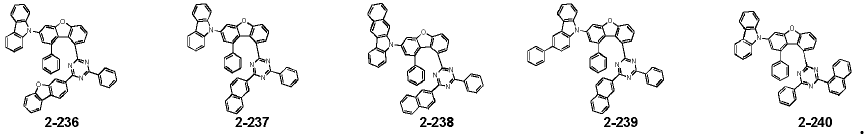

7. An organic light emitting device comprising:
a first electrode;
a second electrode provided opposite to the first electrode; and
one or more organic material layers provided between the first electrode and the second electrode,
wherein one or more layers of the organic material layers comprise the heterocyclic compound of any one of Claims 1 to 6.

8. The organic light emitting device of Claim 7, wherein the organic material layer comprises a light emitting layer, and the light emitting layer comprises the heterocyclic compound.

9. The organic light emitting device of Claim 7, wherein the organic material layer comprises a light emitting layer, the light emitting layer comprises a host material, and the host material comprises the heterocyclic compound.

10. The organic light emitting device of Claim 7, wherein the organic material layer comprises an electron injection layer or an electron transfer layer, and the electron transfer layer or the electron injection layer comprises the heterocyclic compound.

11. The organic light emitting device of Claim 7, wherein the organic material layer comprises an electron blocking layer or a hole blocking layer, and the electron blocking layer or the hole blocking layer comprises the heterocyclic compound.

12. The organic light emitting device Claim 7, further comprising one, two or more layers selected from the group consisting of a light emitting layer, a hole injection layer, a hole transfer layer. an electron injection layer, an electron transfer layer, an electron blocking layer and a hole blocking layer.

13. The organic light emitting device of Claim 7, wherein the organic material layer further comprises a heterocyclic compound of the following Chemical Formula 2: 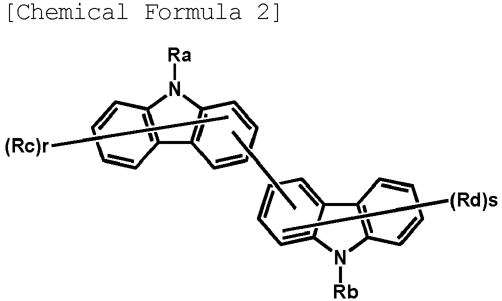 in Chemical Formula 2,
Ra and Rb are the same as or different from each other, and each independently a substituted or unsubstituted C6 to C60 aryl group; or a substituted or unsubstituted C2 to C60 heteroaryl group;
Rc and Rd are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; and a substituted or unsubstituted amine group; and
r and s are an integer of 0 to 7.

14. The organic light emitting device of Claim 13, wherein Chemical Formula 2 is represented by any one of the following compounds:

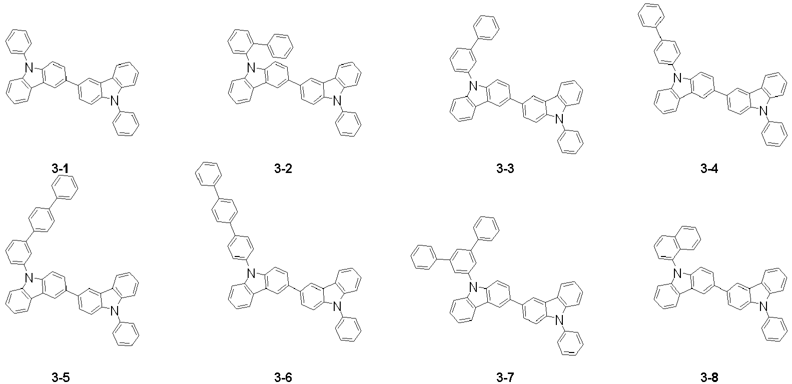

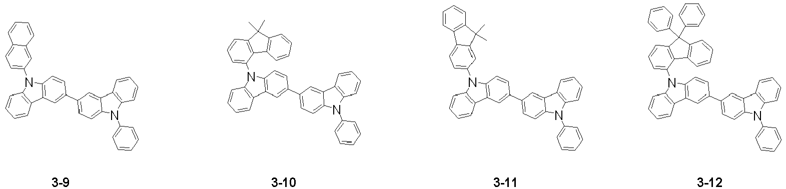

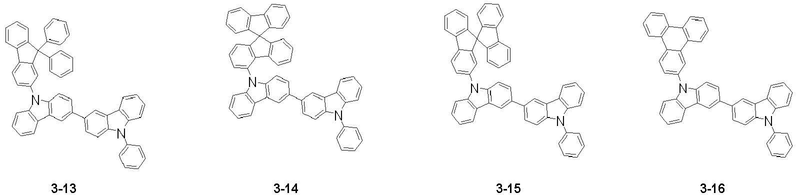

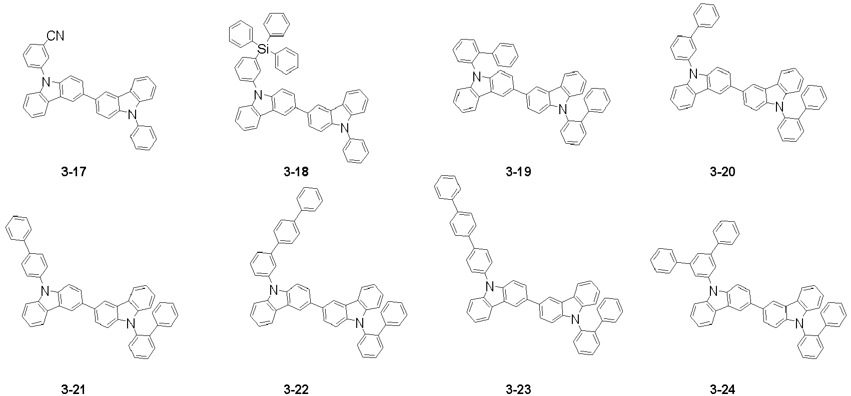

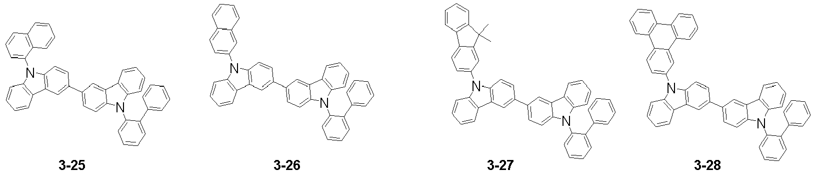

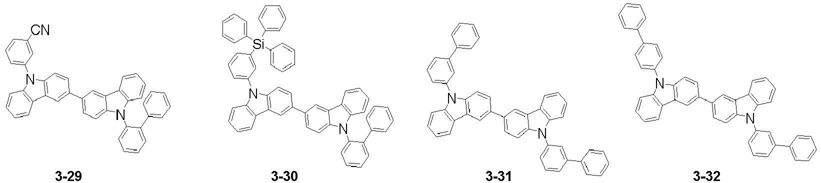

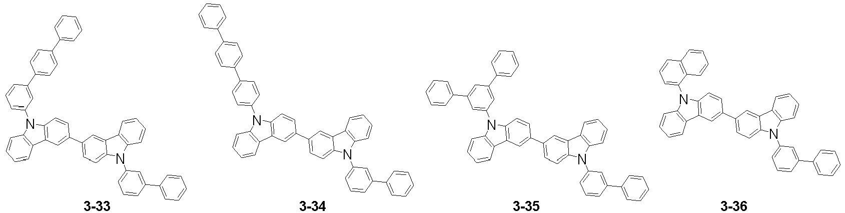

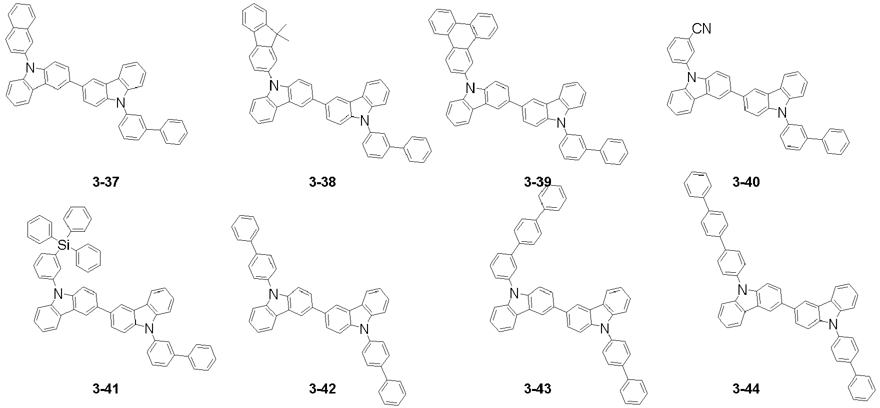

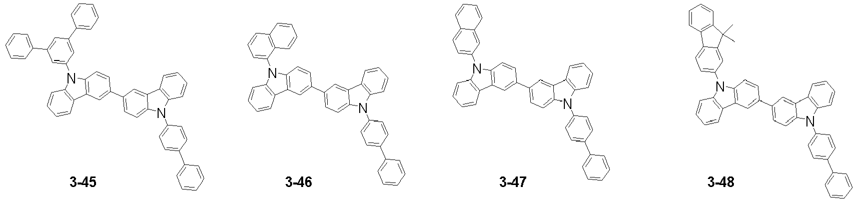

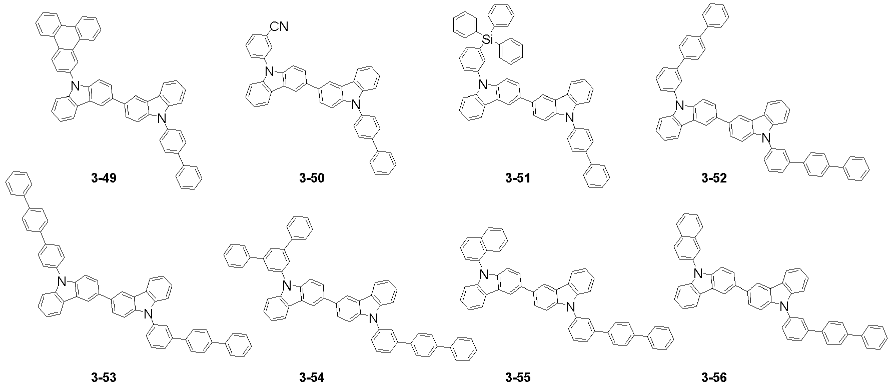

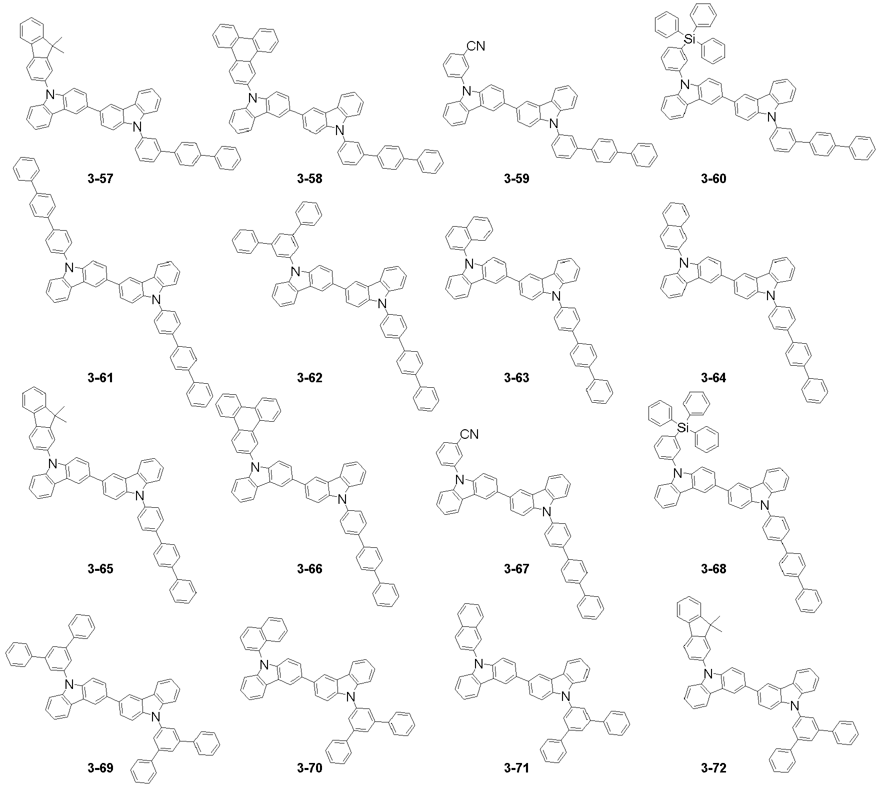

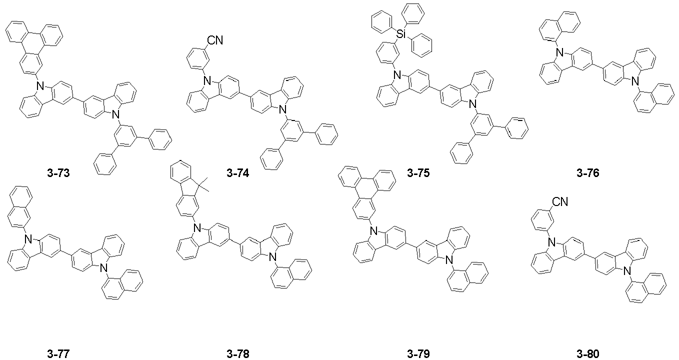

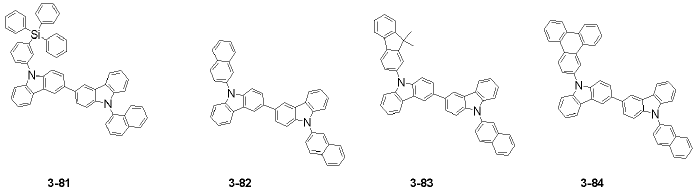

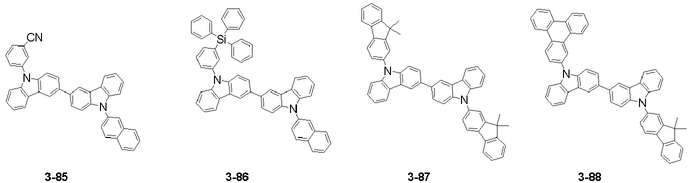

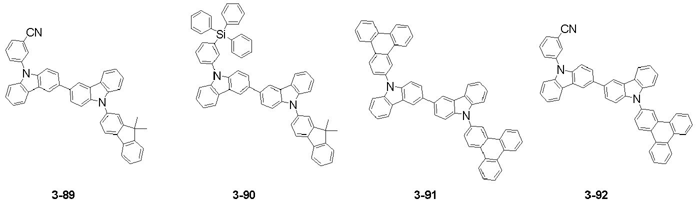

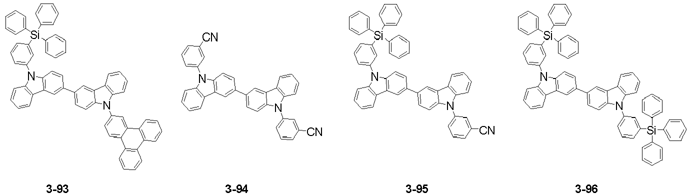

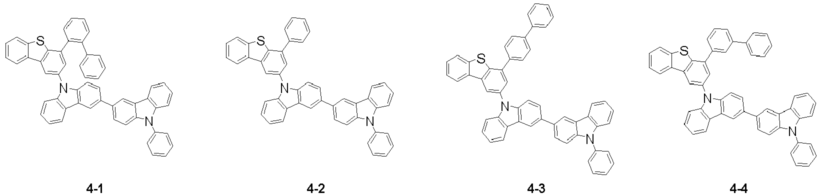

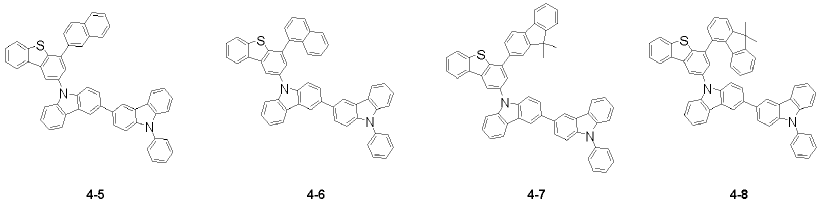

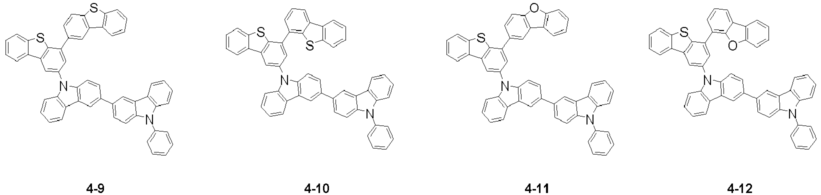

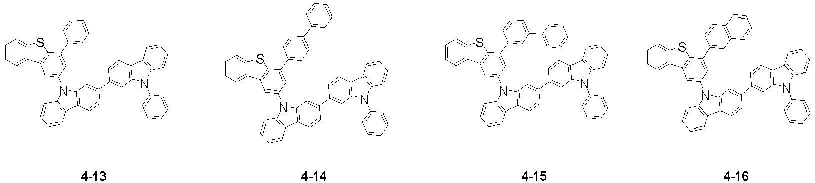

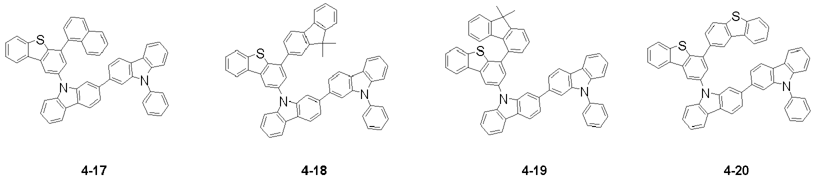

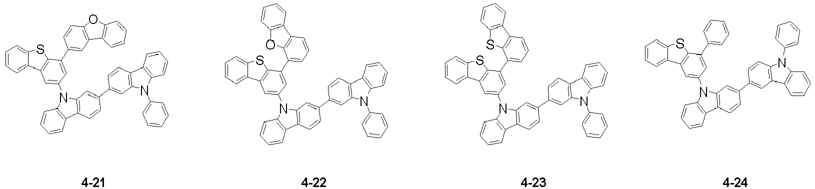

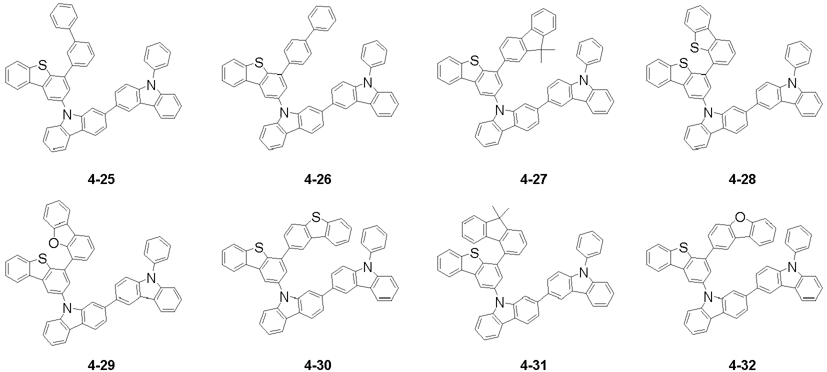

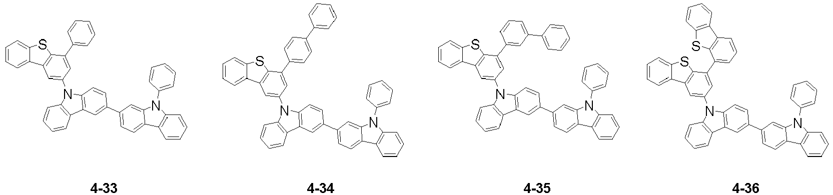

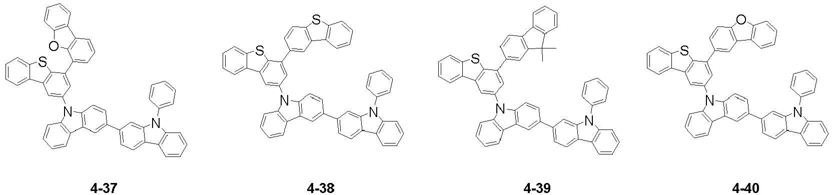

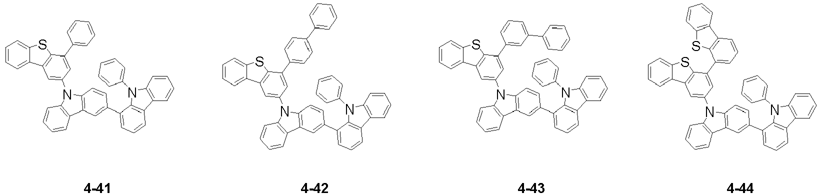

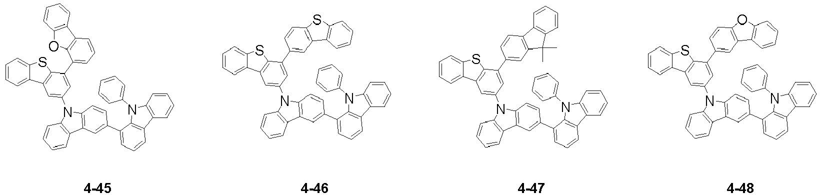

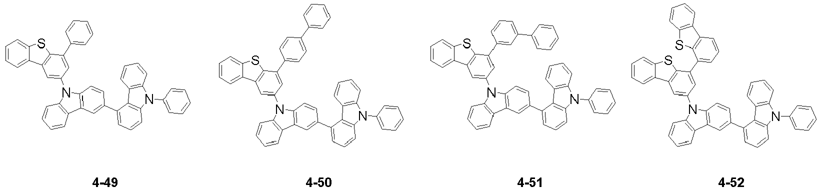

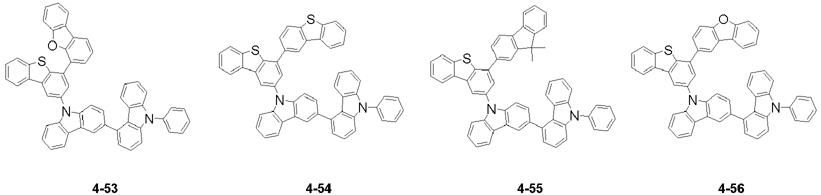

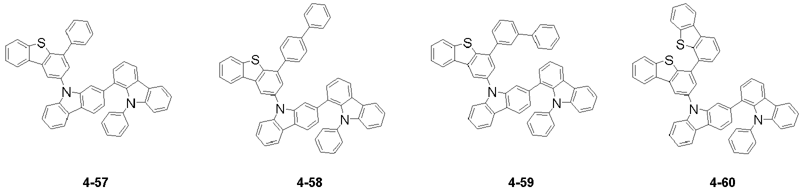

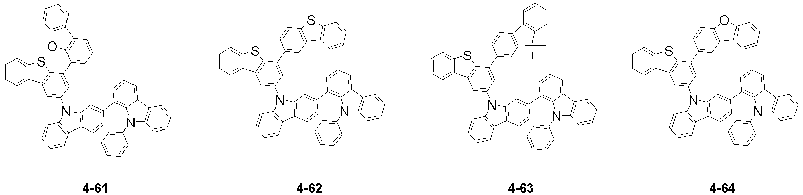

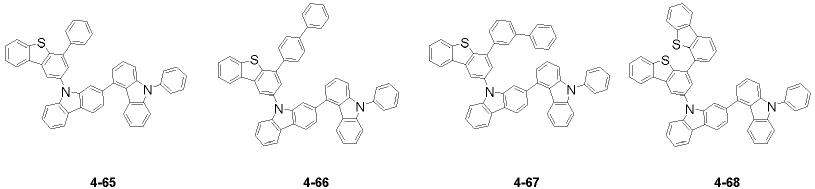

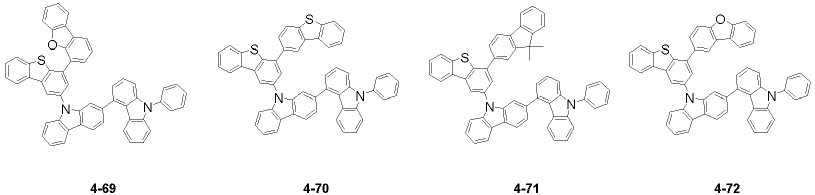

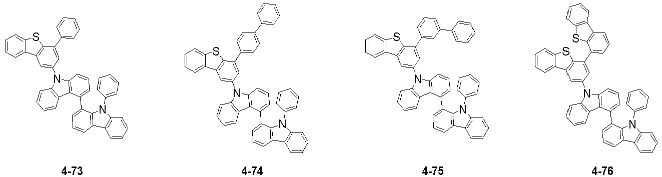

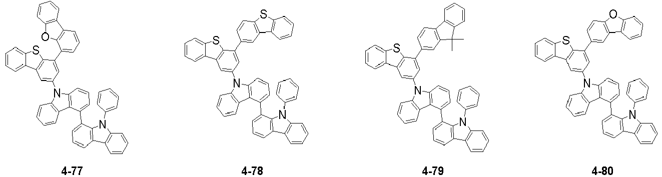

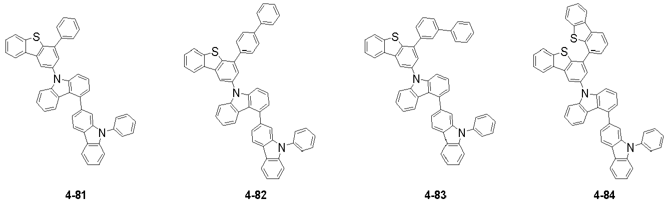

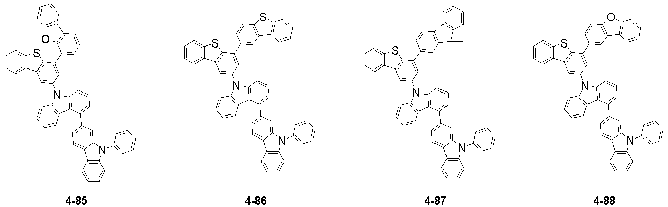

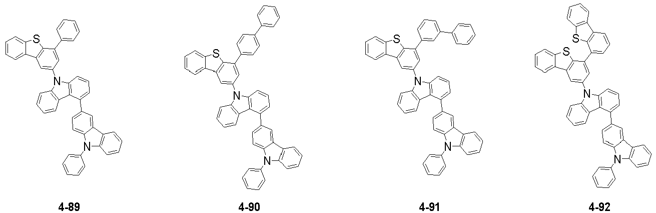

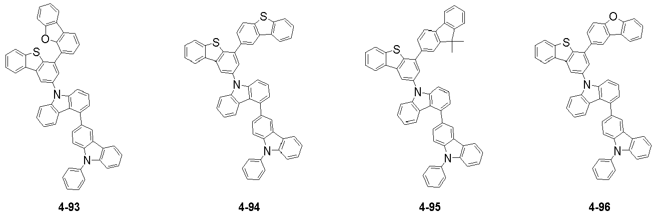

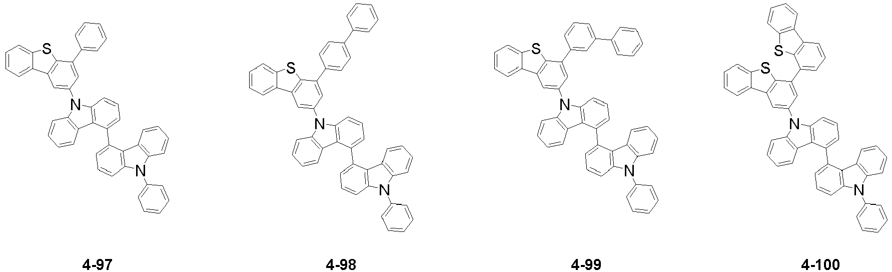

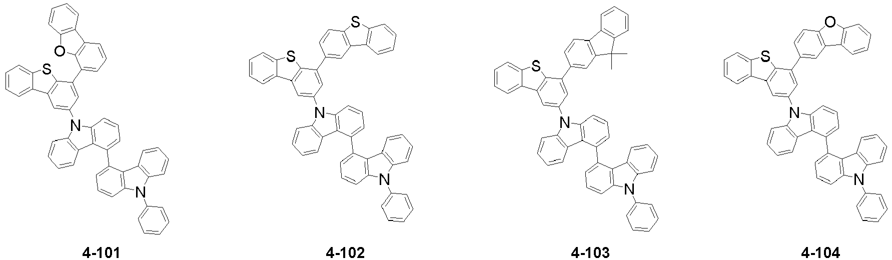

15. A composition for an organic material layer of an organic light emitting device, the composition comprising:
the heterocyclic compound represented by Chemical Formula 1 of Claim 1; and
a heterocyclic compound represented by the following Chemical Formula 2: 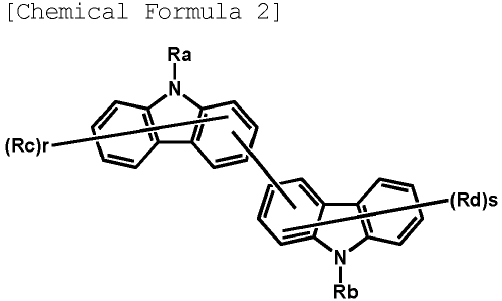
wherein, in Chemical Formula 2,
Ra and Rb are the same as or different from each other, and each independently a substituted or unsubstituted C6 to C60 aryl group; or a substituted or unsubstituted C2 to C60 heteroaryl group;
Rc and Rd are the same as or different from each other, and each independently selected from the group consisting of hydrogen; deuterium; halogen; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; and a substituted or unsubstituted amine group; and
r and s are an integer of 0 to 7.

16. The composition for an organic material layer of an organic light emitting device of Claim 15, wherein, in the composition, the heterocyclic compound represented by Chemical Formula 1 and the heterocyclic compound represented by Chemical Formula 2 have a weight ratio of 1:10 to 10:1.

17. A method for manufacturing an organic light emitting device, the method comprising:
preparing a substrate;
forming a first electrode on the substrate;
forming one or more organic material layers on the first electrode; and
forming a second electrode on the organic material layer,
wherein the forming of organic material layers comprises forming one or more organic material layers using the composition for an organic material layer of Claim 15.

18. The method for manufacturing an organic light emitting device of Claim 17, wherein the forming of organic material layers is forming using a thermal vacuum deposition method after pre-mixing the heterocyclic compound of Chemical Formula 1 and the heterocyclic compound of Chemical Formula 2.
